(19) Europäisches Patentamt
European Patent Office
Office européen des brevets

(11) **EP 3 852 623 B1**

(12) **EUROPEAN PATENT SPECIFICATION**

(45) Date of publication and mention
of the grant of the patent:
**04.12.2024 Bulletin 2024/49**

(21) Application number: **19808933.6**

(22) Date of filing: **29.10.2019**

(51) International Patent Classification (IPC):
**A61B 5/06** *(2006.01)*   **A61B 34/10** *(2016.01)*
**A61B 5/283** *(2021.01)*   **A61B 5/00** *(2006.01)*
**A61B 18/14** *(2006.01)*   **A61B 17/00** *(2006.01)*
**A61B 18/00** *(2006.01)*   **A61B 90/00** *(2016.01)*
**A61B 34/20** *(2016.01)*

(52) Cooperative Patent Classification (CPC):
**A61B 5/063; A61B 5/062; A61B 5/283;**
**A61B 18/1492; A61B 34/10;** A61B 5/6856;
A61B 2017/00243; A61B 2018/00267;
A61B 2034/102; A61B 2034/104; A61B 2034/2051;
A61B 2034/2053; A61B 2090/376

(86) International application number:
**PCT/US2019/058512**

(87) International publication number:
**WO 2020/096810 (14.05.2020 Gazette 2020/20)**

(54) **METHOD FOR MEDICAL DEVICE LOCALIZATION BASED ON MAGNETIC AND IMPEDANCE SENSORS**

VERFAHREN ZUR LOKALISIERUNG VON MEDIZINISCHEN GERÄTEN AUF DER BASIS VON MAGNET- UND IMPEDANZSENSOREN

PROCÉDÉ DE LOCALISATION DE DISPOSITIFS MÉDICAUX BASÉ SUR DES CAPTEURS MAGNÉTIQUES ET D'IMPÉDANCE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **07.11.2018 US 201862756941 P**
**07.11.2018 US 201862756915 P**
**07.11.2018 US 201862756926 P**
**07.11.2018 US 201862756931 P**
**07.11.2018 US 201862756936 P**

(43) Date of publication of application:
**28.07.2021 Bulletin 2021/30**

(73) Proprietor: **St. Jude Medical International Holding S.à r.l.**
**2449 Luxembourg (LU)**

(72) Inventors:
• **HILL, Anthony D.**
**Minneapolis, Minnesota 55407 (US)**

• **MALININ, Yuriy**
**Edina, Minnesota 55436 (US)**
• **THOMPSON, Cable**
**St. Paul, Minnesota 55104 (US)**
• **RYBNIKOV, Silvinia**
**3090925 Zichroon Ya'acov (IL)**
• **YORESH, Maxim**
**3467810 Haifa (IL)**
• **SUDARSKY, Oded**
**4294000 Kfar Yedidya (IL)**

(74) Representative: **Mathys & Squire**
**The Shard**
**32 London Bridge Street**
**London SE1 9SG (GB)**

(56) References cited:
**WO-A1-2016/205807     WO-A1-2016/205809**
**US-A1- 2012 150 022**

**Description**

CROSS REFERENCE

The present application claims the benefit of the filing dates of: U.S. Provisional Application No. 62/756,941 having a filing date of November 7, 2018; U.S. Provisional Application No. 62/756,915 having a filing date of November 7, 2018; U.S. Provisional Application No. 62/756,926 having a filing date of November 7, 2018; U.S. Provisional Application No. 62/756,931 having a filing date of November 7, 2018; and U.S. Provisional Application No. 62/756,936 having a filing date of November 7, 2018.

BACKGROUND

a. Field

The present disclosure relates generally to locating a medical device in a patient reference frame using a medical device model that estimates the shape of a medical device in the patient frame of reference in conjunction with measurements from impedance electrodes and magnetic sensors of the medical device.

b. Background

WO 2016/205809 A1 generally relates to impedance shift and drift detection and correction, wherein respective locations in a reference coordinate system are determined based on capturing processing signals received from a magnetic position sensor and signals received from an electrode.

WO 2016/205807 A1 relates to electromagnetic dynamic registration for device navigation.

US2012150022A1 relates generally to sensing the position of an object, especially a catheter, placed within a living body, and specifically to position sensing of a probe in a living body using multiple measuring parameters, such as impedance and magnetic sensing of electrodes location.

Various systems are known for determining the position and orientation (P&O) of a medical device in a human body, for example, for visualization and navigation purposes. One such system is known as an electrical impedance-based positioning system. Electrical impedance-based systems generally include one or more pairs of body surface electrodes (e.g., patches) outside a patient's body, a reference sensor (e.g., another patch) attached to the patient's body, and one or more sensors (e.g., electrodes) attached to the medical device. The pairs can be adjacent, linearly arranged, or associated with respective axes of a coordinate system for such a positioning system. The system can determine P&O by applying a current across pairs of electrodes, measuring respective voltages induced at the device electrodes (i.e., with respect to the reference sensor), and then processing the measured voltages.

Another system is known as a magnetic field-based positioning system. This type of system generally includes one or more magnetic field generators attached to or placed near the patient bed or other component of the operating environment and one or more magnetic field detection coils coupled with a medical device. Alternatively, the field generators may be coupled with a medical device, and the detection coils may be attached to or placed near a component of the operating environment. The generators provide a controlled low-strength AC magnetic field in the area of interest (i.e., an anatomical region). The detection coils produce a respective signal indicative of one or more characteristics of the sensed field. The system then processes these signals to produce one or more P&O readings associated with the coils (and thus with the medical device). The P&O readings are typically taken with respect to the field generators, and thus the field generators serve as the de facto "origin" of the coordinate system of a magnetic field-based positioning system. Unlike an electrical impedance-based system, where the coordinate system is relative to the patient on which the body surface electrodes are applied, a magnetic field-based system has a coordinate system that is independent of the patient.

Both electrical impedance-based and magnetic field-based positioning systems provide advantages. For example, electrical impedance-based systems provide the ability to simultaneously locate (i.e., provide a P&O reading for) a relatively large number of sensors on multiple medical devices. However, because electrical impedance-based systems employ electrical current flow in the human body, such systems may be subject to electrical interference. As a result, geometries and representations that are rendered based on position measurements may appear distorted relative to actual images of subject regions of interest. Magnetic field-based coordinate systems, on the other hand, are not dependent on characteristics of the patient's anatomy and typically provide improved accuracy. However, magnetic field-based positioning systems are generally limited to tracking relatively fewer sensors.

Efforts have been made to provide a system that combines the advantages of an electrical impedance-based positioning system (e.g., positioning of numerous electrodes) with the advantages of a magnetic-field based coordinate system (e.g., independence from patient anatomy, higher accuracy). For example, such a system may be provided by registering the coordinate systems of an electrical impedance-based positioning system with the coordinate system of a magnetic field-based positioning system. In such an arrangement, locations of electrodes may be identified in an impedance-based coordinate system in conjunction with identifying the locations of one or more magnetic sensors in a magnetic-based coordinate system. For example, at least a portion of the electrodes and magnetic sensors may be co-located to define fiducial pairs. This co-location allows for determining a transformation (e.g., transformation matrix) between the coordinate systems. The transformation may be applied to the locations of any electrode to register these locations in the magnetic-based coordinate system once the transformation is determined. Accordingly, the electrical impedance-based electrodes can be identified in the coordinate system of the magnetic field-based positioning system thereby increasing the positioning accuracy for the electrodes. While providing improved electrode positioning, the determination of a transformation between the impedance-based coordinate system and the magnetic based impedance system and subsequent registration of the electrode locations to the magnetic coordinate system can fail to account for various impedance shifts and/or drifts, associated with the electrode(s).

The previous systems that utilize electrode information (e.g., impedance measurements) and magnetic sensor information to provide improved electrode positioning in three-dimensional space (e.g., within a body of a patient) rely primarily on impedance-based measurements. That is, the magnetic sensor information (e.g., magnetic sensor measurements) delivers additional accuracy. This may be described as an impedance-primary location arrangement. Due to the distortion and temporal instability of the impedance measurements, such an arrangement can suffer from instability. Further, the previous impedance-primary location arrangements, in some instances, fail to account for various errors within the system. Further, such systems may fail to take into account other system inputs (e.g., patient movement, shape of the medical device, etc.), which may affect the calculated locations or positions of the electrodes. In summary, registration of an impedance-based system to magnetic-based system may fail to include additional information which may be observed and/or inferred and which may improve the overall identification of catheter and/or electrode positions in a three-dimensional space.

## BRIEF SUMMARY OF THE INVENTION

Present disclosure provides systems, methods and/or non-transitory computer readable medium storing instructions (i.e., utilities) for use in identifying location of electrodes of a catheter within a patient reference frame, according to independent claims 1, 14, 15. Initially, the utilities are directed to predicting locations of physical electrodes and/or physical magnetic sensors of a physical medical device disposed within the three-dimensional space. Based on the predicted locations of the electrodes and/or sensors, the utilities predict responses or measurements (hereafter 'responses') for the electrodes and/or sensors. Additionally, the utilities obtain actual measurements/responses from the electrodes and/or sensors of the physical catheter. According to the invention, the utilities acquires or measures impedance responses from the physical electrodes, upon application of an applied electrical potential field to the three-dimensional space. Likewise, the utilities may acquire or measure magnetic responses upon the application of a magnetic field to the three-dimensional space. Based on the predicted responses and the measured responses, the utilities updates the locations of electrodes or sensors in the three-dimensional space. Such updated locations utilizes information from both the predicted responses and the measured responses to produce locations (e.g., calculated locations) for the electrodes and/or sensors where the calculated locations have an accuracy that is greater than locations produced by either the predicted responses or the measured responses.

The foregoing and other aspects, features, details, utilities, and advantages of the present invention will be apparent from reading the following description and claims, and from reviewing the accompanying drawings.

The invention is solely defined by the appended claims.

## BRIEF DESCRIPTION OF THE DRAWINGS

FIG. 1 illustrates a schematic block diagram view of a system for determining the position of a medical device using impedance and magnetic measurements.

FIG. 2 illustrates a diagrammatic and block diagram view of an embodiment of an electrical impedance-based positioning system.

FIGS 3A-3D illustrate exemplary external impedance patch pairs suitable for use with the system of FIG. 2.

FIG. 4 illustrates an embodiment of a magnetic field-based positioning system.

FIG. 5A illustrates a set of models utilized for describing a composite model in accordance with the disclosure.

FIG. 5B illustrates a prediction of a catheter shape and translation of the catheter shape to a patient reference frame.

FIG. 5C illustrates the prediction of measurements for predicted locations in a patient reference frame and observed measurements in the patient reference frame.

FIG. 6A illustrates a catheter model having a magnetic sensor and multiple electrodes.

FIG. 6B illustrates a physical catheter and a corresponding catheter shape model.

FIGS. 7A-7C illustrate a state distribution, a regularizing function and application of the regularizing function to the sate distribution.

FIGS. 8A-8D illustrate various views of a catheter shape model of a planar catheter.

FIG. 9 illustrates a testing system for determining shape parameters relative to deformation.

FIG. 10 illustrates curvatures of proximal distal segments of a planar catheter.

FIGS. 11A-11C illustrate various transformation as diagramed in a patient reference frame.

FIGS. 12A and 12B illustrate orienting a catheter model in a patient reference frame.

FIG. 13A illustrates a mathematical graph of patch electrodes.

FIG. 13B illustrates an independent potential field.

FIG. 14 illustrates a constraint defined by a set of external impedance patch pairs.

FIG. 15 illustrates respiration waveforms.

FIG. 16 illustrates a state distribution

FIG. 17 illustrates one dimensional comparisons of observed states and measured states.

FIG. 18 illustrates a constraint manifold and a state distribution offset from the manifold.

FIG. 19 illustrates a block diagram of an example of a computer-readable medium in communication with processing resources of a computing device, in accordance with embodiments of the present disclosure.

FIG. 20 illustrate a flow diagram associated with determining a latent state of a system to identify electrode locations, in accordance with embodiments of the present disclosure.

FIGS. 21A and 22B illustrate call graphs of the interactions of the modules of FIG. 20.

FIG. 22 illustrates a call graph of a routine of FIG. 21A.

## DETAILED DESCRIPTION

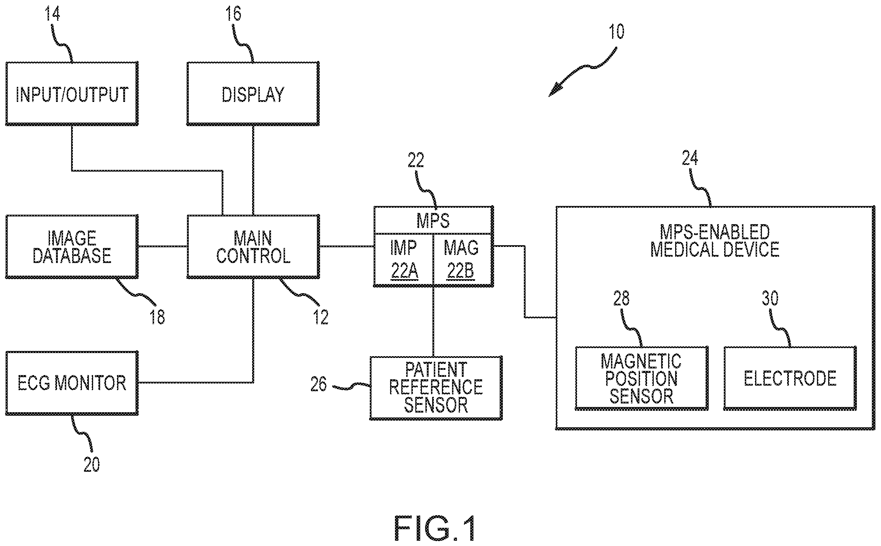

Referring now to the drawings wherein like reference numerals are used to identify identical or similar components in the various views, FIG. 1 is a diagrammatic view of a system 10 in which a medical device, such as a guidewire, catheter, introducer (e.g., sheath) incorporating a magnetic position sensor 28 and an electrode 30 may be used.

Before proceeding to a detailed description of the embodiments of the present disclosure, a description of an exemplary environment in which such devices and sensors may be used will first be set forth. With continued reference to FIG. 1, system 10, as depicted, includes a main electronic control unit 12 (e.g., a processor) having various input/output mechanisms 14, a display 16, an optional image database 18, an electrocardiogram (ECG) monitor 20, a localization system, such as a medical positioning system 22, a medical positioning system-enabled elongate medical device 24, a patient reference sensor 26, magnetic position sensor(s) 28 and electrode(s) 30. For simplicity, one magnetic position sensor 28 and one electrode 30 are shown, however, more than one magnetic position sensor 28 and/or more than one electrode 30 can be included in the system 10.

Input/output mechanisms 14 may comprise conventional apparatus for interfacing with a computer-based control unit including, for example, one or more of a keyboard, a mouse, a tablet, a foot pedal, a switch and/or the like. Display 16 may also comprise conventional apparatus, such as a computer monitor.

Various embodiments described herein may find use in navigation applications that use real-time and/or pre-acquired images of a region of interest. Therefore system 10 may optionally include image database 18 to store image information relating to the patient's body. Image information may include, for example, a region of interest surrounding a destination site for medical device 24 and/or multiple regions of interest along a navigation path contemplated to be traversed by medical device 24. The data in image database 18 may comprise known image types including (1) one or more two-dimensional still images acquired at respective, individual times in the past; (2) a plurality of related two-dimensional images obtained in real-time from an image acquisition device (e.g., fluoroscopic images from an x-ray imaging apparatus), wherein the image database acts as a buffer (live fluoroscopy); and/or (3) a sequence of related two-dimensional images defining a cine-loop wherein each image in the sequence has at least an ECG timing parameter associated therewith, adequate to allow playback of the sequence in accordance with acquired real-time ECG signals obtained from ECG monitor 20. It should be understood that the foregoing embodiments are examples only and not limiting in nature. For example, the image database may also include three-dimensional image data as well. It should be further understood that the images may be acquired through any imaging modality, now known or hereafter developed, for example X-ray, ultra-sound, computerized tomography, nuclear magnetic resonance or the like.

ECG monitor 20 is configured to continuously detect an electrical timing signal of the heart organ through the use of a plurality of ECG electrodes (not shown), which may be externally-affixed to the outside of a patient's body. The timing signal generally corresponds to a particular phase of the cardiac cycle, among other things. Generally, the ECG signal(s) may be used by the control unit 12 for ECG synchronized play-back of a previously captured sequence of images (cine loop) stored in database 18. ECG monitor 20 and ECG-electrodes may both comprise conventional components.

Another medical positioning system sensor, namely, patient reference sensor (PRS) 26 (if provided in system 10) can be configured to provide a positional reference of the patient's body so as to allow motion compensation for patient body movements, such as respiration-induced movements. Such motion compensation is described in greater detail in U.S. patent application Ser. No. 12/650,932, entitled "Compensation of Motion in a Moving Organ Using an Internal Position Reference Sensor". PRS 26 may be attached to the patient's manubrium sternum or other location. PRS 26 can be configured to detect one or more characteristics of the magnetic field in which it is disposed, wherein medical positioning system 22 determines a location reading (e.g., a P&O reading) indicative of the PRS's position and orientation in the magnetic reference coordinate system.

Medical positioning system 22 is configured to serve as the localization system and therefore to determine position (localization) data with respect to one or more magnetic position sensors 28 and/or electrodes 30 and output a respective location reading. In an embodiment, a medical positioning system 22 may include a first medical positioning system or an electrical impedance-based medical positioning system 22A that determines electrode locations in a first coordinate system, and a second medical positioning system or magnetic field-based medical positioning system 22B that determines magnetic position sensors in a second coordinate system. In an embodiment, the location readings may each include at least one or both of a position and an orientation (P&O) relative to a reference coordinate system (e.g., magnetic based coordinate system or impedance based coordinate system). For some types of sensors, the P&O may be expressed with five degrees-of-freedom (five DOF) as a three-dimensional (3D) position (e.g., a coordinate in three perpendicular axes X, Y and Z) and two-dimensional (2D) orientation (e.g., a pitch and yaw) of an electromagnetic position sensor 28 in a magnetic field relative to a magnetic field generator(s) or transmitter(s) and/or electrode 30 in an applied electrical field relative to an electrical field generator (e.g., a set of electrode patches). For other sensor types, the P&O may be expressed with six degrees-of-freedom (six DOF) as a 3D position (e.g., X, Y, Z coordinates) and 3D orientation (e.g., roll, pitch, and yaw).

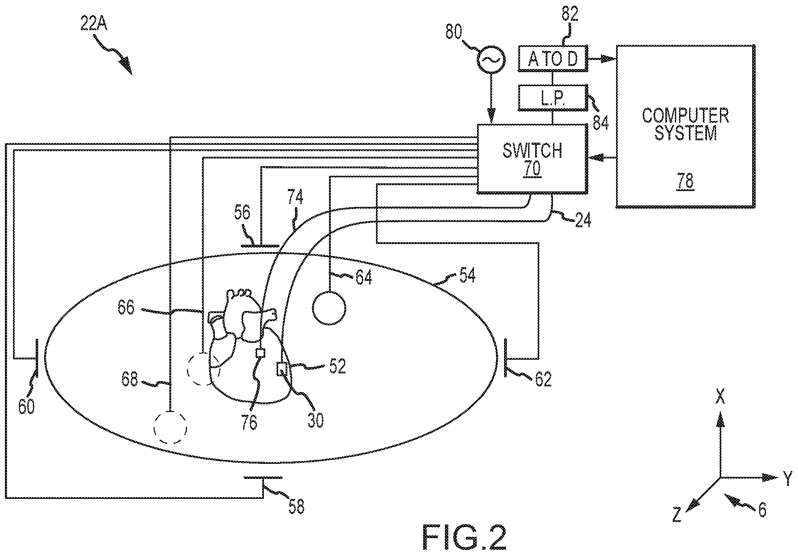

Impedance based medical positioning system 22A determines electrode locations based on capturing and processing signals received from the electrodes 30 and external electrode patches while the electrodes are disposed in a controlled electrical field (e.g., potential field) generated by the electrode patches, for example. FIG. 2 is a diagrammatic overview of an exemplary electrical impedance-based medical positioning system ('MPS system') 22A. MPS system 22A may comprise various visualization, mapping and navigation components as known in the art, including, for example, an EnSite™ Electro Anatomical Mapping System commercially available from St. Jude Medical, Inc., or as seen generally by reference to U.S. Pat. No. 7,263,397 entitled "Method and Apparatus for Catheter Navigation and Location and Mapping in the Heart" to Hauck et al., or U.S. Patent Publication No. 2007/0060833 A1 to Hauck entitled "Method of Scaling Navigation Signals to Account for Impedance Drift in Tissue", both owned by the common assignee of the present invention.

Medical positioning system 22A includes a diagrammatic depiction of a heart 52 of a patient 54. The system includes the ability to determine a catheter electrode location (i.e., position and orientation) as the catheter distal end is moved around and within a chamber of the heart 52. For this purpose, three sets of body surface electrodes (patches) are shown: (1) electrodes 56, 58 (X-axis); (2) electrodes 60, 62 (Y-axis); and (3) electrodes 64, 66 (Z-axis). Additionally, a body surface electrode ("belly patch") 68 is shown diagrammatically. The surface electrodes are all connected to a switch 70. Of course, other surface electrode configurations and combinations are suitable for use with the present invention, including fewer electrodes, e.g., three electrodes, more electrodes, e.g., twelve, or different physical arrangements, e.g., linear arrangement instead of an orthogonal arrangement.

Medical device 24 is shown as a catheter with a distal electrode 30. Catheter 24 may have additional electrodes in addition to electrode 30 (e.g., a catheter tip electrode and/or ring electrodes) as well as one or more magnetic position sensors (not shown). FIG. 2 also shows a second, independent catheter 74 with a fixed reference electrode 76, which may be stationary on the heart for calibration purposes. In many instances, a coronary sinus electrode or other fixed reference electrode 76 in the heart 52 can be used as a reference for measuring voltages and displacements.

It should be understood that catheter 24 may include still other electrodes, and in other embodiments, such as in EP or RF ablation embodiments, the other electrodes may be used for any number of diagnostic and/or therapeutic purposes. For instance, such electrodes and therefore such catheters may be used for performing ablation procedures, cardiac mapping, electrophysiological (EP) studies and other diagnostic and/or therapeutic procedures. Embodiments are not limited to any one type of catheter or catheter-based system or procedure.

FIG. 2 further shows a computer system 78, a signal generator 80, an analog-to-digital converter 82 and a low-pass filter 84. Computer system 78 includes a processing apparatus configured to perform various functions and operations described herein. Computer system 78 may be configured to control signal generator 80 in accordance with predetermined strategies to selectively energize various pairs (dipoles) of surface electrodes. In operation, computer system 78 may (1) obtain raw patch data (i.e., voltage readings) via filter 84 and A-to-D converter 82 and (2) use the raw patch data (in conjunction with electrode measurements) to determine the raw, uncompensated, electrode location coordinates of a catheter electrode positioned inside the heart or chamber thereof (e.g., such as electrode 30) in a three-dimensional coordinate system (e.g., impedance-based coordinate system). Computer system 78 may be further configured to perform one or more compensation and adjustment functions, and to output a location in coordinate system 14 of one or more electrodes such as electrode 72. Motion compensation may include, for example, compensation for respiration-induced patient body movement, as described in U.S. patent application Ser. No. 12/980,515, entitled "Dynamic Adaptive Respiration Compensation with Automatic Gain Control".

Each body surface (patch) electrode is independently coupled to switch 70 and pairs of electrodes are selected by software running on computer system 78, which couples the patches to signal generator 80. A pair of electrodes, for example the Z-axis electrodes 64 and 66, may be excited by signal generator 80 to generate an electrical field in the body of patient 54 and heart 52. In one embodiment, this electrode excitation process occurs rapidly and sequentially as different sets of patch electrodes are selected and one or more of the unexcited (in an embodiment) surface electrodes are used to measure voltages. During the delivery of the excitation signal (e.g., current pulse), the remaining (unexcited) patch electrodes may be referenced to the belly patch 68 and the voltages impressed on these remaining electrodes are measured by the A-to-D converter 82. In this fashion, the surface patch electrodes are divided into driven and non-driven electrode sets. Low pass filter 84 may process the voltage measurements. The filtered voltage measurements are transformed to digital data by analog to digital converter 82 and transmitted to computer 78 for storage under the direction of software. This collection of voltage measurements is referred to herein as the "patch data." The software has access to each individual voltage measurement made at each surface electrode during each excitation of each pair of surface electrodes.

The patch data is used, along with measurements made at electrode 30, to determine a relative location of electrode 30 in what may be termed a patient-based coordinate system or patient reference frame 6. That is, as the patches are applied directly to the patient, the patient defines the reference frame of the impedance measurements. Potentials across each of the six orthogonal surface electrodes may be acquired for all samples except when a particular surface electrode pair is driven (in an embodiment). In one embodiment, sampling while a surface electrode acts as a source or sink in a driven pair is normally avoided as the potential measured at a driven electrode during this time may be skewed by the electrode impedance and the effects of high local current density. In an alternate embodiment, however, sampling may occur at all patches (even those being driven).

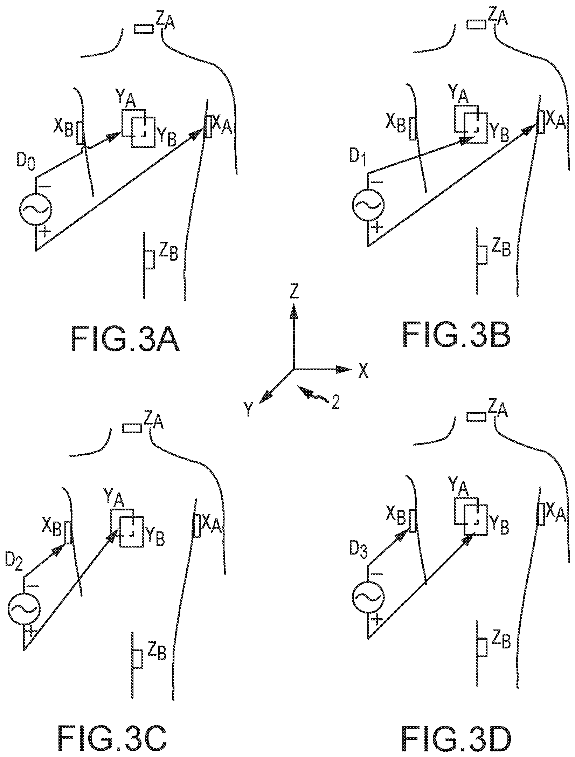

Generally, in one embodiment, three nominally orthogonal electric fields are generated by a series of driven and sensed electric dipoles in order to realize localization function of the catheter in a biological conductor. Alternately, these orthogonal fields can be decomposed and any pair of surface electrodes (e.g., non-orthogonal) may be driven as dipoles to provide effective electrode triangulation. FIGS. 3A-3D show a plurality of exemplary non-orthogonal dipoles, designated $D_0$, $D_1$, $D_2$ and $D_3$, set in the impedance-based coordinate system 2. In FIGS. 3A-3D, the X-axis surface electrodes are designated $X_A$ and $X_B$, the Y-axis surface electrodes are designated YA and YB, and the Z-axis electrodes are designated $Z_A$ and $Z_B$. For any desired axis, the potentials measured across an intra-cardiac electrode 30 resulting from a predetermined set of drive (source-sink) configurations may be combined algebraically to yield the same effective potential as would be obtained by simply driving a uniform current along the orthogonal axes. Any two of the surface electrodes 56, 58, 60, 62, 64, 66 (see FIG. 2) may be selected as a dipole source and drain with respect to a ground reference, e.g., belly patch 68, while the unexcited body surface electrodes measure voltage with respect to the ground reference. The measurement electrode 30 placed in heart 52 is also exposed to the field from a current pulse and is measured with respect to ground, e.g., belly patch 68. In practice, a catheter or multiple catheters within the heart may contain multiple electrodes and each electrode potential may be measured separately. As previously noted, alternatively, at least one electrode may be fixed to the interior surface of the heart to form a fixed reference electrode 76, which may also be measured with respect to ground.

Data sets from each of the surface electrodes and the internal electrodes are all used to determine the location of measurement electrode 30 within heart 52. After the voltage measurements are made, a different pair of surface electrodes is excited by the current source and the voltage measurement process of the remaining patch electrodes and internal electrodes takes place. The sequence occurs rapidly, e.g., on the order of 100 times per second in an embodiment. To a first approximation the voltage on the electrodes within the heart bears a linear relationship with position between the patch electrodes that establish the field within the heart, as more fully described in U.S. Pat. No. 7,263,397 referred to above.

Magnetic-based medical positioning system 22B determines magnetic position sensor locations (e.g., P&O) in a magnetic coordinate system based on capturing and processing signals received from the magnetic position sensor 28 while the sensor is disposed in a controlled low-strength alternating current (AC) magnetic (e.g., magnetic) field. Each magnetic position sensor 28 and the like may comprise a coil and, from an electromagnetic perspective, the changing or AC magnetic field may induce a current in the coil(s) when the coil(s) are in the magnetic field. The magnetic position sensor 28 is thus configured to detect one or more characteristics (e.g., flux) of the magnetic field(s) in which it is disposed and generate a signal indicative of those characteristics, which is further processed by medical positioning system 22B to obtain a respective P&O for the magnetic sensor 28 relative to, for example, a magnetic field generator.

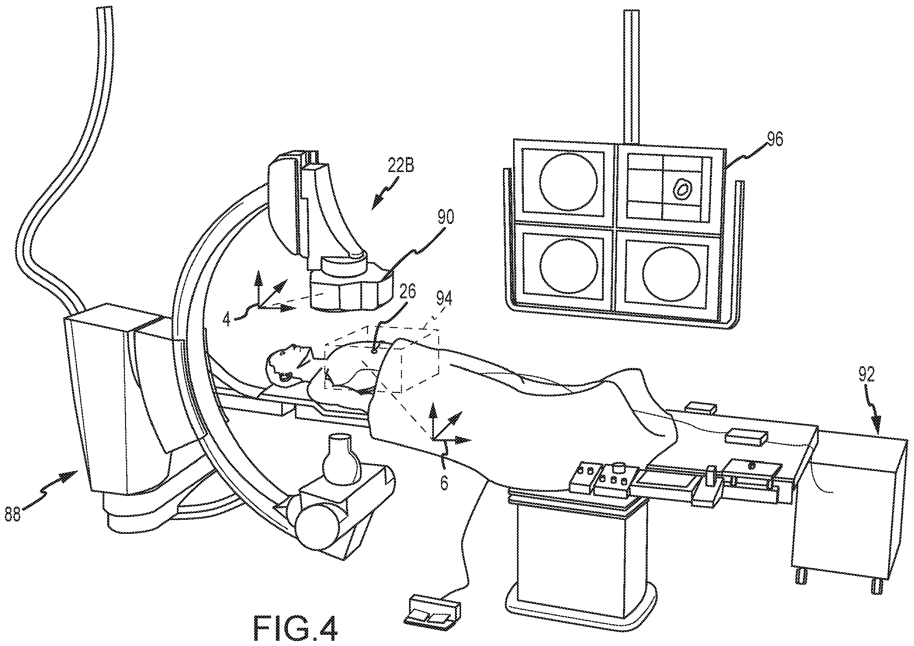

FIG. 4 is a diagrammatic view of an exemplary magnetic field-based medical positioning system 22B in a fluoroscopy-based imaging environment, designated system 88. A magnetic field generator or magnetic transmitter assembly (MTA) 90 and a magnetic processing core 92 for determining position and orientation (P&O) readings generally define the magnetic field-based positioning system 22B. The MTA 90 is configured to generate the magnetic field(s) in and around the patient's chest cavity in a predefined three-dimensional space designated as motion box 94 in FIG. 4. Magnetic field sensors coupled with device 24 (e.g., catheter or another medical device) are configured to sense one or more characteristics of the magnetic field(s) and, when the sensors are in the motion box 94, each generates a respective signal that is provided to the magnetic processing core 92. The processing core 92 is responsive to these detected signals and is configured to calculate respective three-dimensional position and orientation (P&O) readings for each magnetic field sensor. Thus, the MPS system 22B enables real-time tracking of each magnetic field sensor in three-dimensional space, which forms a magnetic-based coordinate system 4. The position of the sensors may be shown on a display 96 relative to, for example only, a cardiac model or geometry. Additional exemplary embodiments of magnetic field-based medical positioning systems are set forth in co-owned U.S. Pat. No. 7,386,339 and U.S. Pat. App. No 2013/066193. It should be understood that variations are possible, for example, as also seen by reference to U.S. Pat. Nos. 7,197,354, and 6,233,476. Unlike the electrical impedance-based system discussed in relation to FIG. 2, which has an origin based on a patient reference frame 6 as the body surface electrodes are applied directly to the patient, the origin of the magnetic field-based system is typically based in or on the MTA 90 (e.g., as shown by the dashed line) and is independent of the patient. Stated otherwise, the patient coordinate system (e.g., patient reference frame) 6 and the magnetic-based coordinate system 4 have different origins.

As further illustrated in FIG. 4, a patient reference sensor (PRS) 26 may be applied to the patient. In an embodiment, the PRS 26 may be attached to the patient's manubrium sternum. However other patient locations for the PRS 26 are possible. In an embodiment, the PRS 26 is a magnetic sensor configured to detect one or more characteristics of the magnetic field in which it is disposed, wherein medical positioning system 22B determines a location reading (e.g., a P&O reading) indicative of the position and orientation of the PRS 26 (e.g., in the magnetic-based coordinate system). For the present application, the PRS defines an origin (e.g., PRF 0,0,0) in the patient reference coordinate system or patient reference frame 6 (PRF). The origin may be offset from the actual location of the senor. That is, predetermined offsets (e.g., x, y, and z) may be applied to the PRS measurements that correspond with estimated distances between the sensor's placement on the patient and the desired origin. For instance, the origin may be offset from the sensor such that it is within the heart of the patient for cardiac applications. Further, two or more PRS may be applied to provide additional orientation information for the PRF 6. In any embodiment, as the PRS 26 is attached to the patient and moves with patient movement, the origin of the PRF 6 also moves. Such movement may result from patient respiration and/or physical movements (shifting, rolling etc.) of the patient. The origin of the PRF 6 is thus dependent on the position of the patient and may be updated over time. More specifically, a measurement of the PRS may be determined in the magnetic field coordinate system and this measurement may be utilized as the origin (e.g., with adjustment) of the PRF.

As previously noted, the impedance-based medical positioning systems and magnetic-based medical positioning systems have different strengths and weaknesses. For instance, impedance-based systems provide the ability to simultaneously locate a relatively large number of electrodes. However, because impedance-based systems employ electrical current flow in the human body, the system can be subject to measurement inaccuracies due to shift and/or drift caused by various physiological phenomena (e.g., local conductivity changes, sweat/patch interactions, etc.). Additionally, impedance-based systems may be subject to electrical interference. As a result, electrode locations, renderings, geometries and/or representations based on such impedance-based measurements may be distorted. Magnetic-based systems, on the other hand, are not dependent on the characteristics of a patient's anatomy and are considered to provide a higher degree of accuracy. However, magnetic position sensors generally are limited to tracking relatively fewer sensors.

Efforts have been made to provide a system that combines the advantages of an electrical impedance-based positioning system (e.g., positioning of numerous electrodes) with the advantages of a magnetic-field based coordinate system (e.g., independence from patient anatomy, higher accuracy). In an embodiment, such a system may be provided by registering the coordinate systems of an electrical impedance-based positioning system with the coordinate system of a magnetic field-based positioning system. In such an arrangement, locations of electrodes may be identified in an impedance-based coordinate system in conjunction with identifying the locations of one or more magnetic sensors in a magnetic-based coordinate system. In an embodiment, at least a portion of the electrodes and magnetic sensors may be co-located to define fiducial pairs. This co-location allows for determining a transformation (e.g., transformation matrix) between the coordinate systems. The transformation may be applied to the locations of any electrode to register these locations in the magnetic-based coordinate system once the transformation is determined. Accordingly, the electrical impedance-based electrodes can be identified in the coordinate system of the magnetic field-based positioning system thereby increasing the positioning accuracy for the electrodes. Such a system is set forth in co-owned U.S. Pat. Pub. No. 2013/0066193, as incorporated above.

While providing improved electrode positioning, the determination of a transformation between the impedance-based coordinate system and the magnetic based impedance system and subsequent registration of the electrode locations to the magnetic coordinate system can fail to account for various impedance shifts and/or drifts, associated with the electrode(s). That is, impedance-based systems can be subject to nonlinear shift and/or drift due to physiological phenomena. Along these lines, previous efforts have been directed to identify shifts and/or drifts and apply corrections to the registrations. Such a system is set forth in co-owned U.S. Pat. Pub. No. 2016/0367168. Generally, such a system determines a transformation between the impedance-based system and the magnetic-based system and applies a correction to the electrode locations.

The previous systems that utilize electrode information (e.g., impedance measurements) and magnetic sensor information to provide improved electrode positioning in three-dimensional space (e.g., within a body of a patient) rely primarily on impedance-based measurements. That is, the magnetic sensor information (e.g., magnetic sensor measurements) delivers additional accuracy. This may be described as an impedance-primary location arrangement. Due to the distortion and temporal instability of the impedance measurements, such an arrangement can suffer from instability. Further, the previous impedance-primary location arrangements, in some instances, fail to account for various errors within the system. By way of example, a transformation between the impedance-based coordinate system and the magnetic-based impedance system may underestimate error or uncertainty in the electrode and/or magnetic sensor measurements. By way of further example, such systems may fail to take into account other system inputs (e.g., patient movement, shape of the medical device, etc.), which may affect the calculated locations or positions of the electrodes. In summary, registration of an impedance-based system to magnetic-based system may fail to include additional information which may be observed and/or inferred and which may improve the overall identification of catheter and/or electrode positions in a three-dimensional space.

To provide an improved system for determining the locations of electrodes in a three-dimensional space such as within a body of a patient, the present disclosure is directed to a location arrangement (e.g., sensor fusion process or algorithm) that continuously integrates (e.g., fuses) impedance measurements from the electrodes and external patches with position and orientation measurements from magnetic sensors to estimate the latent state (e.g., position) of a medical device disposed within a patient reference frame. The latent state is used to track catheter electrodes within a body of a patient as though there were a magnetic sensor located at each catheter electrode, thereby achieving both accuracy and stability. More broadly, the presented arrangement expands the number of observed parameters utilized to locate the electrodes within a patient reference frame without relying on direct transformation between the impedance-based coordinate system and the magnetic-based impedance coordinate system based on the existence of fiducial pairs of electrodes and sensors. Fiducial pairs are not required by the systems and methods of the present disclosure. Rather, the impedance measurements and magnetic measurements are utilized as inputs to an overall system model that estimates/predicts and updates catheter electrode locations in a patient reference frame. Catheter and/or electrode locations may be tracked using both magnetic and impedance measurements.

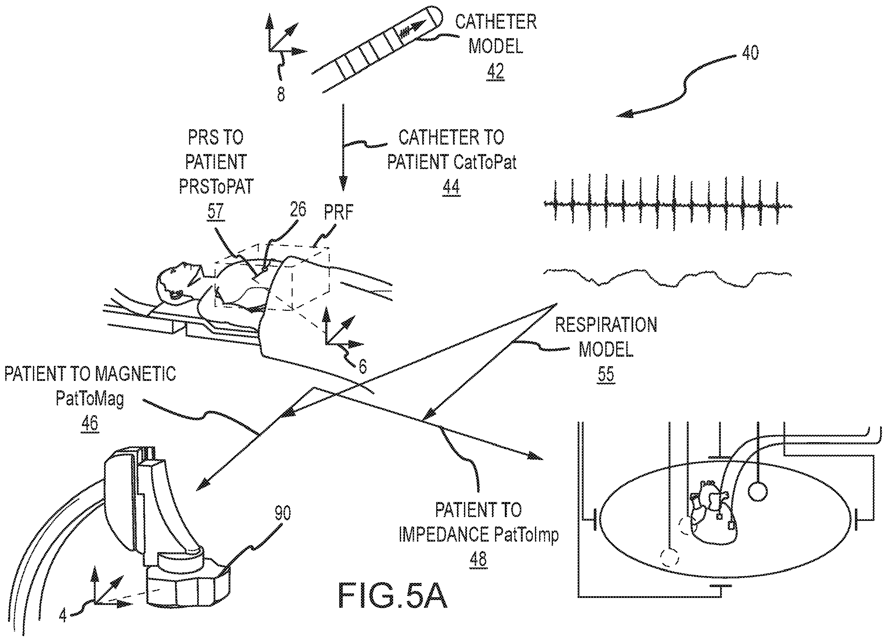

FIG. 5A illustrates an embodiment of independent models that are used to mathematically define a catheter and/or electrode location system model. That is, the independent models define a composite model 40 of the system (e.g., in the patient reference frame). The illustrated embodiment of the composite system model 40 includes five models: a catheter model 42 (e.g., medical device model) that predicts the shape (e.g., catheter configuration) of a catheter having one or more electrodes and magnetic sensors in a catheter frame of reference 8, according to the invention as defined by the appended claims; a catheter position and orientation model 44 that transforms the catheter model from the catheter reference frame 8 into the patient reference frame 6 based on a unique transformation that is specific to the catheter; a magnetic model 46 that predicts magnetic sensor measurements in the patient reference frame; an impedance model 48 that predicts electrode impedance measurements in the patient reference frame, according to the invention as defined by the appended claims; and a respiration model 55 that predicts artifacts in the predicted impedance and/or magnetic measurements based on patient respiration. Each model mathematically describes a portion of the overall system. However, it will be appreciated that not all models are required for the composite model. That is, the composite model may use different combinations of some or all of the models. In an embodiment, the magnetic model further includes a patient reference sensor model 57 that tracks adjustments of the position of the PRS 26 relative to the patient reference frame.

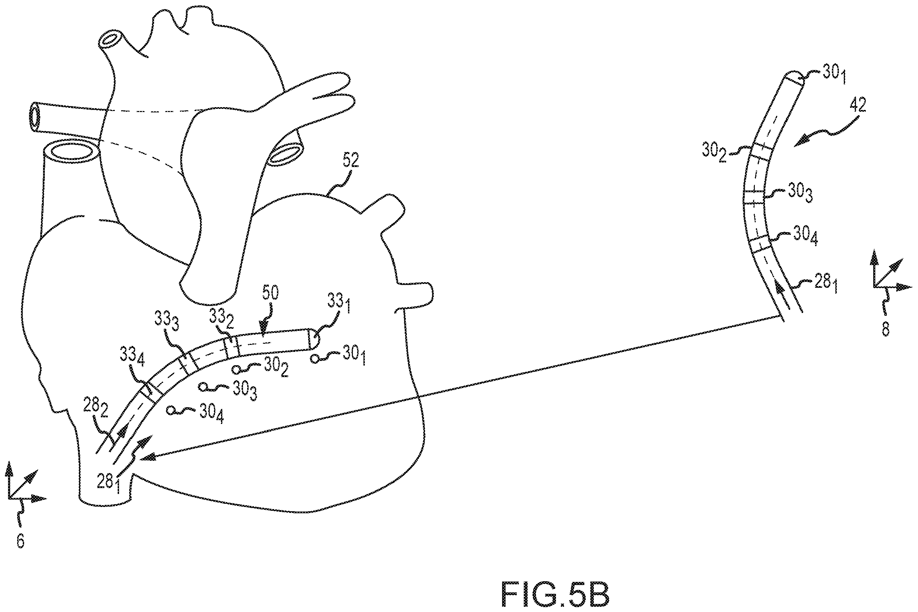

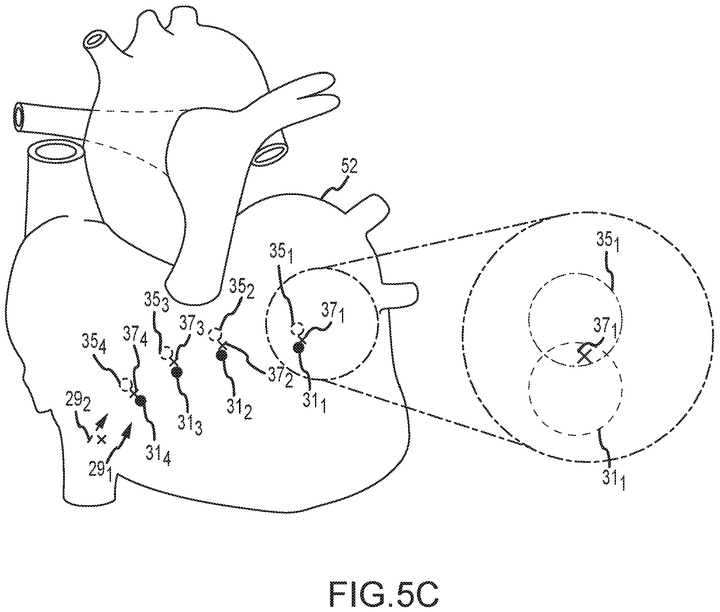

FIG. 5B further illustrates the cooperation various one of the models. Initially, the catheter model 42 predicts a catheter shape of a corresponding physical catheter 50 disposed within a patient reference frame, according to the invention as defined by the appended claims, (e.g., heart 52), where the physical catheter 50 has a set of electrode $33_1$-$33_4$ and a magnetic sensor $28_2$. The catheter shape model 42 includes model positions or locations of model electrodes $30_1$-$30_4$ and a model magnetic sensor $28_1$ which correspond to the physical electrode $33_1$-$33_4$ and magnetic sensor $28_2$ in a catheter reference frame 8. A position and orientation model 44 applies one or more transformations to the catheter model 42 to translate the model from the catheter reference frame 8 to the patient reference frame 6. Upon transformation, locations (e.g., predicted locations) of the model electrodes $30_1$-$30_4$ and/or model magnetic sensor $28_1$ are predicted (e.g., projected) in the patient reference frame 6, as illustrated by the solid circles for the electrodes $30_1$-$30_4$ and the vector for the magnetic sensor $28_1$ as shown located in the patient heart 52. According to the invention, the impedance model 48 predicts impedance responses or measurements $31_1$-$31_4$ for the predicted electrode locations of the model

electrodes $30_1$-$30_4$ in the patient reference frame while, in an embodiment, magnetic model 46 predicts a response or measurement for the predicted location of the model sensor $28_1$ in the patient reference frame. This is illustrated in FIG. 5C where the predicted electrode responses (e.g., locations) $31_1$-$31_4$ for each predicted model electrode location are represented by solid dots and the predicted magnetic measurement $29_1$ for the model sensor $28_1$ is represented by the solid vector. The impedance-based medical positioning system measures actual responses $35_1$-$35_4$ (e.g., observed measurements) of the physical electrodes $33_1$-$33_4$ within the patient body (e.g., patient reference frame) to an applied potential field to determine responses (e.g., locations) of the electrodes, as represented the dashed circles. If utilized, the magnetic-based medical positioning system measures the response (e.g., location) $28_2$ of the magnetic sensor in the patient body, as represented by the dashed vector $29_2$. As shown by the magnified portion of FIG. 5C, measured responses of the physical electrode(s) (e.g., $35_1$) and/or sensor(s) (not shown) and the predicted responses of the electrode (e.g., $31_1$) and or sensors (not shown) each contain some unknown error or noise (e.g., uncertainty). In an embodiment, the predicted responses include a respiration artifact from the respiration model 55. In an embodiment, the uncertainty of the measured responses and predicted responses may partially overlap. The predicted measurements and the observed measurements are then utilized to predict true (e.g., updated) or calculated locations of the electrodes $37_1$-$37_4$ as represented by the X's in FIG. 5C. As shown in the magnified portion of FIG. 5C, the calculated location $37_1$ may reside in the overlap of the predicted response location and the measured response location. In any embodiment, the calculated locations typically have a higher accuracy than locations resulting from either the predicted responses or the observed responses. The calculated locations is then output to a display. See, e.g., FIG. 1. That is, an updated representation or rendering of a catheter or other medical device is output to the display using the calculated locations.

Catheter model

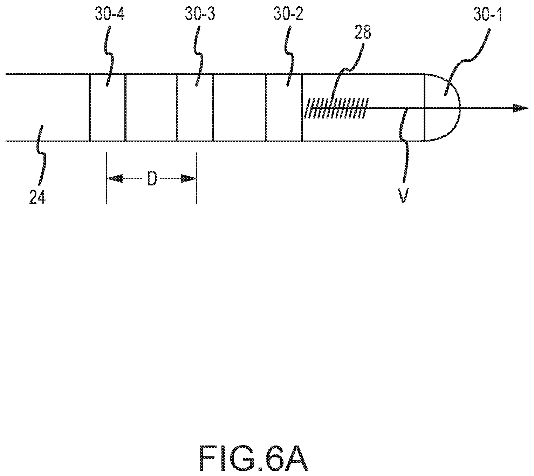

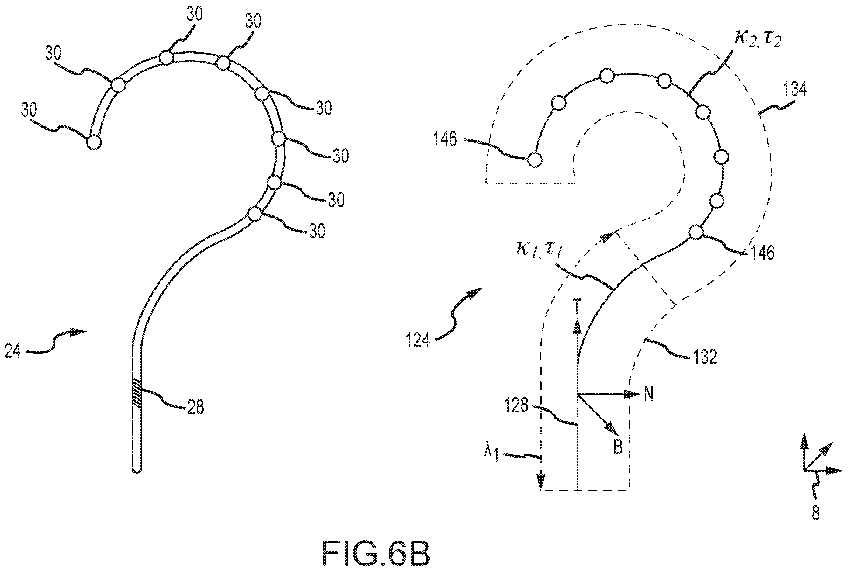

The following provides one simplified catheter model (i.e., FIG. 6A) that allows identifying locations of magnetic sensors and electrodes within a catheter reference frame. The model of FIG. 6A is directed to a rigid catheter with a single magnetic sensor and four electrodes having a known orientation relative to the magnetic sensor. However, it will be appreciated that other more complex catheter models are possible and such complex catheter models are further discussed in relation to FIGS. 6B-8D. As discussed below, more complex catheter models may provide for catheter deformations such that the model includes deformable sections (e.g., a small number of curvature and torsions along a Frenet-Serret reference frame) for use with a rigid-body transformation (e.g., a unit quaternion and translation) to describe the catheter shape, and/or position and orientation in the patient reference frame. In an example, catheter models for use in determining electrode locations in a catheter reference frame are described in U.S. Provisional Application No 62/756,915 titled "Mechanical Models of Catheters for Sensor Fusion Processes", filed on November 7, 2018.

Referring again to FIG. 6A, a side view of an exemplary medical device or catheter 24 is depicted where the catheter 24 has a single magnetic position sensor 28 and four electrodes 30-1, 30-2, 30-3, 30-4 (hereafter 30 unless specifically referenced). In order to reduce the complexity or reduce the dimensionality of the model (e.g., number of model parameters), it may be desirable to determine the position and orientation of the electrodes in the catheter reference frame as a function of the position of the magnetic sensor. For example, using specifications associated with the catheter (e.g., manufacturer specifications detailing the position of the electrodes 30 with respect to the magnetic position sensors 28), the locations of the electrodes in the catheter reference frame may be determined from the position of the magnetic sensor(s). For example, based on a position and orientation of a magnetic position sensor 28 (e.g., a five or six degree-of freedom sensor), a vector for the magnetic position sensor can be determined. In some embodiments, the vector can be in a direction facing towards the distal end of the magnetic position sensor 28 (e.g., magnetic coil) and can be coaxial with the magnetic position sensor 28. Because the magnetic position sensor 28 is disposed within a shaft of a rigid catheter, the position and orientation of the catheter shaft can be determined based on the vector associated with the magnetic position sensor. Specifications associated with a positioning of one or more electrodes 30 on the shaft with respect to the magnetic position sensor 28 (e.g., manufacturer specifications) can be used to determine model positions of the electrodes 30 in the catheter reference frame (i.e., along the vector). Accordingly, a model equation (e.g., state vector) may be determined that identifies the location of the sensor 28 and electrodes 30 in the catheter reference frame. That is, coupled with sensor location and electrode spacing, all electrode and sensor positions and orientations are known in the catheter reference frame.

FIG. 6B illustrates one embodiment of a deformable physical catheter 24 and corresponding catheter shape model 124. The deformable catheter 24 includes a single catheter spline, a plurality of electrodes 30 and a magnetic sensor 28. The catheter model, in the present embodiment, divides the spline into two model segments, a proximal shaft segment 132 and a distal hoop segment 134. Each segment 132, 134 is described by a moving Frenet frame of constant parameters that follows an arc of the corresponding segment of the physical catheter. Model electrodes 146 are located on distal hoop model segment 134 according to mechanical specifications. For example, the position of each electrode may be defined by its distance or length $l$ along the length $\lambda$ of Frenet frame (e.g., from an origin of the frame). In the present embodiment, all electrodes are shown as being located on the distal hoop segment 134, however, it will be appreciated that each model segment may include electrodes, depending on the physical configuration of the physical catheter 24. In the present embodiment, the proximal shaft model segment 132 includes a single model magnetic sensor 128. Again, it will be appreciated that each model segment may include one or more magnetic sensors and/or one or more electrodes. The parameterization of the model segments thus fully describes the electrode locations in a catheter reference frame 8 of the catheter model 124.

Frenet formulas describe the geometric properties of a continuous, differentiable curve in three-dimensional space. More specifically, the Frenet formulas describe the derivatives of the tangent 'T', normal 'N", and binormal 'B' unit vectors in terms of one other along at each point along the length λ of the frame. See Fig. 6B. The tangent, normal, and binormal unit vectors, or collectively the Frenet frame are defined where T is the unit vector tangent to the curve, pointing in the direction of motion, N is the normal unit vector, the derivative of T with respect to the arc length parameter of the curve, divided by its length and B is the binormal unit vector, which is the cross product of T and N. The Frenet Formulas are:

$$\frac{dT}{ds} = \kappa N$$

$$\frac{dN}{ds} = -\kappa T + \tau B$$

$$\frac{dB}{ds} = -\tau N$$

where $d/ds$ is the derivative with respect to arc length, $\kappa$ is the curvature (e.g., inverse or radius of a curve), and $\tau$ is the torsion of the curve. The two scalars $\kappa$ and $\tau$ effectively define the curvature and torsion of a curve. For each segment in a homogenous coordinate system, the Frenet frame ($F_F$) for a curve defined by $\kappa$ and $\tau$ at a distance λ along the curve is defined as:

$$F_F = \begin{bmatrix} 0 & \kappa & 0 & 0 \\ -\kappa & 0 & \tau & 0 \\ 0 & -\tau & 0 & 0 \\ 0 & 0 & 0 & 0 \end{bmatrix} \lambda$$

Utilization of the Frenet frame effectively permits defining each model segment utilizing two parameters curvature $\kappa$ and torsion $\tau$.

In the embodiment of FIG. 6B, the catheter shape model includes two continuous curves (e.g., model segments) of constant curvature and torsion rotated 90 degrees from one another. The first curve represents the bend between the proximal shaft segment 132 and the distal hoop segment 134. The first curve is defined by $\kappa_1$ and torsion $\tau_1$. The second curve represents the distal hoop segment 134. The second curve is defined by $\kappa_2$ and torsion $\tau_2$. Thus, the catheter shape model 124 is defined by four parameters: two curvatures and two torsions, which define all possible shapes that the catheter model may take. These parameters each typically have a predetermined or experimentally determined numerical range (e.g., from a corresponding physical catheter). Further, the curve parameters typically form state variables in a stochastic process that predicts potential shapes of the catheter model. Locations of model electrode and/ or magnetic sensors may be derived by their known locations along their respective frame for a given model.

In an embodiment, state transition models (e.g., matrixes) , which apply the effect of each curve parameter at time k-1 to the curve parameters at time k are as follows:

$$f(x)_i = \kappa_{i(k-1)} + \hbar_{i-curve}\left(\hbar_i - \kappa_{i(k-1)}\right)$$

$$f(x)_i = \tau_{i(k-1)} + \hbar_{i-torsion}\left(t_i - \tau_{i(k-1)}\right)$$

where:

$i$ represents the curve segment (e.g., $i$ = 1 or 2 in the present embodiment);

$\mathcal{k}$ represents a default curvature for each curve segment;

$\mathcal{t}$ represents a default torsion for each curve segment; and

$\mathcal{f}$ represents a matrix defining the forcing factors for each curve parameter.

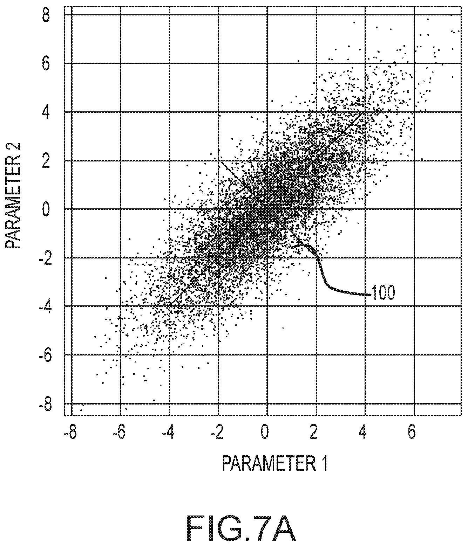

The transition matrix when applied, varies each of the state variables to generate a plurality of possible catheter shapes. In an embodiment, this produces a state distribution of possible catheter shapes. See FIG. 7A. Typically, the mean of the state distribution represents the most likely catheter shape and corresponding set of catheter parameters.

In an embodiment, the forcing factor(s) may be derived from catheter specific mechanical parameters. In an embodiment, the forcing factor may represent the returning force of a shape metal wire that forms the spline of the catheter. In such an embodiment, the forcing factor $F$ applies a returning force to a deformation associated with the given shape parameters that represents the force applied by the shape metal wire attempting to return to an un-deformed or nominal state from a current shape parameter. In an embodiment for a single-segment catheter which is nominally straight and untwisted with the same torsional stiffness as the rotational stiffness:

$$\text{let } x_k = \begin{bmatrix} \kappa_{1(k)} \\ \tau_{1(k)} \end{bmatrix}$$

$$F = \begin{bmatrix} 1 - \mathcal{f}_1 & 0 \\ 0 & 1 - \mathcal{f}_1 \end{bmatrix} = \begin{bmatrix} 0.99 & 0 \\ 0 & 0.99 \end{bmatrix}$$

$$x_{\text{default}} = \begin{bmatrix} \mathcal{k}_1 \\ \mathcal{t}_1 \end{bmatrix} = \begin{bmatrix} 0 \\ 0 \end{bmatrix}$$

$$x_k = f(x_{k-1}) = F x_{k-1} + x_{\text{default}} \mathcal{f}_1$$

As will be appreciated, such a forcing factor $F$ is unique for a specific catheter. The inclusion of the forcing factor prevents the state transition model from being identity to a prior state.

Through application of the above noted transition models, a state distribution of potential catheter shapes, in the catheter frame of reference, may be estimated for time k. FIG. 7A illustrates one exemplary distribution. Based on the most likely shape (e.g., the mean of the distribution), the locations of the model electrodes may be determined in the catheter frame of reference.

An observational model may be implemented to map the state parameters into a physical domain (e.g., catheter frame of reference). In an embodiment, this is performed by evaluating the matrix exponential for the Frenet Frame. In an embodiment, the matrix exponential is an integrated differential matrix with constant terms (e.g., curvature and torsion) over the arc length for all electrodes where the position $l$ of the electrodes varies over the arc length. In an embodiment, the matrix evaluation may be computed using a Givens rotation and trigonometric functions.

In an embodiment, a Given's rotation is initially computed to eliminate two terms of the Frenet Frame:

$$\theta = \sqrt{\kappa^2 + \tau^2}$$

such that:

$$G(\kappa,\tau) = \begin{bmatrix} \dfrac{\kappa}{\theta} & 0 & \dfrac{\tau}{\theta} & 0 \\ 0 & 1 & 0 & 0 \\ \dfrac{-\tau}{\theta} & 0 & \dfrac{\kappa}{\theta} & 0 \\ 0 & 0 & 0 & 1 \end{bmatrix}$$

After expanding the first several terms of the remaining matrix exponential, the following trigonometric series identities can be recognized:

$$\Phi(\kappa,\tau,\ell) = G(\kappa,\tau)\begin{bmatrix} \cos(\theta\ell) & \sin(\theta\ell) & 0 & 0 \\ -\sin(\theta\ell) & \cos(\theta\ell) & 0 & 0 \\ 0 & 0 & 1 & 0 \\ \dfrac{\kappa}{\theta^2}\sin(\theta\ell) & \dfrac{\kappa}{\theta^2}(1-\cos(\theta\ell)) & \dfrac{\tau\ell}{\theta} & 1 \end{bmatrix}G^T(\kappa,\tau)$$

Where Φ is a transformation from the state space to the catheter frame of reference. For the full Φ matrix, it is useful to leave the Given's rotations in the solution. However, the last row, which contains the Cartesian coordinate for a given arc length along the curve, is evaluated for each electrode on the hoop:

$$P(\kappa,\tau,\ell) = \begin{bmatrix} \dfrac{\kappa^2}{\theta}\sin(\theta\ell) + \tau^2\ell & \kappa(1-\cos(\theta\ell)) & -\dfrac{\kappa\tau}{\theta}\sin(\theta\ell) + \kappa\tau\ell & \theta^2 \end{bmatrix}\dfrac{1}{\theta^2}$$

Where P is a coordinate at position $\ell$ of the Frenet Fame. The model electrodes and/or coil(s) are then identified in the catheter reference frame by computing the arc length along a specified curve for each electrode, computing $P$ as above and composing it with any Φ which may be more proximal.
For the model electrodes on the distal hoop (e.g., subscript 2 in the current embodiment) for the model of FIG. 6B:

$$C_i = P\left(\kappa_2, \tau_2, \lambda_2 - \sum_{i'=1}^{i-1}\Delta_{i'}\right)\Phi_h\Phi_1(\lambda_1)$$

Where:

$C_i$ is the position of each electrode in the catheter frame of reference;
$\lambda_2$ is the length of the distal hoop curve;
$\Delta_{i'}$ is the intra-electrode distance specification (e.g., center to center);
$\Phi_h$ is a transformation between the curves of the first and second Frenet Frames to provide smoothing, and which in an embodiment where the first and second frames have a 90 degree clockwise rotation is:

$$\Phi_h = \begin{bmatrix} 1 & 0 & 0 & 0 \\ 0 & 0 & 1 & 0 \\ 0 & -1 & 0 & 0 \\ 0 & 0 & 0 & 1 \end{bmatrix}$$

$\Phi_1$ is the transformation for the distal hoop curve; and
$\lambda_1$ is the length of the proximal shaft curve.

For the magnetic sensor or electrode (if present) on the proximal shaft of the two segment catheter model of FIG. 6B:

$$C_i = \left[ \sum_{i'=1}^{2} \lambda_{i'} - \sum_{i'=1}^{i-1} \Delta_{i'} \quad 0 \quad 0 \quad 1 \right]$$

Once the positions of the model electrodes and/or magnetic sensors are known in the catheter reference frame, they may be transformed into the patient reference frame using any appropriate transformation. In an embodiment, a six degree of freedom rigid transformation is utilized to orient the catheter model locations of the electrodes and magnetic sensor into the patient reference frame based on the position and orientation of the magnetic sensor relative to a position and orientation of a magnetic patient reference sensor. For each model electrode position in the patient reference frame, impedance measurements may be predicted and impedance measurements may be obtained (e.g., observed) from the physical electrodes. The predicted measurements and observed measurements may be utilized to update the parameters of the catheter model to more closely approximate a physical configuration of the deformable catheter.

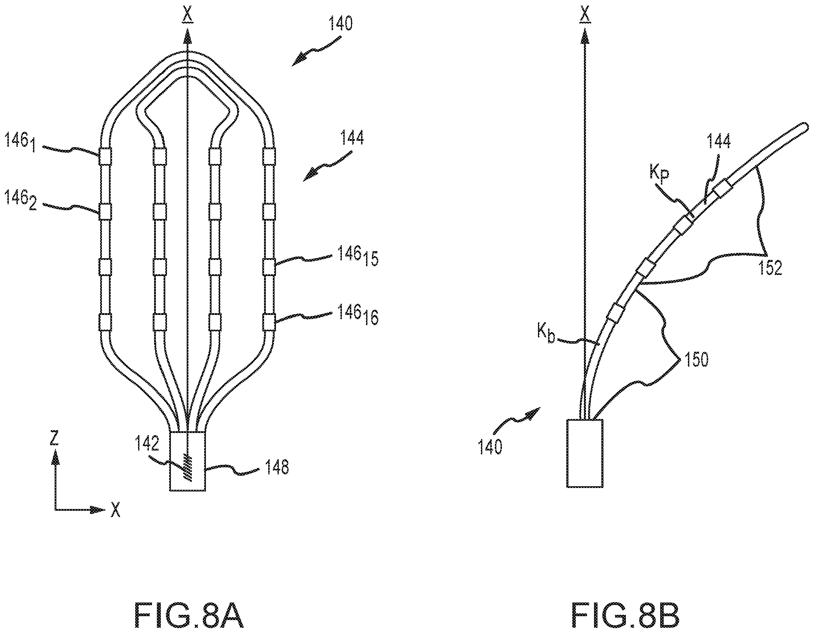

While previously discussing the modeling of a relatively simple single spline catheter, it will be appreciated that more complex catheters may be modeled based on a limited set of parameters. FIGS. 8A-8D illustrate a planar catheter 140 having a substantially rigid shaft 142 having one or more magnetic sensors 148 and one or more electrodes (not shown) and a flexible paddle 144, which includes sixteen electrodes $146_{1-16}$ (hereafter 146 unless specifically referenced) arranged in a square matrix. In an embodiment, the planar catheter 140 corresponds to the HD Grid Catheter commercially available from Abbott Laboratories of Lake Bluff, Illinois, United States. In the illustrated embodiment, the flexible paddle 148 is defined by four shape metal wires, which each support four electrodes 146. In a non-deflected or relaxed state, the flexible paddle 144 is substantially planar in the XZ plane with an origin at the end of the rigid shaft. A reference axis $\underline{x}$ extends from the origin longitudinally, for example, in axial alignment with the rigid shaft. In an embodiment, the catheter 140 is modeled as a curving plane with two model segments (proximal and distal). In addition to curvature, the axis of the distal segment's curvature may be rotated to capture off-axis deformations and both segments may be rolled laterally. The three-dimensional locations of electrodes may then be determined by the two-dimensional location on the curved plane. Further, the locations of electrode and/or sensors may be defined along the length of the various planes.

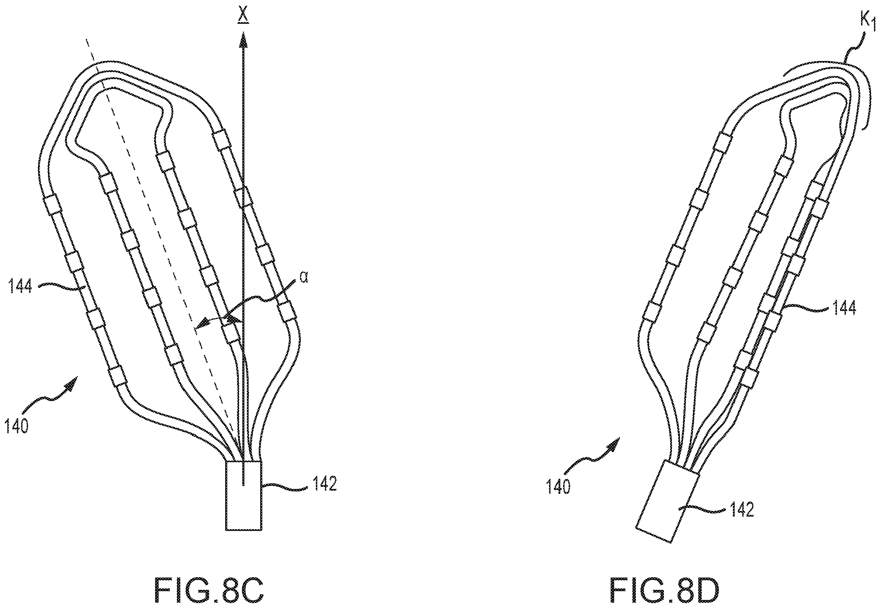

In an embodiment, the planar catheter is modeled by four parameters, a base curvature, a paddle curvature, a slanting angle and a tube curvature. Such parameters relate to physical actions that may occur during the course of a clinical procedure that would cause the catheter 140 to take a particular shape (e.g., deform). For instance, during a cardiac procedure the catheter 140 typically presses against a cardiac wall and/or is pushed into a lumen (e.g., blood vessel, artery). Pressing against a cardiac wall typically results in a change in the base curvature and paddle curvature from the relaxed state where the paddle 144 is displaced from the reference axis $\underline{x}$ as shown in the side view of FIG. 8B. Additionally, pressing against the sidewall may result in a slanting of the paddle 144 relative to the reference axis $\underline{x}$ as shown in FIG. 8C. Finally, displacing the catheter in a lumen may result in a cylindrical curvature along the length of the paddle 144 as shown in FIG. 8D.

The base curvature $\kappa_b$ and paddle curvature $\kappa_p$ are best shown in FIG. 8B. As shown, the base curvature $\kappa_b$ corresponds to the curvature (e.g., inverse of radius) of the proximal segment 150 of the paddle 144 while the paddle curvature $\kappa_p$ corresponds to the curvature (e.g., inverse or radius $R_1$) of the distal segment 152 of the paddle 144. Through experimentation, it has been determined that, for the corresponding physical catheter, upright pressure on the paddle 144 (e.g., applied to the distal tip without slanting) results in the proximal segment 150 curving in a consistent manner. Accordingly, the base curvature $\kappa_b$ may be expressed by a single curvature parameter having a value range (e.g., $\pm 0.25$) that may be established based on expected curvatures or determined through experimentation. Typically, the proximal segment 150 of the paddle 144 is less rigid than the distal segment 152 of the paddle upon application of the same pressure. However, the curvature of the two segments are related. In an embodiment, the relationship between paddle curvature $\kappa_p$ and base curvature $\kappa_b$ can be expressed as:

$$\kappa_p \approx c \bullet f(\kappa_b)$$

where the functional factor $f(\kappa_b)$ is positive. This relationship may be determined through experimentation where a number of paddle deformations are examined (e.g., in benchtop testing) to determine the shape or range of the base curvature. In an embodiment, a plot of the relative values of base curvature $\kappa_b$ and paddle curvature $\kappa_p$ may be prepared such that. for example, a best fit curve may define the relationship of the parameters. In an embodiment, the relationship between these curvatures for the specific catheter was found to be:

$$\kappa_p \approx c \bullet f(\kappa_b) = c \bullet c_1 \arctan(c_2 \, \kappa_b)$$

where c1 and c2 are experimentally determined constants.

FIG. 8C illustrates a slanting angle applied to the paddle. When upright pressing is exerted on a device, it can be imagined as wrapping around a generalized cylinder with lateral axis (i.e. perpendicular to the rigid shaft direction). A slanted pressing also results .in a generalized cylindrical surface, but its axis is not lateral, but rather has some angle $\alpha$. The slanting angle may have an established range (e.g, $\pm 45°$). FIG. 8D illustrates the curvature or tube curvature $\kappa_b$ along the length of the paddle. By way of example, when the catheter is pushed into a lumen, the paddle attains a tubal shape, with curvature that is generally transverse to the longitudinal axis or reference axis $\underline{x}$ of the catheter. The catheter model utilizes the four noted parameters to define all possible shapes (e.g., states) that the planar catheter 140 may assume. Again, these parameters may define state variables in a stochastic process that predicts potential shapes of the model. Accordingly, based on the known spacing of the electrodes, their position may be determined for a possible state in the catheter reference frame in a manner similar to that described above.

The catheter models may be implemented to estimate shapes or states of a catheter as part of a stochastic process. In such an arrangement, a catheter model may be used to predict or estimate a current shape of a catheter and thereby the locations of electrodes in a catheter reference frame based on a previous known shape of the catheter. In such an arrangement, a shaping function may be applied to adjust each set of model parameters (e.g., previous curvatures, torsions, slant angles etc.) to estimate new potential catheter shapes. In such an arrangement, the model parameters may form hidden state variables and, in an embodiment, an Extended Kalman filter or other estimator may be used to estimate these hidden state variables to predict catheter shapes. In such an arrangement, a state distribution of all possible catheter shapes may be generated and transformed from the catheter reference frame to the patient reference frame to predict electrode locations within the patient reference frame. Predicted electrode measurements (e.g., from an impedance model) associated with the predicted locations of the electrodes in the patient reference frame (e.g., from the catheter model) may be utilized with actual electrode measurements in the patient reference frame to update a set of shape parameters associated with the updated shape estimate. This may allow identifying a true catheter shape and electrode locations in the patient reference frame.

When estimating or predicting the shape of a catheter described by a small number of parameters, it has been recognized that the shape estimation is over-determined. That is, the state distribution of predicted catheter shapes based on the shape parameters may include shapes that, while possible, are not likely. For instance, the loop catheter of FIG. 6B may be straightened by setting all curvatures to zero or the planar catheter of FIGS. 8A-8D may be rolled into a tight loop by when tube curvatures are set to large values. Neither condition is likely. Further, the electrode measurements all contain some error such that there is no combination of shape parameters that exactly reproduces the predicted or observed electrode measurements. Accordingly, it would be beneficial to eliminate unlikely states from the estimate to improve overall accuracy of the process.

In an embodiment, the present disclosure describes a technique for pruning the parameter space to physically achievable states by determining the likelihood of a set of shape parameters. More specifically, a likelihood function is applied to an estimated state distribution of the catheter shape model to exclude unlikely states from the estimated state distribution. This results in biasing the estimator towards more likely parameters.

In an embodiment, the likelihood function may be determined experimentally by deforming a physical catheter associated with a catheter model under constant force and computing the energy associated with a set of shape parameters for each shape of the catheter. The stored energy may then be used as proportional to the negative log likelihood of an associated set of shape parameters for particular catheter shape. Along these lines, it is recognized that many catheters have one or more shape memory wires or splines that, when deformed, attempt to return to a nominal or original configuration. By way of example, the planar catheter discussed above may return to the planar configuration once a deforming force is removed from the catheter. Accordingly, the energy stored in the catheter when bent may be assumed to be proportional to the likelihood of the deformation. When a catheter is deformed by pushing it against a structure, it may be assumed that the catheter will adopt the lowest-energy configuration. For example, for a deformation in response to an obstacle, if a lower energy configuration can produce the same measurements, the catheter will be in the lower energy configuration. Thus, it follows that the energy of a deformation is proportional to the likelihood of the corresponding set of shape parameters.

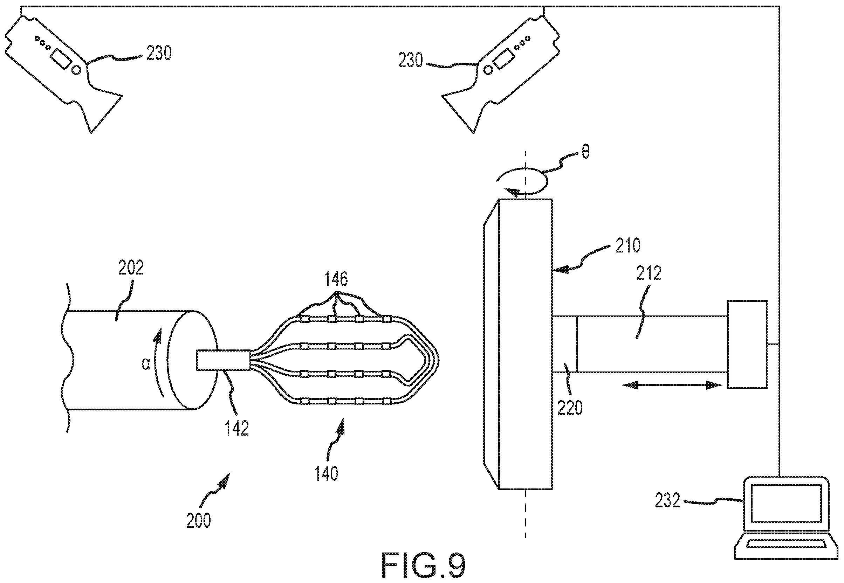

FIG. 9 illustrates a testing system 200 for experimentally determining the energy of deformation of a catheter 140 in a bending procedure. As shown, the system has support or collet 202 that receives and holds the shaft 142 of the catheter 140 in a known orientation, a movable sled 210 and actuator 212 that displaces a distal end of the catheter, a force sensor 220, one or more cameras 230 and a controller 232. The system is utilized to apply deformations of known magnitude and/or displacements and record the locations of the catheter electrodes 146 in 3D space.

The collet 202 holds the catheter shaft at a desired roll angle $\alpha$. In an embodiment, the collet is configured to rotate about an axis that is substantially co-axial with the longitudinal axis of the catheter shaft. Thus, the collet may rotate to any desired roll angle $\alpha$. During each bending procedure, the catheter shaft may be maintained at a predetermined fixed roll angle. The movable sled 210 is moved into contact with the distal end of the catheter at an angle theta $\Theta$ (e.g., contact angle) to the longitudinal axis of the catheter 140. The sled 210 is attached to an actuator 212 through the force sensor 220. The sled 210 is then controllably displaced by the actuator 212 which is configured to maintain a force set point (e.g., via PID control). The sled 210 is displaced toward the catheter until it contacts the distal end of the catheter at a known angle theta Θ and the force set point is achieved. Once an initial force set point is achieved, the sled may be additionally displaced for additional force set points. For each advancement or displacement of the sled and force set point, the catheter is bent or deformed. By recording the displacements and the forces, these may be integrated to compute the energy stored in the catheter. Similar processes may be provided where the distal end of the catheter is displaced (e.g., pushed) into a lumen of a known size and orientation.

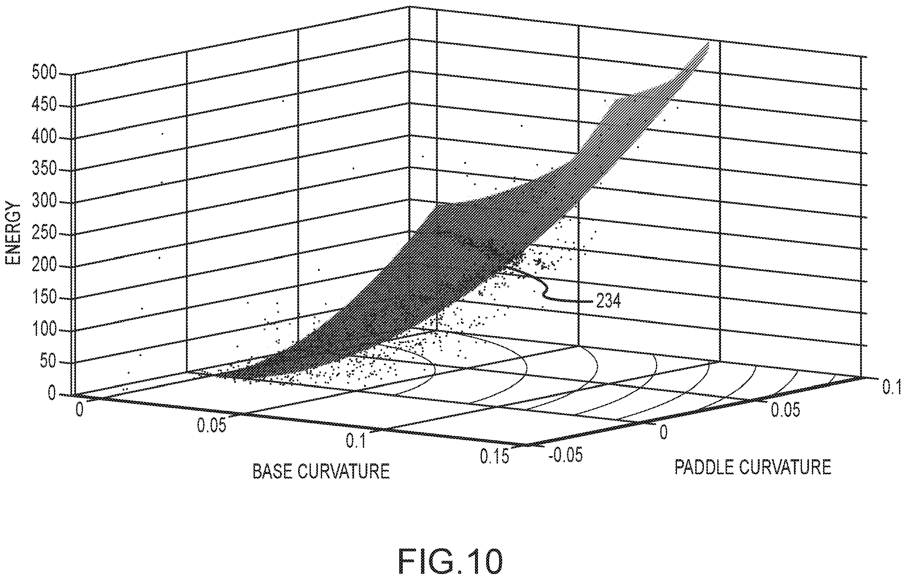

This bending procedure is conducted in a calibrated multicamera system. That is, cameras 230 identify the position of each electrode 146 such that a low-error 3D coordinate of each electrode is determined for each deformation. The controller 232, for each deformation, utilizes the coordinates from the cameras, the angular information from the collet and sled, the forces and the displacements to determine corresponding shape parameters (e.g., curvatures, slanting angles etc.). In an embodiment, a nonlinear least-squares minimization of the shape, position and orientation parameters is used to find the shape parameters associated with a particular deformation. By iterating the constant force displacement over the permutations of alpha, theta and force, samples of the shape/energy landscape are acquired. Curves describing the energy as a function of the shape parameters can then be fit to the samples. FIG. 10 shows an example over the curvatures of the proximal and distal plane segments of the catheter 140. Numerous curves may be generated for any given catheter.

In an embodiment, the experimentally determined curves are the basis of the likelihood function r(x). The likelihood function is used to regularize an estimated state distribution. Generally, the likelihood function describes the plausibility of a state (e.g., catheter shape). In an embodiment, a negative log likelihood is utilized. In such an embodiment, impossible states have a negative log likelihood of infinity and the most likely state has the minimum negative log likelihood. To apply this regularization, in an embodiment, a probability density function (regularizing PDF) is computed by negating, exponentiating and normalizing the negative log function. The estimated state distribution is then multiplied by the regularizing PDF and renormalized to create a regularized state distribution that omits unlikely states (i.e., states outside the combination of the state distribution and the regularizing PDF).

In an embodiment, the log likelihood function may be approximately applied through a second-order Taylor series expansion of the negative log likelihood function at the mean of the estimated state distribution to create a probability density function. In an embodiment, the approximation of the negative log likelihood function may be made via the following equation:

$$-\ln r(x) \cong -\ln r(x') - \mathcal{L}(x - x') - \frac{1}{2}(x - x')^{T}\mathcal{H}(x - x')$$

Where the Hessian $\mathcal{H}$ of the second order expansion is treated as the inverse of the covariance, with the Gaussian mean given by the multiplication of the Jacobian of the second-order expansion by the inverse of the Hessian. This approximation is equivalent to a Guassian PDF, which can be multiplied with the state distribution by well understood means.

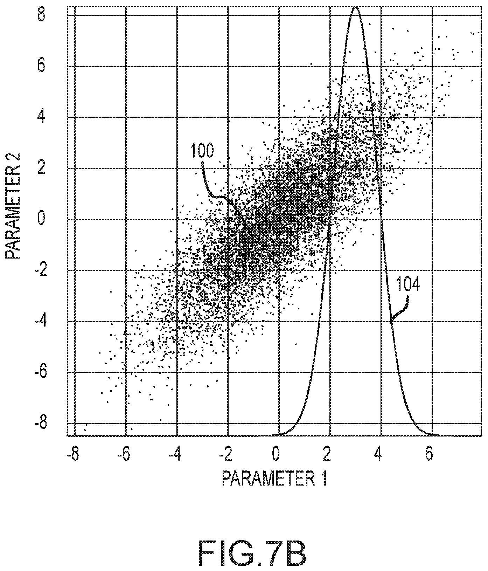

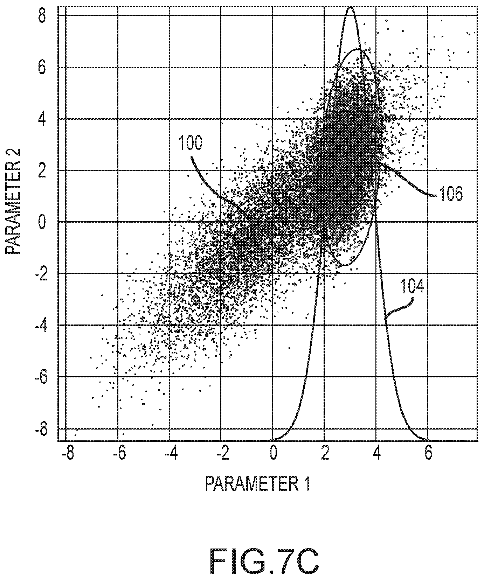

The regularization of a state distribution estimate is graphically illustrated in FIGS. 7A-7C. Specifically, FIG. 7A shows the state distribution 100 of possible catheters shapes predicted by a catheter model. FIG 7B shows the regularization PDF 104 applied to the state distribution. FIG. 7C illustrates the regularized state distribution 106, which is generally enclosed by a dashed circle for purposes of illustration. As will be appreciated, the regularized state distribution excludes unlikely states from the initial state distribution estimate. This results in a new or updates state distribution (e.g., regularized state distribution) having an updated mean and an updated covariance. Stated otherwise, the regularization process results in a tighter state distribution that more accurately predicts the true state of the system.

Catheter position and orientation model

For any catheter model having a magnetic sensor, the magnetic sensor will typically define a vector having six degrees-of-freedom. Three degrees-of-freedom for position (i.e., x, y, z), which may define an origin model in the catheter reference frame as defined by one or more magnetic sensors of the model, and three degrees of freedom for orientation (i.e., yaw, pitch, roll). The three degrees of freedom for the orientation may define a 3D bivector ($b_{yz}$, $b_{zx}$ and $b_{xy}$), which is the log of the quaternion. The catheter shape model may be transformed into the patient reference frame utilizing a transformation (e.g., catheter transformation) that preserves shape and size of the catheter model. That is, catheter position and orientation model may be represented by a rigid-body transformation (e.g. six degree of freedom rigid-body translation) that translates the vector (e.g., state vector) of the shape model into the patent reference frame. For instance, such a transformation may align the origin and orientation of the catheter model (e.g., vector in an embodiment) relative to the origin of the patient reference frame (e.g., as determined by the patient reference sensor). At such time, the locations of the magnetic sensor and electrodes are known or estimated within the patient reference frame. Of note, the origin of the patient reference frame as well as the origin of the catheter reference frame may shift due to patient motions (e.g., respiration, physical patient movement, etc.). Accordingly, the transformation and registration between the patent reference frame and the catheter reference frame may be updated.

PRS Model and Magnetic Model

In an embodiment, a magnetic patient reference sensor model or PRS model (e.g., PRStoPat) is used to describe the displacement of the patient reference sensor(s) relative to a patient reference frame 6. As will be appreciated, magnetic measurements are produced in a static magnetic-based coordinate system or magnetic reference frame 4, which typically has an origin based on a magnetic field generator. To eliminate patient movement relative to the static magnetic reference frame, patient movement may be monitored by a magnetic positional patient reference sensor (PRS) 26. See FIG. 5A. Using this approach magnetic measurements from a medical device are transformed to the patient reference frame. This method is prone to changes in displacements of the positional reference sensor relative to the patient body. These displacements may be caused by the movements of the patient skin due to respiratory or cardiac motion, stretching of the body, patient talking, etc. An uncompensated displacement of the PRS 26 leads to a shift of magnetic measurements and an increased inaccuracy of medical device locations. In an embodiment, the present disclosure describes a PRS model accounting for magnetic location changes due to the displacements of the PRS 26. The magnetic PRS model may be used to describe the displacement of the PRS(s) by means of a hidden state vector. That is, the model is defined as a stochastic process where a true state of the model, which is a hidden or latent state, is determined.

The PRS model includes a position and orientation of a one or more positional reference sensors (e.g., patient reference sensors) defined in the patient reference frame, where each patient reference sensor has a further 6 degrees of freedom (e.g., a 3D position and a 3D orientation), which are also expressed by state variables of the sensor fusion algorithm. The model allows tracking of motion of the patient reference sensor(s) relative to the patient. This motion can be modeled as having error of a small magnitude. Consistent placement of patient reference sensor(s) on the patient allows at least one of them to be considered as being at a specified initial translational offset from the origin of the patient frame. For example the patient origin can be placed at or within a typical heart location. Alternatively, a patient origin may be defined by a patient reference sensor.

A magnetic model (PatToMag) defines a rigid transformation from a patient coordinate frame (e.g., patient reference frame) to a magnetic field generator frame, which can vary over time. In an embodiment, the magnetic model defines an initial transformation between a location in the patient reference frame (e.g., origin) and an origin of the magnetic reference frame. In an embodiment, the magnetic model permits predicting magnetic values for locations in the patient reference frame. The magnetic model has 6 degrees of freedom, expressed by state variables of a sensor fusion process or algorithm. The magnetic model allows for tracking patient motion in substantially real time. At the start of a procedure, the patient typically lies in a consistent orientation on a surgical table, which is at or near a known orientation relative to the magnetic field generator. Therefore, a fixed orientation of the magnetic field generator (e.g., magnetic reference frame 4) relative to the patient reference frame can be assumed for an initial state.

The purpose of the models is to determine a time-variable three-dimensional Patient to Magnetic model/transformation at any time k (i.e., $PatToMag_k$). This transformation, at any given time, provides a rigid translation from the patient reference frame to the magnetic reference frame (e.g., field generator frame). The time variable transformation allows for transforming locations in the patient reference frame to the magnetic reference frame and periodically or continuously updating these locations in response to patient movement. Broadly, the models allow for representing one frame of reference in another frame of reference such that a point in one frame at a given time (e.g., patient reference frame) may be identified in the coordinates of the other frame (magnetic reference frame) at that time. In this regard, a first frame of reference can be represented in terms of a second frame of reference by applying a transformation matrix M. That is a set of resulting coordinates (e.g., position and orientation) in one frame of reference are a function of the coordinates (e.g., position and orientation) in another frame of reference. In a generalized equation:

$$\begin{bmatrix} x' \\ y' \\ z' \end{bmatrix} = M \begin{bmatrix} x \\ y \\ z \end{bmatrix}$$

where the column vector [x, y, z] is a location in the patient reference frame, the matrix M represents the $PatToMag_k$ matrix, the column vector [x', y', z'] are predicted measurements of the location in the magnetic reference frame. This relationship may implemented in a stochastic process such that the $PatToMag_k$ transformation/model may be used to predict magnetic measurements for predicted locations of magnetic sensors (e.g., from a medical device model) in the patient reference frame. Such predicted measurements may be updated or corrected based on subsequent observations. For instance, actual magnetic measurements for a magnetic sensor at or near a predicted location may be obtained from the medical positioning system 22. The actual measurement may be utilized with the predicted measurements to determine a true location of a magnetic sensor in a patient reference frame. Further, the actual measurements and the predicted measurements may be utilized to update the matrix M, which predicts the values for locations in the patient reference frame.

The model utilizes a patient reference sensor to establish a reference position in the patient frame of reference. As the patient reference sensor moves with a patient, the $PatToMag_k$ model must evolve over time to account for such patient movement. By way of example a transformation may be performed by:

$$\begin{bmatrix} x' \\ y' \\ z' \end{bmatrix} = \begin{bmatrix} a & b & c & t_x \\ d & e & f & t_y \\ g & h & i & t_z \end{bmatrix} \begin{bmatrix} x \\ y \\ z \\ 1 \end{bmatrix}$$

where positional values further use of a 1 or 0 in the last entry of the column vectors to represent a point/position or a direction, respectively. In the embodiment where the process is stochastic process, the variables of the matrix (e.g., a-i) are allowed to evolve over time.

In the present embodiment, the models have the following relationships:

$$pat_k = PRSToPat_{i,k} ref_k$$

and:

$$mag_k = PatToMag_k pat_k$$

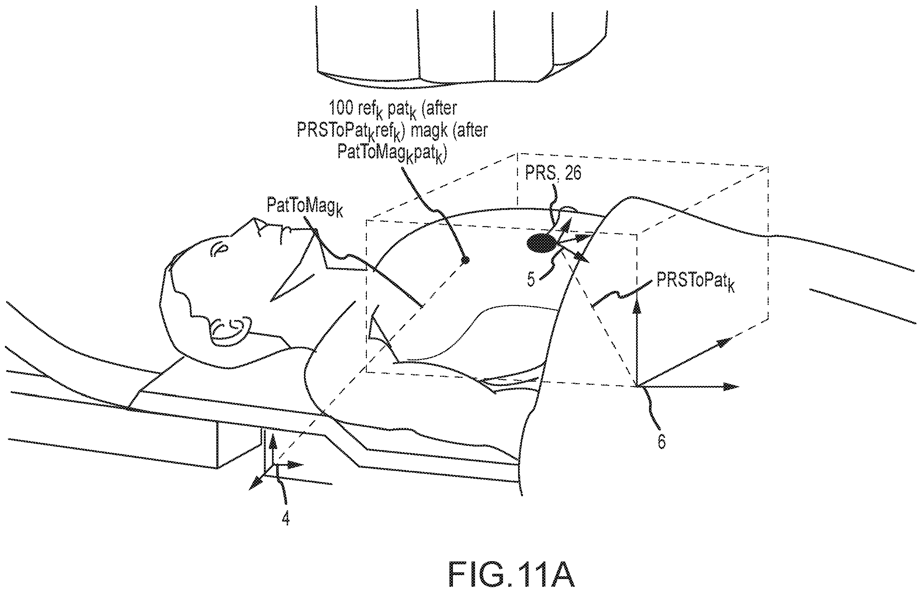

where $ref_k$ is a coordinate 100 in the patient body (e.g., reference sensor space) at time k (e.g., prior to alignment with the patient reference frame to account for patient movement), $pat_k$ is the value of the coordinate in patient reference frame at time k (e.g. patient frame coordinate), and $mag_k$ is the value of the coordinate in the magnetic reference frame at time k (e.g., $pat_k$ after being transformed from the patient reference frame to the magnetic reference frame). This is illustrated in FIG. 11A. In an embodiment, the patient reference sensor to patient transformation may be denoted as $PRSToPat_{i,k}$ where the subscripted i represents a particular patient reference sensor (e.g., i=1, i=2, i=n etc.). This subscript may be omitted in the case of a single patient reference sensor. These relationships provide a means for predicting a magnetic measurement or value for any coordinate or location in a patient reference frame.

The $PRSToPat_{i,k}$ transformation aligns (e.g., rotates) the patient reference sensor 26 at $ref_k$ to the patient reference frame 6. That is, the $PRSToPat_{i,k}$ transformation represents a patient reference sensor transformation between the patient reference sensor and the patient reference frame. In an embodiment, the patient reference sensor may define an origin of the patient reference frame. In another embodiment, the patient reference sensor may be offset from an origin of the patient reference frame. In either embodiment, the initial orientation of the patient is known. For instance, at the start of a procedure, the patient typically lies in a consistent orientation on a surgical table such that the orientation of patient reference frame is known. The patient reference frame 6 may extend from, for example, head to foot of the patient, which is initially aligned with a support/surgical table, left to right and vertically up and down. The patient reference sensor 26 has six degrees of freedom (e.g., x, y, z, roll, pitch and yaw). As the patient reference sensor is applied externally to the patient, an initial orientation 5 of the sensor 26 (e.g., roll, pitch, yaw) may not align with the patient reference frame 6. However, the orientation of the patient reference sensor as applied to a patient can be determined (e.g., using the medical positioning system) and transformed to align the patient reference sensor 26 with the patient reference frame 6 defining the $PRSToPat_{i,k}$ transformation. The relationship between the patient reference sensor 26 and the origin or any coordinate in the patient body is assumed to remain substantially constant as the patient reference sensor 26 and origin (as well as other coordinates in a patient body) both move with patient movements. Therefore, a nominal patient reference sensor to patient transformation (e.g., NomPRStoPat) is a definable and fixed transformation.

The $PatToMag_k$ transformation aligns the patient reference frame with the magnetic reference frame 4. Stated otherwise, the $PatToMag_k$ transformation defines a transformation between a magnetic reference frame having an origin associated with the magnetic field generator of the magnetic-based medical positioning system and a coordinate or location in the patient reference frame. This transformation, $PatToMag_k$, may initially be based on a fixed orientation of the magnetic field generator (e.g., magnetic reference frame 4) relative to an initial orientation of the patient reference frame, which is known. Stated otherwise, a nominal patient to magnetic transformation (e.g., NomPatToMag) is a definable and fixed transformation.

Based on these relationships and transformations, a position and orientation of any coordinate (e.g., $pat_k$) in the patient reference frame may be determined in the magnetic reference (e.g., $mag_k$) frame using the $PatToMag_k$ transformation. In this regard, the relationship $mag_k = PatToMag_k pat_k$ forms a portion of an observational model, as discussed further herein. In an embodiment, the position of the patient reference sensor may be monitored to identify displacements caused by patient movement. That is, the medical positioning system may identify displacements of the patient reference sensor and these displacements may be used to update the above-noted relationships.

In order to implement the model, it is necessary to define the transformations between the coordinate systems (e.g., patient coordinate system and magnetic coordinate system). The relationship from the patient reference sensor space to the magnetic field generator (e.g., magnetic coordinates) is at any time k:

$$PRStoMag_{i,k} = PatToMag_k PRSToPat_{i,k}$$

That is, the transformation from the patient reference sensor to the magnetic field generator is a product of the two transformations $PatToMag_k$ and $PRStoPat_{i,k}$. As the patient reference sensor is a magnetic sensor, its position in the magnetic field of reference may also be directly observed. However, due to patient motion, both $PatToMag_k$ and $PRStoPat_{i,k}$ may change or evolve over time though these two transformation should remain near their nominal relationships $NomPRSToPat_i$, which is also defined as $PRStoPAT_{i,nom}$, and NomPatToMag, which is also defined as $PatToMag_{nom}$. In an embodiment, the nominal relationships are the initial relationships (e.g. at k=0). $PatToMag_k$ and $PRStoPat_{i,k}$ are defined to track deviations from their nominal relationships and capture these changes so that the transformations between these frames of reference are updated in conjunction with patient movements (e.g., movement of the patient reference sensor).

In an embodiment, the $PRStoPat_{i,k}$ can be defined to track deviations as follows:

$$PRStoPat_{i,k} = NomPRSToPat_i PRSToNomPRS_{i,k}$$

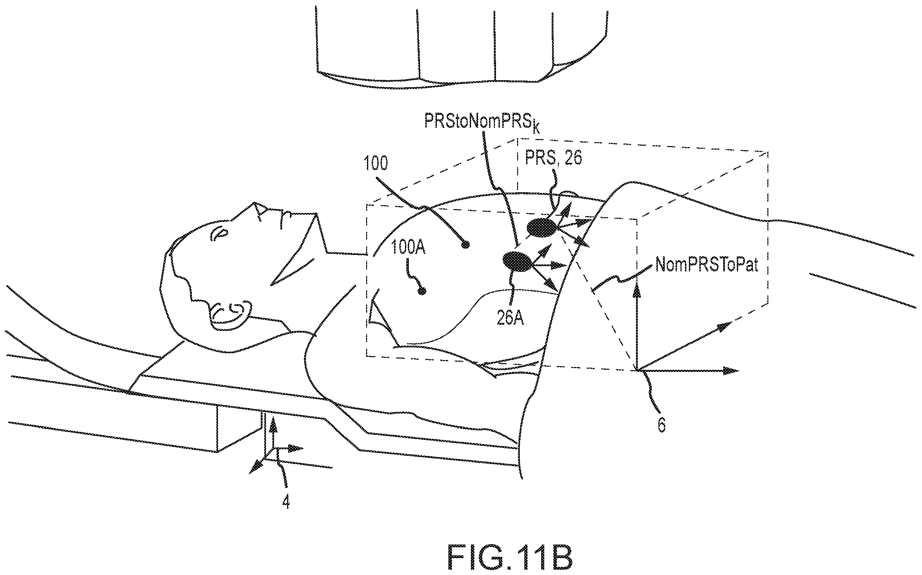

This is diagramed in FIG. 11B. As shown, the patient reference sensor 26 moves from a nominal (e.g., initial in an embodiment) a shifted position 26A as may be identified by the medical positioning system. Likewise, other coordinates (e.g., 100) move from an initial position to a shifted position 100A. In the illustrated embodiment, the fixed transformation between the nominal (e.g. initial) patient reference sensor location and orientation and the patient reference frame 6 is designated as the nominal patient reference transformation $NomPRSToPat_i$. In addition, a time varying transformation $PRSToNomPRS_{i,k}$ defines movement between the initial sensor location 26 and a subsequent/current sensor location 26A. See FIG. 11B. That is, $PRSToNomPRS_{i,k}$ is patient reference sensor displacement transformation. This displacement may be determined, at least in part, based on monitoring movement of the patient reference sensor using the magnetic field generator. In this embodiment, $PRStoPat_{i,k}$ is a product of the nominal transformation and the patient reference sensor displacement transformation.

In embodiment, $PatToMag_k$ can be defined to track deviations as follows:

$$PatToMag_k = NomPatToMag\ PatToNomPat_k$$

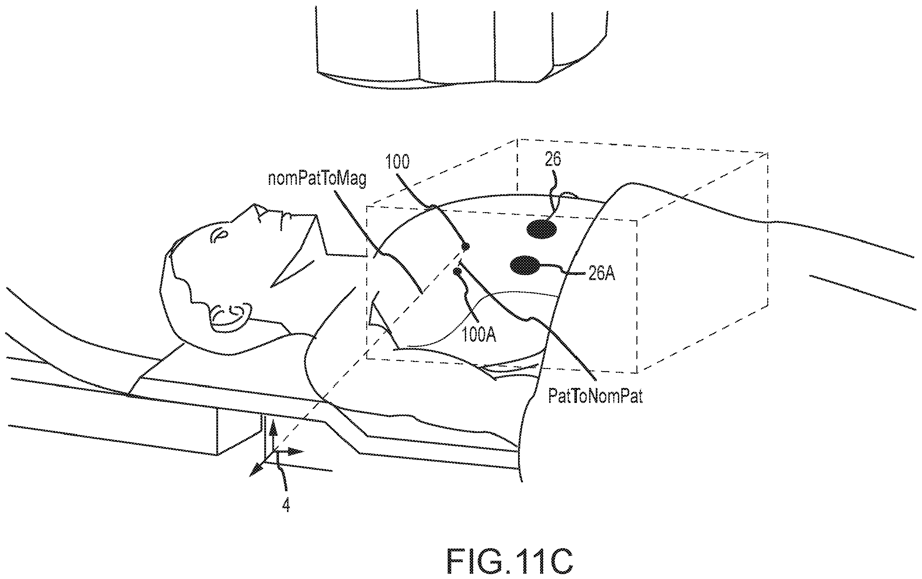

This is diagramed in FIG. 11C. As shown, the coordinate 100 moves from a nominal or initial position to a displaced position 100 in response to patient movement. In the illustrated embodiment, the transformation between the patient reference frame and the magnetic reference frame is designated as the initial or nominal magentic transformation NomPatToMag and is a fixed transformation. In addition, a time varying transformation $PatToNomPat_k$ between the initial coordinate location 100 and a subsequent/current location 100A defines the displacement of the coordinate in the patient reference frame. That is, $PatToNomPat_k$ is a coordinate displacement transformation. This displacement may be determined, at least in part, based on monitoring movement of the patient reference sensor and, hence, the patient reference frame. In this embodiment, $PatToMag_k$ is a product of the nominal transformation and the coordinate displacement transformation. In an embodiment, $PRSToNomPRS_{i,k}$ and $PatToNomPat_k$ are governed by state variables, which may be determined during a sensor fusion process. In an embodiment, these variables are determined in an estimation system, such as a recursive Bayesian estimator (e.g. an extended Kalman filter or particle filter), to fit magnetic and catheter state variables to magnetic measurements and other measurements. In summary, these transformations, $PRSToNomPRS_{i,k}$ and $PatToNomPat_k$, are not directly observable and are subject to continued changes due to, for example, patient respiration and other patient movements and are allowed to evolve over time. However, in combination with observable parameters, (e.g., $PRSToMag_{i,k}$), these transformations may be estimated.

As previously noted, a patient typically lies in a consistent orientation on a surgical table, at the start of a procedure. Therefore, a fixed orientation of the magnetic field generator (e.g., magnetic reference frame 4) relative to the patient reference frame can be assumed for an initial state. Accordingly, in an embodiment it is assumed that the nominal relationships (e.g., transformations, sensor locations etc.) are substantially equal to the initial relationships at the beginning of the procedure. In an embodiment:

$$PRSToNomPRS_{i,nom} = I \approx PRStoNomPRS_{i,0}$$

$$PatToNomPat_{nom} = I \approx PatToNomPat_0$$

That is, it is expected that the nominal configuration is near the initial configuration I which is substantially equal to the nominal configuration at time 0. Therefore, the initial patient reference sensor locations are a suitable value for $PRSToMag_{0,nom}$, as they are expected to be close to $PRSToMag_{0,0}$.

In an embodiment, the patient reference sensor may be measured (i.e., observed) in the magnetic field:

$$PRSToMag_{0,nom} = \begin{bmatrix} P_{nom} & p_{nom} \\ 0 & 1 \end{bmatrix}$$

where $P_{nom}$ is the observed orientation of the patient reference sensor at the nominal time (e.g., time 0 or a combination of several observations near time 0) and $p_{nom}$ is the position of the patient reference sensor at the nominal time. Accordingly, the $PRSToMag_{0,nom}$ transformation may be determined from direct measurements. Based on the $PRSToMag_{0,nom}$ transformation, the following transformations may be derived:

$$PatToMag_{nom} = \begin{bmatrix} A_{nom} & a_{nom} \\ 0 & 1 \end{bmatrix}$$

$$PRSToPat_{o,nom} = \begin{bmatrix} B_{nom} & b_{nom} \\ 0 & 1 \end{bmatrix}$$

where:

$A_{nom}$ is a fixed nominal rotation between the patient frame and the magnetic frame, which is assumed to be fixed;
$a_{nom}$ is a position of the patient relative to the field generator (e.g., position on a bed of the magnetic field-based positioning system), which may be estimated as set forth below;
$B_{nom}$ is the orientation of the patient reference sensor on the patient, which may be estimated as set forth below; and
$b_{nom}$ is a position of the patient reference sensor on the patient, which is known.

In an embodiment, it is desirable to set a fixed position of the patient reference sensor (e.g., 0 or origin) in the patient frame so that the patient origin is typically inside the heart. In an exemplary embodiment, for a posterior patient reference sensor, an offset between the patient reference sensor and an origin inside the heart is chosen as $b_{nom} = [0\ 175\ 0]^T$., which is a column vector as noted by the superscripted T. Other offsets are possible and typically depend on the system in which they are implemented and/or where the patient reference sensor is placed on the patient (e.g., chest, back etc.).

As the patient is laid on the table in a characteristic orientation, the orientation between the field generator, the table and, hence, the patient reference frame may be known initially. In an embodiment, a fixed nominal rotation between the patient reference frame and the magnetic reference frame may be established. For example:

$$A_{nom} = \begin{bmatrix} 1 & 0 & 0 \\ 0 & 0 & 1 \\ 0 & -1 & 0 \end{bmatrix}$$

This fixed nominal rotation typically depends on the configuration of a specific magnetic field-based positioning system. The remaining degrees of freedom are $a_{nom}$ and $B_{nom}$. Given the previous relationship:

$$\mathrm{PRStoMag}_k = \mathrm{PatToMag}_k\mathrm{PRSToPat}_k$$

then:

$$PRSToMag_{0,nom} = \begin{bmatrix} A_{nom}B_{nom} & A_{nom}B_{nom} + a_{nom} \\ 0 & 1 \end{bmatrix}$$

$$\mathrm{P}_{nom} = \mathrm{A}_{nom}\mathrm{B}_{nom}$$

$$\mathrm{p}_{nom} = \mathrm{A}_{nom}\mathrm{b}_{nom} + \mathrm{a}_{nom}$$

Therefore:

$$\mathrm{B}_{nom} = \mathrm{A}^{\mathrm{T}}_{nom}\mathrm{P}_{nom}$$

$$\mathrm{a}_{nom} = \mathrm{p}_{nom} - \mathrm{A}_{nom}\mathrm{b}_{nom}.$$

Therefore each of $A_{nom}$, $a_{nom}$, $B_{nom}$ and $b_{nom}$ may be measured and/or derived allowing the estimation of the transformations of $PatToMag_{nom}$ and $PRSToPat_{0,nom}$. Stated otherwise, enough observable parameters exist to permit estimations of the variable transformations.

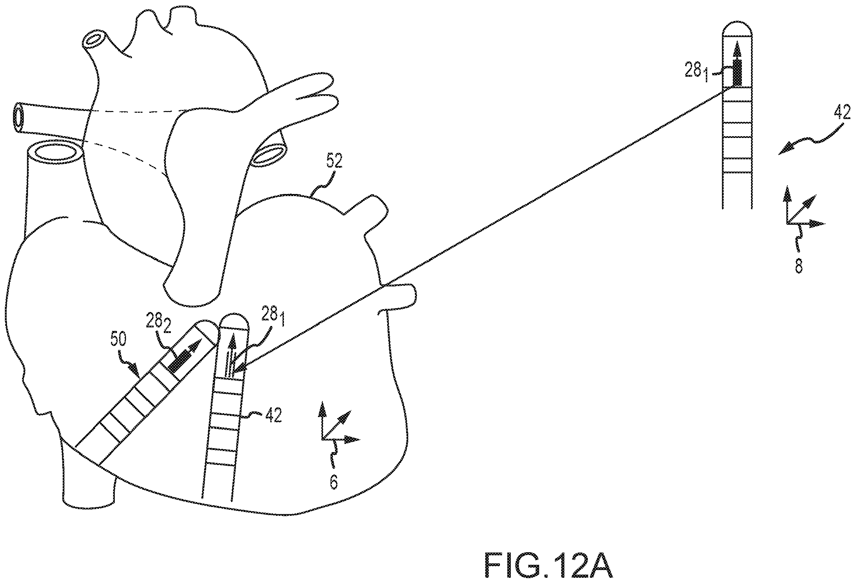

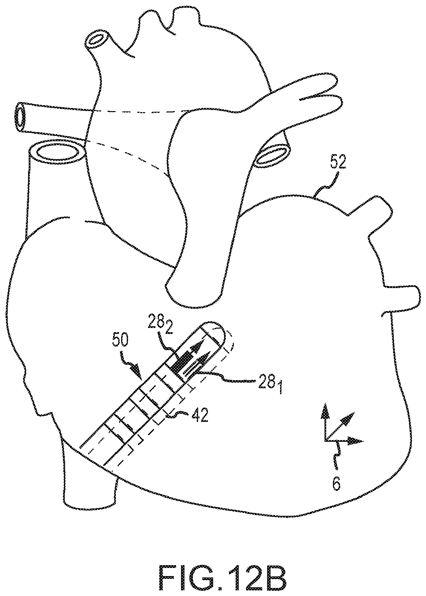

The models define a time-varying rigid transformation from the patient coordinate frame to a magnetic field generator frame to permit determining a magnetic value for a location in the patient reference frame. In an embodiment, the PRS model is used in conjunction with the catheter model and the position and orientation model. As shown in FIG. 12A, a catheter model 42 (e.g., of the catheter 24 of FIG. 6A) is initially transformed into the patient reference frame 6 using the position and orientation model 44. That is, the model 42 is used to predict locations of sensors and/or electrodes in the heart 52 of the patient as shown in phantom. FIG. 12A also shows a physical catheter 50 disposed within the heart. The catheter model 42 corresponds the physical catheter 50 disposed within the patient heart 52. Initially, a location of model magnetic sensor $28_1$ of the catheter model is predicted in the patient reference frame 6 using the position and orientation model. That is, the catheter model is transformed from the catheter reference frame to the patient reference frame to place the model magnetic sensor $28_1$ at, for example, a coordinate within the heart. This position may originally represent the coordinate $pat_k$. At this time, a magnetic measurement/value of the model magnetic sensor $28_1$ may be determined (e.g., predicted) in the magnetic reference frame utilizing the $PatToMag_k$ transformation, as set forth above. Correspondingly, a magnetic measurement/value may be measured, in the magnetic reference frame, for the corresponding physical magnetic sensor $28_2$ of the physical catheter 50. For instance, the medical positioning system may obtain an actual measurement (e.g., with some amount of system noise) for the magnetic sensor $28_2$ of the physical catheter. The predicted measurement and the actual measurement may be utilized in a stochastic process to update the various models. That is, the predicted measurement and the actual measurement (or multiple measurements if multiple sensors are present) may be utilized to adjust the position and orientation of the catheter model 42 within the patient reference frame and/or generate location of the physical magnetic sensor. In an embodiment, $pat_k$ is updated. Over a number of iterations, the position and orientation of the catheter model 42, as projected into the patient reference frame, may be updated to more closely approximate the position and orientation of the physical catheter 50 as disposed within the patient heart. See FIG. 12B. In an embodiment, the PRS model permits for near continuous updating of the location of the catheter model within the patient reference frame to account for patient movements. That is, rather than aligning the catheter model based on a static transformations, the evolution of the PRSToPat and PatToMag transformations provides continuous updating of the location of the model magnetic sensor of the catheter model, even in the presence of patient motion, such that its predicted location more closely represents the physical location of the physical magnetic sensor.

In an embodiment, an Extended Kalman filter is used to infer hidden state variables corresponding to the hidden state variables of the transformations of the models. From the hidden state variables, at any time, hidden state measurements (e.g., magnetic values in the patient reference frame) can be predicted and estimates of the state variables can be updated using an Extended Kalman filter (or other estimator) framework in a fashion that allows updates to those parts of the hidden state variables that are accessible. Thus, at any instant in time, while there may not be enough information to determine parts of state variables, by using the Extended Kalman filter framework, predictions associated with appropriate parts of the state variables associated with the transformation from an impedance based domain to a patient domain can be made.

Differences between the predictions for the appropriate parts of the state variables associated with the model and actual measurements can be made and the appropriate parts of the state variables can be updated based on the differences between the predictions and the actual measurements. As such, the state variables can be modified over a given period of time, rather than at a given instant in time. For example, the prior prediction of the appropriate parts of the state variables can be corrected based on measurements at a current time point.

As discussed further herein, the magnetic and/or PRS models may form a part of the overall or composite system model. During implementation, the models are queried to predict sensor locations in the patient reference frame. Subsequently, these predictions are utilized with sensor locations measurements to further refine the estimated locations of the sensors in the patient reference frame and update the magnetic model and/or PRS model. It will be appreciated that additional magnetic models are possible and considered within the scope of the present disclosure. In an example, a magnetic model for use in transforming between patient relative coordinates to magnetic coordinates is described in U.S. Provisional Application No. 62/756,936 titled "Patient Reference Sensor Model for Medical Device Localization based on Magnetic and Impedance Sensor Measurements", filed on November 7, 2018.


Impedance Model


Within the context of a sensor fusion process, the usefulness of impedance measurements to locate physical catheters and their electrodes in a three-dimensional space (e.g., patient reference frame) depends on having an effective model, for any catheter configuration, to predict the impedance measurements within an electrical or impedance potential field. That is, based on a predicted location (e.g., model location) of a catheter and/or catheter electrodes (e.g., model electrodes) in a patient reference frame (e.g., three-dimensional space), it is desirable to predict the impedance measurements of the modeled catheter electrodes to refine the location of the physical catheter and/or its electrodes and/or to update the impedance model. It has been further recognized that previous efforts of impedance modeling of electrodes locations has, in some instances, lacked accuracy due to the failure to account for noise.

In an embodiment, an impedance model transforms between the patient coordinate system and the impedance measurements (e.g., PatToImp). Further, the impedance model may incorporate noise and and/or distance dependent modeling errors between individual electrodes to improve the estimation of impedance measurements for determining electrode locations within a patient. In an embodiment, the impedance model is a stochastic process where a true state of the model is a hidden or latent state that is determined. The model generates estimates of electrode impedance measurements in a three-dimensional space (e.g., location-to-impedance-values). Such estimates and/or the model may be refined based on actual impedance measurements of electrodes located in the three-dimensional space. In an embodiment, such an impedance model implements various separate methodologies, which can be used in combination.

In an embodiment, a first methodology is directed to modeling the location-to-impedance-value as mapping a linear combination of harmonic basis functions, such as regular solid harmonics. However, it will be appreciated that additional harmonic basis functions are possible and considered within the scope of the present disclosure. However, it is believed that regular solid harmonic basis functions provide suitable descriptiveness with reduced degrees of freedom, simplifying the model. Further, as the electrodes are located within a common blood pool, they experience generally uniform conditions such that a Laplacian of the harmonic basis functions should be zero providing a constraint for the model. Yet further, the linear combination of harmonic basis functions may be constrained to obey Kirchhoff's voltage law. Collectively, this helps to account for spatial nonlinearity of the impedance measurements. In an embodiment, a second methodology is directed to modeling the measurement noise characteristics of the impedance system, including covariance among measurements from distinct electrodes. In an embodiment, a third methodology is directed to introducing an artificial measurement noise covariance among distinct electrodes that falls off with the distance between those electrodes. This noise term represents the amount of otherwise un-modeled error and helps to account for spatial nonlinearity of the impedance measurements and/or respiration-related artifacts.

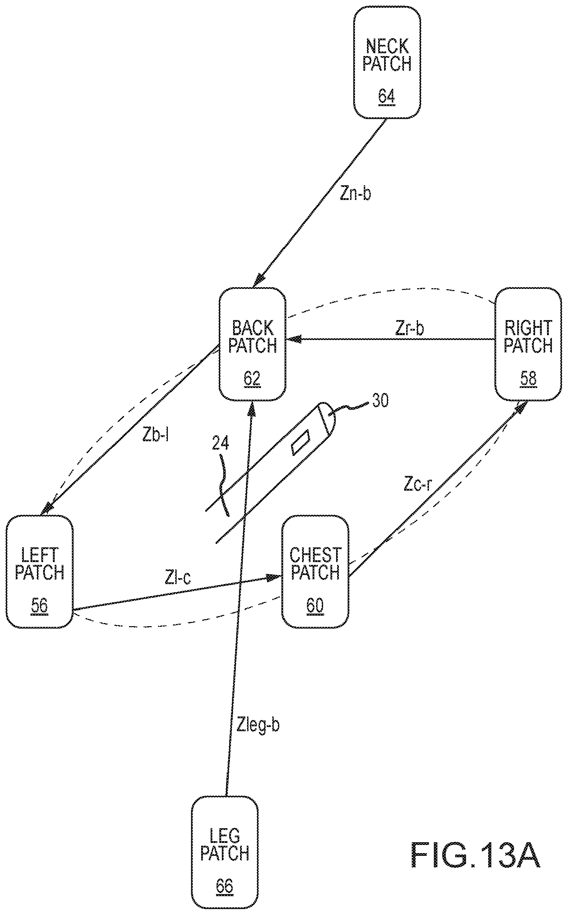

The hardware for impedance-based location measurements include of a set of electrical patches affixed to the patient/patient reference frame (e.g. 6 patches: neck, leg, chest, back, right, left). See. FIGS. 3A-3D and 13A. AC voltages are applied to sets of patch pairs (e.g. back->left, left->chest, right->back, chest->right, neck->back, leg->back) and the potentials (e.g., impedances) on each catheter electrode 30 of a catheter 24 disposed in the resulting impedance field are measured while each patch pair is driven. See FIG. 13A. The measured potentials depend on the relative impedances between the electrode(s) and each of the driven patches. That is, each driven patch pair induces a potential field across the patient refence space that the electrodes measure. Accordingly, the intent of the impedance model is to model the potential field and its measurement characteristics such that an impedance measurement may be estimated for any location within the potential field. By way of example, after establishing such and impedance model of a three-dimensional space (e.g. patient refence space), impedance values may be estimated or predicted for any location in that space at a given time.

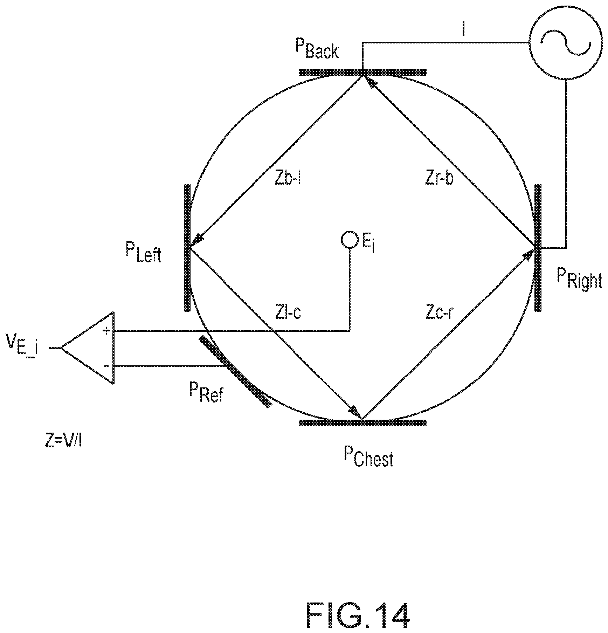

FIG. 13A illustrates a mathematical graph of the impedance patches where each patch forms a vertex of the graph and a graph edge (e.g., solid connecting line) extends between the vertexes of each driven patch pair. In a mathematical graph, a cycle is a path of edges and vertices wherein a vertex is reachable from itself. That is, the cycle forms a closed loop. In the present embodiment, the set of driven patch pairs defines a single cycle: back->left->chest->right->back. Each cycle of the graph (i.e., one in the present embodiment) that forms a closed loop or circuit (e.g., cycle) is constrained by Kirchhoff's voltage law, which implies that the potential differences around that cycle must sum to zero. That is, $Z_{B-L} + Z_{L-C} + Z_{C-R} + Z_{R-B} = 0$. See FIG. 14. Correspondingly, the sum of driven potentials on any electrode from that cycle must be zero as the potential drop around the circuit back->electrode->left->electrode->chest->electrode->right->electrode->back must also be zero.

Based on these constraints, the number of independent impedance potential fields is the number of driven patch pairs (i.e., six in the present embodiment) less the number of cycles (i.e., one in the present embodiment) in the graph. In the present embodiment, there are five independent impedance potential fields. Further, there is a linear mapping from these independent impedance potential fields to the larger set of potentials driven by the patch pairs. For the set of driven patch pairs shown in the present embodiment, the mapping is as follows:

$$\begin{bmatrix} z_{back \rightarrow left} \\ z_{left \rightarrow chest} \\ z_{right \rightarrow back} \\ z_{chest \rightarrow right} \\ z_{neck \rightarrow back} \\ z_{leg \rightarrow back} \end{bmatrix} = .5 \begin{bmatrix} -1 & 1 & 0 & -1 & 0 \\ 1 & 1 & 0 & 1 & 0 \\ -1 & -1 & 0 & 1 & 0 \\ 0 & 0 & 1 & 0 & 1 \\ 0 & 0 & -1 & 0 & 1 \end{bmatrix} \begin{bmatrix} y_{left \rightarrow right} \\ y_{back \rightarrow chest} \\ y_{neck \rightarrow leg} \\ y_{xy} \\ y_z \end{bmatrix}$$

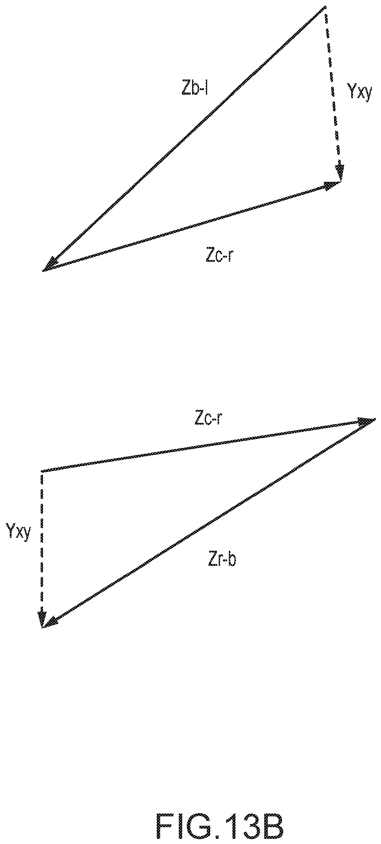

Where z is the vector of measured or estimated potentials, and y is the vector of the independent impedance potentials, which, in an embodiment, are hidden state variables of a stochastic process. The numeric matrix, denoted Mbelow, maps the five independent impedance potential fields y to the six potentials z. In an embodiment, the matrix M forms an observational model for the stochastic process. The independent impedance potential fields represent virtual potential fields that, in the presented embodiment, are never excited though exist within the system. That is, the independent impedance potential fields exist between the non-exited patch pairs. Two of these independent impedance potential fields $y_{left-right}$t and $y_{back-chest}$ are shown in the graph of FIG. 13A. By way of example, $y_{left-right} = z_{left-chest} + z_{chest-right}$ and $-y_{left-right} = z_{right-back} + z_{back-left}$. The independent impedance potential field for $y_{xy}$ is illustrated in FIG. 13B. This potential field is based on the four patches, back, left, chest right that lie in a substantially common plane XY as illustrated in FIG. 13A. A similar graph may be provided for the YZ plane of the patient to describe the remaining independent impedance potential fields. Thus, the independent impedance potential fields may be calculated or estimated as algebraic functions of the measured impedance values. In an embodiment, the independent impedance potential fields define state variables of the stochastic process and are described with harmonic basis functions as set forth below.

Each time point and for each independent driven patch pair or 'impedance mod', model impedance measurements for each independent impedance potential field i and electrode j are set forth as follows:

$$y_{ij} = \varphi_{ij} + \varepsilon_{ij}$$

and:

$$z_k = \text{Vec}(My_j) + v_k$$

Where $\varphi_{ij}$ is a potential in independent impedance potential field i for electrode j computed from a series of harmonic bases $Y_\ell$, $\varepsilon_{ij}$ is a modeling error term which covaries between a pair of electrodes as function of their distance, and $v_k$ is a measurement noise term.

For each independent driven patch pair or impedance mod i', $\varphi_{i'j}$ is a function of the electrode location $x_j$ (e.g., in the patient reference frame) and of the state variables of the impedance model (e.g., Patient to Impedance transformation). In an embodiment, the impedance transformation may be a global dynamic non-rigid transformation that maps the patient frame of reference to the impedance frame of reference. $\varepsilon_{i'}$ is the vector of all distance dependent modeling error terms for impedance mod $i'$ and is modeled as a multivariate normal random variable whose entries have a covariance dependent on the distances between pairs of electrodes. Finally, v, the vector of the electrical noise terms for all electrodes, is a multivariate normal random variable reflecting electrical noise characteristics of the measurement system. This model of impedance measurement behavior is used as part of the sensor fusion process or algorithm, such as a recursive Bayesian estimator (e.g. an extended Kalman filter or particle filter), to fit impedance and catheter state variables to impedance measurements and other measurements.

In an embodiment, $\varphi_{ij}$ is a linear combination of basis functions. Each basis function at a point in space maps to an electrical value of the modeled potential field to an electrical value (e.g, voltage, impedance, etc.). If electrodes are at locations $x_j$ then:

$$\varphi_{ij} = \sum_{\ell} b_{i,\ell} Y_\ell(x_j)$$

Where $Y_\ell$ is a scalar-valued function evaluating the $\ell$'th solid harmonic basis function for an electrode location, and $b_{i,\ell}$ is the weights on the basis functions (e.g., $\ell$'th basis function) relating the patient frame of reference to the impedance potential field. All basis functions $Y_\ell$ should be harmonic. That is, the Laplacian everywhere should be zero. In an embodiment, $Y_\ell$ can be the regular solid harmonics of up to a predetermined order. For example, $Y_\ell$ can be the regular solid harmonics of up to the fourth order. Use of harmonics up to the fourth order results in 25 basis functions per electrode. As will be appreciated, limiting the harmonic basis functions to the fourth order truncates information in higher order harmonics, which may provide additional description of the potential field. The exclusion of this information is accounted for in the modeling error term $\varepsilon$. In an embodiment, the weights $b_{i,\ell}$ may be set to predetermined or default values, which may be based on experimentally determined baselines. During operation of the impedance model, these weights $b_{i,\ell}$ are adjusted to fit impedance and catheter state variables to impedance measurements and other measurements.

Distribution of the modeling error term $\varepsilon$:

The intent of the modeling error term is to represent sources of unmodeled signals in the impedance measurements, such as unmodeled high-order terms of the harmonic basis or perturbations caused by patient respiration. Explicitly incorporating the expected magnitude of unmodeled phenomena or error in the impedance basis model makes the system robust to such discrepancies.

For each independent impedance mod $i'$,, let $\varepsilon_j = n_j + r_j$ where $n_j$ is a multivariate normal random variable representing error due to nonlinearity of the impedance not modeled by the $g_k$ and $r_j$ is a multivariate normal random variable representing error due to, for example, unmodeled respiratory artifacts.

In an embodiment $n_j$:

$$n_j = N\left(0, \begin{bmatrix} h(|x_l - x_l|) & ... & h(|x_l - x_m|) \\ ... & ... & ... \\ h(|x_m - x_l|) & ... & h(|x_m - x_m|) \end{bmatrix}\right)$$

*where $h(d) = ae^{-bd2}$* Where $N$ is a normal random distribution, 0 represents the mean of the distribution and the matrix $h$ represents the covariance for each electrode based on the absolute distance $d$ (e.g., vector) between each electrode (i.e., $|x\#-x\#|$). See. e.g., FIG. 6A. This choice of covariance results in error expected to vary smoothly over space. The parameter $a$ represents a magnitude of the modelling error. The larger the parameter $a$, the greater the expected magnitude of nonlinearity-derived error. The parameter $b$ represents a width or radius over which the modelling error decays. The larger the parameter $b$, the smaller its expected distance scale.

In an embodiment, $r_j$:

$$r_j = N\left(0, \begin{bmatrix} c & ... & c \\ ... & ... & ... \\ c & ... & c \end{bmatrix}\right)$$

At each time i, respiration applies a shared translation to all points of each independent impedance potential field. The parameter c controls the magnitude of respiratory error. In an example, a system and method for modeling a respiratory error or artifact is described in U.S. Provisional Application No. 62/756,926 titled "Respiration Model for Device Localization Based on Impedance Sensor Measurements", filed on November 7, 2018.

Distribution of the electrical noise term $v_k$:

For each driven patch pair j:

$$v_k = \ \sim N\left(0, \begin{bmatrix} d+e & ... & d \\ ... & ... & ... \\ d & ... & d+e \end{bmatrix}\right)$$

In this embodiment, each electrode has a noise component of variance e that is independent of other electrodes and a noise component of variance d that is shared with other electrodes.

In use, the impedance model mathematically defines the impedance field for a patient reference frame. In an embodiment, the impedance field is defined such that impedance potentials for a set of driven patch electrodes, at any location in the impedance field, are a function of a set independent impedance potential fields, which are mapped to the impedance potentials. Various method may be implanted to generate the impedance model. In an embodiment, the impedance model is generated on the fly during a procedure. That is, a catheter 24 may be disposed within an impedance field at the beginning of a medical procedure. See FIG. 13A. During the procedure, electrode impedances measurements are acquired as the catheter 24 moves through the impedance field and the independent impedance potential fields are mapped to the impedance measurements. In another embodiment, an initial impedance model may be generated using a mapping catheter having a high number of electrodes. In such an embodiment, the mapping catheter may be moved (e.g., swept) around an internal patient cavity (e.g., heart chamber) to acquire impedance measurements for a large number of locations. This may result in an impedance model having a large number of locations and corresponding impedance measurements and mapped independent impedance potential fields. In such an embodiment, the mapping catheter may be removed after generating an initial impedance model and replaced with a catheter used to perform a medical procedure (e.g., an ablation catheter). On exemplary mapping catheter is described in U.S. Patent No. 8,744,599, entitled "High Density Mapping Catheter". In a further embodiment, a default impedance map may be provided that assumes an initial state of the impedance model. In this embodiment, subsequent impedance measurements are used to more rapidly update the model for a current impedance field or patient reference space. In any embodiment, locations, impedances and independent impedance potential fields may be recorded. Subsequently, the impedance model may utilize this information to, for example, interpolate impedance measurements for locations between known locations in the modeled impedance field.

In an embodiment, the independent impedance potential fields and their parameters are state variables of a stochastic process such that they may evolve over time. Initially the impedance model estimates a set of impedance measurements $z_i$,est (e.g. back->left, left->chest, right->back, chest->right, neck->back, leg->back) for electrodes $j$ at a locations $x_j$. See. e.g., predicted electrode locations $30_1$-$30_4$ in the heart 52 in FIG. 5B and predicted measurements $31_1$-$31_4$ in FIG. 5C. In an embodiment, the predicted locations for these electrodes may be estimated in the patient reference frame using a catheter model. The predicted set of impedance measurements are generated, in an embodiment, based at least in part on the weights $b_{i,\ell}$ applied to the harmonic basis functions. When implementing the impedance model, a set of impedance measurements (e.g., actual measurements) $Z_{i,act}$ may be obtained for a corresponding physical electrode in the patient reference frame. See. e.g., measurements $35_1$-$35_4$ in FIG. 5C. The predicted measurements $z_{i,est}$ may be utilized with the actual measurements $Z_{i,act}$ (e.g., observable parameters/electrode impedance measurements, calculated independent impedance fields) to determine, for example, a correction or gain in a stochastic process. This correction/gain may then be utilized to adjust hidden state variable of the impedance model. For example, the weights $b_{i,\ell}$ as well as other hidden state variables (e.g., basis functions of the independent impedance potential fields) of the impedance model may be adjusted to better fit or match the actual impedance measurements. In application, the impedance model adjusts (e.g., recursively) to more closely approximate the actual impedance measurements. Accordingly, the updated impedance model may subsequently be used to predict an impedance measurement for locations (e.g., as predicted by a catheter model) in the modeled potential field. Further, the correction/gain may be utilized to estimate an updated electrode location in the patient reference frame. See, e.g., true locations $37_1$-$37_4$ in FIG. 5C.

In an embodiment, an Extended Kalman filter is used to infer hidden state variables corresponding to the hidden state variables of the model. From the hidden state variables, at any time, hidden state measurements (e.g., impedance values at locations in space) can be predicted and estimates of the state variables can be updated using an Extended Kalman filter framework in a fashion that allows updates to those parts of the hidden state variables that are accessible. Thus, at any instant in time, while there may not be enough information to determine parts of state variables, by using the Extended Kalman filter framework, predictions associated with appropriate parts of the state variables associated with the transformation from the location in the patient reference frame to impedance measurement can be made.

Differences between the predictions for the appropriate parts of the state variables associated with the model and actual measurements can be made and the appropriate parts of the state variables can be updated based on the differences between the predictions and the actual measurements. As such, the state variables can be modified over a given period of time, rather than at a given instant in time. For example, the prior prediction of the appropriate parts of the state variables can be corrected based on measurements at a current time point.

As discussed further herein, the impedance model forms a part of the overall or composite system model. During implementation, the impedance model is queried for use with predicted electrode locations in the patient reference frame as estimated by the catheter model. More specifically, the impedance model is used to predict measurements for each electrode location in the patient reference frame. Subsequently, these predictions are utilized with actual electrode measurements to further refine the estimated locations of the electrodes in the patient reference frame as well as update the impedance model. In an example, impedance models for use in determining impedance measurement or values for a location in a patient reference frame are described in U.S. Provisional Application No. 62/756,931 titled "Impedance Transformation Model for Estimating Catheter Locations", filed on November 7, 2018.

Respiration Model

Respiration artifacts can contribute significantly to impedance measurement errors thereby increasing an error in location determinations for medical devices. In an embodiment, the present disclosure describes a respiration model accounting for impedance changes due to respiration/breathing. In an embodiment the respiration model describes catheter electrode impedance artifacts allowing for increasing the accuracy of impedance measurements and/or predictions thereby providing improved accuracy when determining a location of a medical device and/or its electrodes.

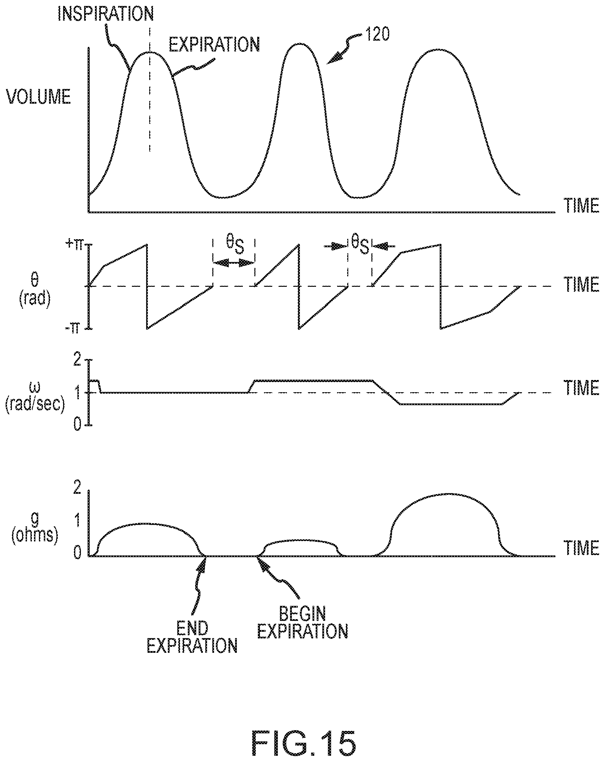

During a cardiac medical procedure, in vivo impedance measurement errors co-vary significantly due to respiration. That is, respiration induces a time-varying artifact relative to spatially-varying impedance measurements within a patient reference frame (e.g., on or within a patient chest). The time-varying artifact occurs during each respiration cycle due to changes in a volume of the chest of a patient increasing and decreasing. More specifically, the change in volume alters the physiological state of the patient and thereby alters impedance measurements of an impedance potential field within in the patient reference frame. Accordingly, it is desirable to monitor and account for such artifacts. Monitoring respiration induced artifacts is complicated by the fact that respiration varies between cycles. While respiration does move through a periodic phase-space, from the end of expiration through inspiration and back to expiration, respiration is not regularly periodic. Each breath is unique in amplitude and in duration with the time between breaths also varying. This is illustrated in FIG. 15, which shows a waveform 120 of series of respirations. The waveform 120 increases during inspiration and decreases during expiration of a patient. This waveform is not directly monitored in the presented process and is illustrated by way of example. The phase angle $\Theta$ of the respiration waveform has a periodic domain varying from $\pi$ to $-\pi$. More specifically, the phase angle $\Theta$ increases from zero to $\pi$ during inspiration and decreases from $-\pi$ to zero during expiration. As shown, need not be constant during either inspiration or expiration. For instance, the rate of inspiration or expiration may change during a single inspiration or expiration cycle as shown by the non-liner slope of the various phase angle waveforms. The rate of change of the of the respiration waveform, denoted $\underline{\omega}$, is a derivative of the phase angle $\Theta$. As shown, the amplitudes and durations of adjacent respirations vary. That is, each respiration is a quasiperiodic process rather than a regularly periodic process. As respiration is quasiperiodic, the aim of the present disclosure is to model artifact induced via respiration as a quasiperiodic function. In an embodiment, the artifact is modeled as a function of the current location and position in a given respiratory cycle.

While each respiration/breath is unique, there are some relatively stable or constant respiration parameters, which may be used in modeling respiration as a quasiperiodic function. For instance, the Functional Residual Capacity (FRC), which is the volume of air present in the lungs at the end of passive expiration, is relatively constant for a given individual. In contrast, Tidal Volume (TV) is the lung volume representing the normal volume of air displaced between normal inhalation and exhalation. While FRC is relatively constant, TV (e.g., amplitude) varies more from breath to breath. The result is that the phase between end of expiration and the start of inhalation represents a relatively stable phase $\Theta_S$ with each breath representing a perturbation from the stable phase.

To model the respiration-related artifact in impedance measurements, each electrode impedance measurement, $Z_i$, is modeled as the composition of a quasiperiodic function, $g_j(\Theta)$, which is a temporally dependent function in an embodiment, and an aperiodic function, $f_i$, which is a spatially dependent function in an embodiment. That is, $f_i$, may represent an impedance measurement for a location within a patient reference frame and/or within an impedance field. The quasiperiodic function is dependent on the phase angle $\Theta$ of the respiration cycle. That is, the quasiperiodic function is dependent on the location in the respiration cycle between the beginning of inhalation and the end of exhalation, which is denoted as the phase angle $\Theta$ in present analysis. As there is a common respiratory process affecting all impedance measurements within the patient reference frame, it is assumed that a single phase angle, $\Theta$ and amplitude, $\gamma$, governs all quasiperiodic functions at a time sample, $k$.

FIG. 13A illustrates a medical device or catheter 24 disposed within an impedance field defined by six external or surface patch electrodes. In the illustrated non-limiting embodiment, the patch electrodes include a left patch electrode 56, a right patch electrode 58, a chest patch electrode 60, a back patch electrode 62, a neck patch electrode 64 and a leg patch electrode 66. For any electrode impedance measurement i of an electrode 30 on the catheter 24 within the impedance field for a driven patch pair, one quasiperiodic function, $g_j$ applies. In an embodiment, it is assumed that the same quasiperiodic function applies to all electrode impedance measurements (e.g., impedance measurements of different catheter electrodes if multiple catheter electrodes are present) measured in response to driving a single pair of the of the surface patch electrodes (e.g., impedance mod). By way of example, in the embodiment of FIG. 13A where there is a single catheter electrode 30 (e.g,. electrode *i*) in the impedance field and six surface patch electrodes defining the impedance field, driving the chest to right patch pair (e.g., impedance mod) would result in a single quasiperiodic functions $g_j$. Where *j* represents the quasiperiodic function. In this embodiment, this results in one quasiperiodic functions (e.g., $g_j$). That is, if current were driven between the chest and right patch electrodes, a first quasiperiodic function would govern the electrode 30. In an embodiment, each electrode impedance is a combined measurement of six driven patch pairs (e.g. back->left, left->chest, right->back, chest->right, neck->back, leg->back). Accordingly, with one quasiperiodic functions for each driven patch pair this results in six quasiperiodic functions. Adding a second electrode does not result in new quasiperiodic functions.

In an embodiment, the relationship between the impedance measurement and the quasiperiodic functions (e.g., measurement vector) may be written as:

$$Z_{i+(j-1)*numElec}(x_k, \Theta_k, \gamma_k) = f_{ij}(x_k) + \gamma_k\, g_j\,(\Theta_k)$$

where the electrode impedance measurement $Z_{i+j*numElec}$ for the electrode at time *k* is a function of the an impedance for a location of the electrode given by the aperiodic function *f(x)*, which may be provided by the impedance model discussed above, and the an artifact for that location determined by the quasiperiodic functions $g_j$ modified by the amplitude $\gamma$ of the respiration cycle at time *k* and the location $\Theta$ in the respiration cycle at time *k*. In an embodiment, the aperiodic function *f(x)* is a constant. While each quasiperiodic function is governed by the same phase angle, each function may lead or lag relative to the others. Each quasiperiodic function is similarly expected to have either a larger or smaller, positive or negative amplitude relative to the others.

For each driven patch pair, four patches are not driven and four additional quasiperiodic functions govern the non-driven patch pairs. By way of example, in the embodiment of FIG. 13A, driving the chest to right patch pair (e.g., impedance mod) would result in four additional quasiperiodic functions (e.g., Neck->Ref patch; Leg->Ref patch; Back->Ref patch; and Left->Ref patch described by $g_7$, $g_8$, $g_9$ and $g_{10}$). In an embodiment, each electrode impedance is a combined measurement of six driven patch pairs (e.g. back->left, left->chest, right->back, chest->right, neck->back, leg->back). Accordingly, with four quasiperiodic functions for each driven patch pair this results in twenty-four additional quasiperiodic functions. In an embodiment, the non-driven patch measurements may be added to the end of the measurement vector, with the relationship between the additional non-driven patch impedance measurements and the quasiperiodic functions may be written as:

$$Z_{6*numElec+j'}(x_k, \Theta_k, \gamma_k) = f'_{j'}(x_k) + \gamma_k\, g_{6+j'}(\Theta_k)$$

As previously noted, respiration may be considered to have a relatively stable period $\Theta_s$ when the phase angle is zero (e.g., between respirations). During this stable period, the quasiperiodic functions should have no influence on the measurement. Consequently, it is desired that the all quasiperiodic functions equal zero when the phase angle is zero. Accordingly, in an embodiment, each quasiperiodic function may be formulated as:

$$g_j(\Theta_k) = \alpha_j(1-\cos(\Theta_k)) + \beta_j\sin(\Theta_k)$$

where $\alpha$ and $\beta$ represent weights to adjust the phase of each quasiperiodic function. In an embodiment, $\alpha$ and $\beta$ are constants. In an embodiment, each quasiperiodic function and the governing phase angles and amplitudes vary according to a stochastic process, with some normally-distributed error from sample to sample. That is, phase and amplitude are hidden state variables that may be predicted from previous values and observable parameters (e.g., impedance measurements).

In an embodiment, it is presumed that once a respiratory cycle begins, it will advance at a relatively steady rate, $\underline{\omega}$, except when the phase is close to zero, defined by a stable phase angle, $\Theta_s$. When sufficiently close to zero, the phase angle is predicted to be stable, trending toward zero. The phase angle has a periodic domain, from $\pi$ to $-\pi$. In an embodiment, the evolution of $\Theta$ from a previous time step (e.g., k-1) to a current time step (e.g,. k) is advances at the steady rate, $\underline{\omega}$. That is, the model assumes a predictable advancement of $\Theta$ between time steps. Though $\underline{\omega}$ and each

$\alpha_j$ and $\beta_j$ are presented as constants, these parameters may also vary. That is, these parameters may form hidden state variables of a stochastic processes, albeit ones which change on a slower time scale.

As noted, phase angle and amplitude are hidden state variables that may be predicted from previous values and current measurements. In this regard, $\Theta_{k-1}$ and $\gamma_{k-1}$ may be known and utilized to predict current phases $\Theta_k$ and amplitudes $\gamma_k$, for use in predicting electrode impedance measurements $Z_i(X_k, \Theta_k, \gamma_k)$ for location(s) (x) at time k. For example, in conjunction with a catheter model that predicts the location of an electrode(s) in the impedance field (e.g., patient reference frame) at location (x), an impedance model may predict an impedance value for the location and the respiration model may predict an artifact for that electrode. As a result, impedance measurements including respiratory artifact may be predicted for a model electrode location in the patent reference frame. Correspondingly, actual impedance measurements of a physical electrode (e.g., of a physical catheter) corresponding to the model electrode may be obtained/measured. For instance, the medical positioning system may obtain actual impedance measurements (e.g., with some amount of system noise) for a corresponding electrode of a physical catheter disposed within the patient reference frame. The predicted measurements and the actual measurements may be utilized to update the various models. That is, the predicted measurements and the actual measurements may be utilized to determine the true location of the physical electrode within the patient reference frame. In addition, the phases $\Theta$ and amplitudes $\gamma$ may be updated based on the predicted measurements and the actual measurements. As will be appreciated, the model permits estimating the phase and amplitude though these parameters are never directly observed. In this regard, the stochastic process infers the phase and amplitude and, through an iterative process, adjusts the respiration model to substantially align with the actual phase and amplitude of the respiration cycle.

In an embodiment, an Extended Kalman filter is used to infer the hidden state variables of the quasiperiodic functions. From the hidden state variables, at any time, hidden state measurements can be predicted and estimates of the state variables can be updated using an Extended Kalman filter framework in a fashion that allows updates to those parts of the hidden state variables that are accessible. Thus, at any instant in time, while there may not be enough information to determine parts of state variables, by using the Extended Kalman filter framework, predictions associated with appropriate parts of the state variables associated with the quasiperiodic functions can be made.

Differences between the predictions for the appropriate parts of the state variables associated with the quasiperiodic functions and actual measurements can be made and the appropriate parts of the state variables can be updated based on the differences between the predictions and the actual measurements. As such, the state variables can be modified over a given period of time, rather than at a given instant in time. For example, the prior prediction of the appropriate parts of the state variables can be corrected based on measurements at a current time point.

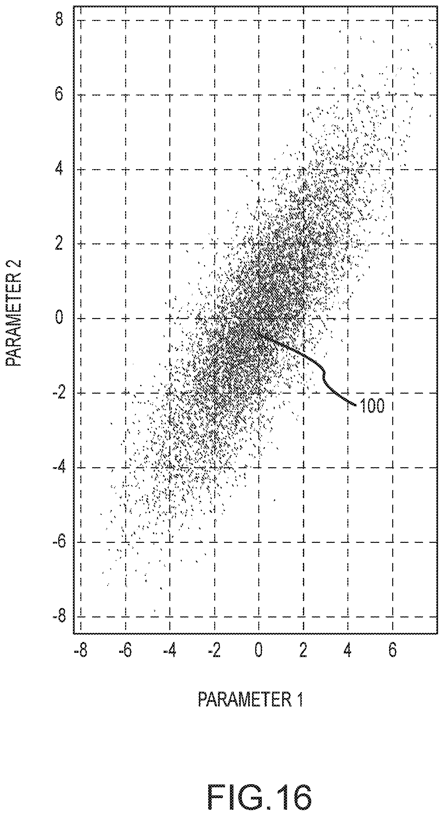

Collectively, the models fully describe the movement of the medical device in the absence of noise. Stated otherwise, the models describe possible states of the system and represent the individual state variables of the system. Generally, knowledge of the state variables at an initial time with at least partial knowledge of system inputs and/or outputs permits estimating current states and/or subsequent states of the system as points or a distribution in a state space (e.g., geometrical manifold). In such an arrangement, the state variables are disposed on the coordinate axes of the state space (e.g., and N-dimensional space). To abstract from the number of inputs, outputs and states, the state variables (e.g., models) are expressed as vectors, which are combined to form the state vector of the system. The state of the system can be represented as a distribution 100 of possible states within the state space (i.e., represented as points in the state space). See FIG. 16. Each point in the state distribution includes information for all models (e.g., electrode locations, sensor locations, model variables, etc.). Typically, the mean of the distribution is considered to represent the most likely or true state of the system. Accordingly, the mean may be utilized as a best estimate of all state variables. Generally, it is desirable to reduce the number of state variables (e.g., state vector components) to reduce the computational complexity of the system. However, it will be appreciated that additional variables (e.g., models) may be incorporated into the system model in addition to the models discussed above.

The state vector describes the movement of the medical device in the absence of noise. However, actual measurements of the electrodes and magnetic sensors are noisy. That is, measurements of these parameters each include errors or noise of an unknown magnitude. The actual system is a stochastic process as are a number of the system components (e.g., individual models). To provide improved modeling and estimation of the system, noise should be included within the system model. Accordingly, the present disclosure provides an observational model defining the relationship between the state vector, noisy measurements and control vector (e.g., known inputs). This observational model utilizes new measurements (e.g., with some amount of noise) with the previous state of the system to estimate a new state(s) of the system.

One benefit of the disclosed system is that is provides for continuous updates or estimate of the system state at each time step. That is, the observational model provides continuous correction as opposed to a static correction factor. For instance, the disclosed system may predict and update states of the system approximately 100 times per second. Of further benefit, the observation model only requires information about the previous state of the system (e.g., at time $k$-1) and the current system measurements (e.g., at time $k$) to generate updates/estimates of the system state where the state estimates are provided by an estimator.

The overall stochastic process estimates new locations of the medical device and/or new locations of the electrodes of the medical device as well as estimates for various state variables of each of the models (e.g., impedance model, magnetic model, catheter model etc.). In an embodiment, the process assumes that the state of a system at time *k* evolved from a prior state at *k*-1 according to the equation:

$$x_k = F_k(x_{k-1}) + B_k(u_k) + w_k$$

where:

$x_k$ is the state vector containing parameters of interest for the system (e.g., parameters of the models). This equation is used to predict subsequent states with error.

$F_k$ is the state transition matrix which applies the effect of each system state parameter at time *k*-1 to the system state at time *k*. Stated otherwise, the transition matrix defines the relationship between a previous state vector and a current state vector.

$u_k$ is the vector containing any control inputs (e.g., robotic controls to the medical device).

$B_k$ is the control input matrix which applies the effect of each control input parameter in the vector $u_k$ on the state vector. Of note, the present embodiment does not utilize any control inputs and input matrixes and $B_k$ and $u_k$ are empty. However, it will be appreciated that if control inputs are incorporated into the system, a control vector and control input matrix may be included.

$w_k$ is the vector containing process noise terms for each parameter (e.g., model) in the state vector. In an embodiment, the process noise is assumed to be drawn from a multivariate distribution with covariance defined by a covariance matrix $Q_k$.

Measurements of the system, with error, are also performed at each time step according to the model:

$$z_k = h_k(x_k) + v_k$$

where:

$z_k$ is the measurement vector; the set of variables measured by the sensors (e.g., impedance measurements, magnetic sensor measurements, etc.).

$h_k$ is the observational model (i.e., transformation matrix) that maps the state vector parameters into the measurement domain. Stated otherwise, the observational model defines the relationship between the state vector and noisy measurements; and

$v_k$ is the vector containing the measurement noise terms for each measured variable in the measurement vector. In an embodiment, the process noise is assumed to be drawn from a multivariate distribution with covariance defined by a covariance matrix $R_k$.

The stochastic process is utilized to determine a true state of the system, which is a hidden or latent state. The purpose of the process is to generate estimates of the system state (e.g., electrode locations, hidden variables of the models, etc.) and determine the true state (e.g., more accurate state) from these estimates. In an embodiment, an estimator is implemented in an extended Kalman filter adapted for use with non-linear system models or linearized system models and/or with models having non-Gaussian noise distributions. However, it will be appreciated that variations may be implanted using other estimators such as the unscented Kalman filter, Markov Models and/or particle filters, which each may be applied to nonlinear systems and/or systems with non-Gaussian noise distributions.
Each estimate of the estimator is a mean (i.e., center of a distribution of state estimates) and covariance describing a probability about the mean. In application, the estimates include an *a priori* estimate (predict) prior to incorporating the measurements and an *a posteriori* estimate (update) after incorporating the measurements. The *a priori* estimate uses the state estimate from the previous time step to produce an estimate (e.g., prediction) of the latent state (mean $x_{k|k-1}$ and covariance $P_{k|k-1}$) at the current time step:

$$\underline{x}_{k|k-1} = F_k \underline{x}_{k|k-1} + B_k u_k$$

$$\underline{P}_{k|k-1} = F_k \underline{P}_{k|k-1} + F_k^T + Q_k$$

That is, the *a priori* estimate is an estimate from the transformation matrix that produces an estimated distribution and covariance from the prior state (i.e., k-1). The transformation matrix takes every point in the original distribution and moves it to a new predicted distribution, which may have an expanded covariance (e.g., the addition of $Q_k$ to the covariance matric P) to account for unknown system noise. In the *a posteriori* estimate, the current *a priori* prediction is combined with the observation model to refine the state estimate. More specifically, the observational model maps the estimation (e.g. mean $\underline{x}_{k|k-1}$ and covariance $\underline{P}_{k|k-1}$) to the measurement domain to predict measurements:

$$\underline{z}_k = h_k \underline{x}_{k|k-1}$$

The predicted measurements $\underline{z}_k$ may be compared with the actual measurements $z_k$ of observable parameters (e.g., electrode measurements and sensors measurements of the system):

$$y_k = z_k - \underline{z}_k$$

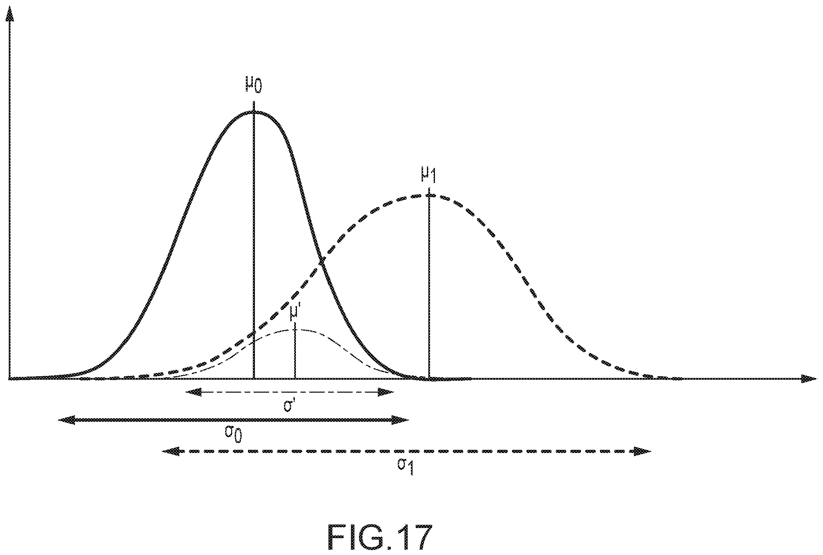

This allows for determining the gain K of the system, where K minimizes the expected sum squared error between $\underline{x}_{k|k}$ - $x_k$. This is graphically illustrated in FIG. 17 which is a 1-D representation of the state distribution combined with the observational model that produces the predicted measurements with a first predicted mean $\mu_o$ and a first predicted covariance $\sigma_o$. The actual observation measurement is represented by a second distribution with a second mean $\mu_1$ and a second covariance $\sigma_1$. The overlap of these distribution defines the system gain (e.g., Kalman gain), which is used to correct the estimated state and estimated covariance. Stated otherwise, the two distributions are fused to generate an updated distribution with a fused mean $\mu'$ and a fused covariance $\sigma'$ (e.g., two Gaussian distributions multiple together generate an Gaussian distribution of the overlapping portion of these two distributions). The gain K may be combined with the estimated state distribution and estimated covariance to generate an updated state distribution (e.g., updated state mean and updated covariance):

$$\underline{x}_{k|k} = \underline{x}_{k|k-1} + K_k y_k$$

$$\underline{P}_{k|k} = (I - K_k H_k) \underline{P}_{k|k-1} (I - K_k H_k)^T + K_k R_k K^T.$$

The updated mean state may be utilized to determine updated or true locations (e.g., calculated locations) of the electrodes and/or magnetic sensors. Further, this state may be utilized to update the various state variables of the various models.

Constraints

While the above noted process allows for predicting a current state of the system, it is further realized that the state vector and corresponding estimates of the state may be subject to various constraints. Such constraints may be utilized to limit or otherwise refine the state distributions and thereby improve the overall accuracy of the system. Given a state vector, x, a model constraint can be expressed in a functional form as g(x) = 0. In this form, any true state must satisfy this equation. By way of example, impedance measurements are made by driving current across surface patch electrodes to excite an electrode. As previously noted, the electrode excitation process occurs rapidly and sequentially as different sets of patch electrodes are selected and one or more of the unexcited (in an embodiment) surface electrodes are used to measure voltages. During the delivery of the excitation signal (e.g., current pulse), the remaining (unexcited) patch electrodes may be referenced to the reference or belly patch while the voltages impressed on these remaining electrodes are measured. Potentials across each of the surface patch electrodes may be acquired for all samples except when a particular surface electrode patch pair is driven. In the two-dimensional representation shown in FIG. 14, the back, left, chest and right surface patch electrodes define a current loop within the patient body. Kirchhoff's Voltage law dictates a linear constraint on this voltage loop. Specifically, the sum of the driven potentials (i.e., impedances) from that cycle across all of the pairs of patch electrodes must be zero. That is:

$$Z_{B\text{-}L} + Z_{L\text{-}C} + Z_{C\text{-}R} + Z_{R\text{-}B} = 0$$

Correspondingly, the sum of driven potentials on any electrode from that cycle must be zero. Accordingly, this constraint may be applied to the portion(s) of the state vector that relates to impedance measurements (e.g., impedance model). Another constraint may be that the magnetic model may be constrained to changes that correspond to a rigid-body transformation without scaling. That is, all identified objects before and after transformation must have the same relative orientations. Other constraints may be applied to the composite model or the independent models. In application one or more such constraints may be applied to limit or otherwise refine the state distributions.

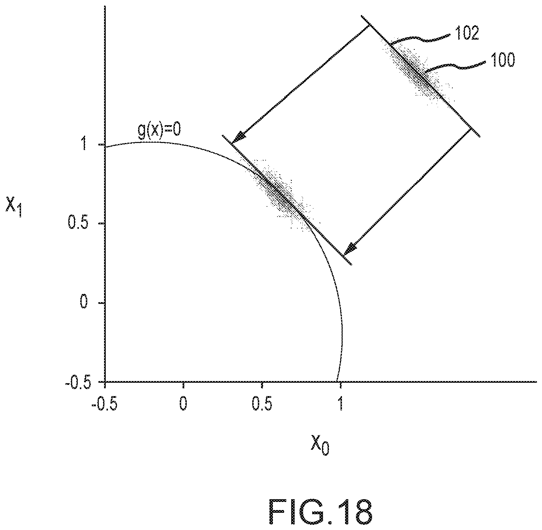

FIG. 18 illustrates a constraint g(x) = 0 in relation to a state distribution estimate. As shown, the constraint forms a feasibility manifold or constraint manifold in the state space where the constraint is satisfied. That is, the states where g(x) = 0. As shown in FIG. 11 the initial state distribution estimate 100 does not lie on the constraint manifold. Accordingly, to enforce the constraint for the state distribution estimation, the state distribution estimation must be moved to the constraint manifold. This constraint application is performed by generating a delta function that satisfies the constraint and multiplying it by the state distribution estimate to produce a constrained state distribution estimate.

The constraint application may be approximated using a first-order Taylor series expansion which generates a linear representation or tangent line 102 about the mean of the unconstrained state distribution estimate 100. This produces a first-order approximation about the unconstrained mean of the state distribution estimate. This tangent line may be projected to the surface of the constraint. More specifically, this first-order approximation may be projected orthogonally to the null-space of the Jacobian of the constraint:

$$G = \left.\frac{\partial g}{\partial x}\right|_{x'}$$

with the distribution projected into the null space of G. With successive projections through G, the estimated state distribution will track the constraint manifold even if the constraint is not exactly linear. The result is that the state distribution estimate is constrained to the constraint.

Unlikely States

The parametrization of the system (e.g., within the system models) may describe possible system states that are not realizable nor well determined by the measurements of the system. Accordingly, the present disclosure may further include penalizing such unlikely states. More specifically, the present disclosure provides a means to regularize estimations such that more likely estimates/states are produced for the system. To regularize an estimated state distribution, a regularizing function may be defined which expresses a quantity proportional to the likelihood of a state. Generally, a likelihood function describes the plausibility of a state given an observation and is the product of a probability distribution function and a state distribution. In an embodiment, a negative log likelihood is utilized. In such an embodiment, impossible states have a negative log likelihood of infinity and the most likely state has the minimum negative log likelihood. To apply this regularization, in an embodiment, a probability density function (regularizing PDF) is computed by negating, exponentiating and normalizing the negative log function. The estimated state distribution is then multiplied by the regularizing PDF and renormalized to create a regularized state distribution that omits unlikely states (e.g., states outside the combination of the state distribution and the regularizing PDF).

In an embodiment, a general negative log likelihood function may be approximately applied through a second-order Taylor series expansion of the negative log likelihood function at the mean of the estimated state distribution to create a probability density function. In an embodiment, the approximation of the negative log likelihood function may be made via the following equation:

$$-\ln r(x) \cong -\ln r(x') - \mathcal{L}(x - x') - \frac{1}{2}(x - x')^T \mathcal{H}(x - x')$$

Where the Hessian $\mathcal{H}$ of the second order expansion is treated as the inverse of the covariance, with the Gaussian mean given by the multiplication of the Jacobian of the second-order expansion by the inverse of the Hessian. This approximation is equivalent to a Gaussian PDF, which can be multiplied with the state distribution by well understood means.

The regularization of a state distribution estimate is graphically illustrated in FIGS. 7A-7C. Specifically, FIG.

7A show a state distribution 100. FIG 7B shows the regularization PDF 104 applied to the state distribution. FIG. 7C illustrates the regularized state distribution 106, which is generally enclosed by a dashed circle for purposes of illustration. As will be appreciated, the regularized state distribution excludes unlikely states from the initial state distribution estimate. This results in a new state distribution (e.g., regularized state distribution) having a different mean and a smaller covariance. That is, the regularization process results in a tighter state distribution that more accurately predicts the true state of the system.

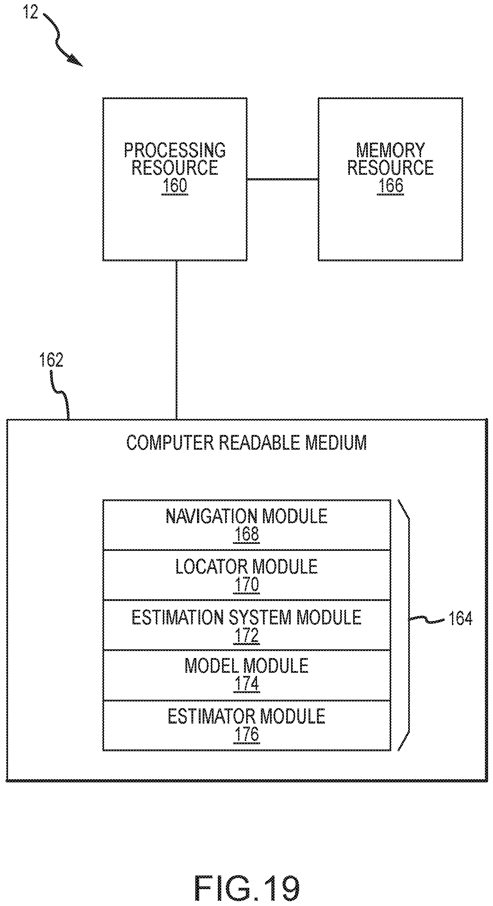

FIG. 19 depicts a block diagram of an example of a computer-readable medium in communication with processing resources of a computing device, in accordance with embodiments of the present disclosure. The main control 12, as discussed in relation to FIG. 1, can utilize software, hardware, firmware, and/or logic to perform a number of functions. The main control 12 can include a number of remote computing devices.

The main control 12 can be a combination of hardware and program instructions configured to perform a number of functions. The hardware, for example, can include one or more processing resources 160, computer readable medium (CRM) 162, etc. The program instructions (e.g., computer-readable instructions (CRI) 164) can include instructions stored on CRM 162 and executable by the processing resource 160 to implement a desired function (e.g., determine an updated location of an electrode on an impedance based medical device using the observation model, etc.). The CRI 164 can also be stored in remote memory managed by a server and represent an installation package that can be downloaded, installed, and executed. The main control 12 can include memory resources 166, and the processing resources 160 can be coupled to the memory resources 166.

Processing resources 160 can execute CRI 164 that can be stored on an internal or external non-transitory CRM 162. The processing resources 160 can execute CRI 164 to perform various functions, including the functions described above.

A number of modules 168, 170, 172, 174, 176 can be sub-modules or other modules. For example, the estimation module 172 and estimator module 174 can be sub-modules and/or contained within a single module. Furthermore, the number of modules 168, 170, 172, 174, 176 can comprise individual modules separate and distinct from one another.

A navigation module 168 can comprise CRI 164 and can be executed by the processing resource 160 to acquire measurements from a medical device 24 and render an output on a display 16. The measurements can include impedance measurement of an electrode 30 disposed on a catheter and/or impedance surface patch measurements. The measurements can also include magnetic locations of a magnetic position sensor 28 disposed on the catheter and/or magnetic measurements of a patient reference sensor 26. The navigation module 168 may call the location module 170 to obtain updated locations of electrodes and/or sensors of the medical device 24.

A locator module 170 can comprise CRI 164 and can be executed by the processing resource 160 to coordinate the operation of the estimation module 172, the model module 174 and the estimator module 176. In an example, the locator module can receive raw measurements from the navigator module in conjunction with an update request. The locator module 170 may call the estimation system module 172 to pre-process the raw measurements. Once the pre-processed measurements are acquired from the estimation module, the locator module 172 may provide the pre-processed measurements to the estimator 176 to with a request to update the current state of the system.

The estimation system module 172 can comprise CRI 164 and can be executed by the processing resource 160. In an embodiment, the estimation system module 172 defines the stochastic process of the overall system including the state transition(s) and the observational model(s). In an embodiment, the estimation system may be a Kalman system that that implements Kalman filtering techniques. In an embodiment, the estimation system module 172 calls the model module 174 to and estimator module 176 to obtain an updated state estimate.

A model module 174 can comprise CRI 164 and can be executed by the processing resource 160. The model module may include a plurality of individual models. These individual models may include one or more catheter models. In an embodiment, a medical device/catheter may be represented one or more models. Additionally, catheter models may include models of different medical devices for use when more than one catheter is within a patient reference frame. The individual models may also include a magnetic model (e.g., magnetic transformation model) that transforms locations from the patient reference frame of reference to the magnetic reference frame. The individual models may also include an impedance model or impedance transformation model that predicts impedances for locations in the patient reference frame.

An estimator module 176 can comprise CRI 164 and can be executed by the processing resource 160. The estimator module may receive update requests and inputs from the estimation system 172 and provide updated state estimates and/or predicted measurement in response. In an embodiment, the estimator module may be implemented as an extended Kalman filter.

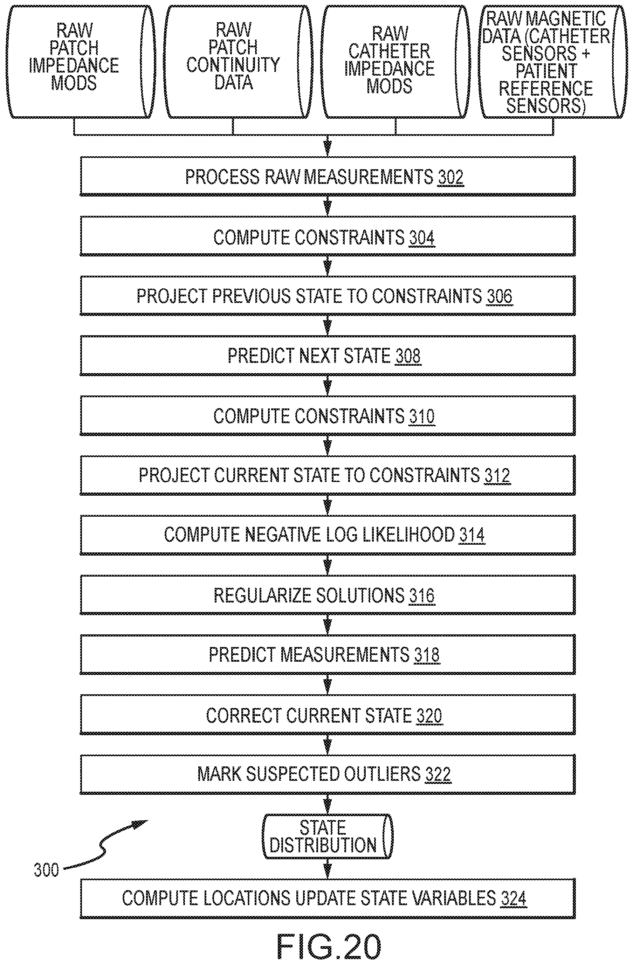

FIG. 20 depicts a flow diagram 300 associated with an overall process (e.g., sensor fusion process) to update estimated electrode locations within the three-dimensional space, in accordance with embodiments of the present disclosure. Initially, the flow diagram includes processing raw measurements at box 302. Raw measurements may include raw patch impedance measurements from the surface patch electrodes as well as patch continuity data. The patch continuity data may provide an indication regarding the contact of each surface patch and, hence, reliability of the same.

Raw electrode impedance measurements are also received for electrodes of the medical device/catheter (hereafter catheter). Raw magnetic data is also received for magnetic sensors of the catheter and for the patient reference sensor. Processing the raw measurements may include processing to raw measurements to detect any measurements that are outside a predetermined statistical range for the measurements (e.g., have a non-Gaussian error). Any such outlaying measurements may be excluded from subsequent processing. In the case of raw electrode impedance measurements, the impedance measurements can be filtered in some embodiments to remove noise from the impedance signal. In an embodiment, bio impedance scaling may be performed to help account for drift in impedance measurement (e.g., position values) of the electrodes, in some embodiments. Such bio impedance scaling may to compensate for systemic changes to conductivity with the assumption that a scalar multiplier explains the changes in impedance measurements over time. Such scaling may measure an average impedance on the non-driven patches and scaling all impedance measurements by the ratio between the current average and a historical value. In another embodiment, patch center subtraction may be applied. Patch center subtraction is a drift-compensation algorithm complimentary to bio-impedance scaling that compensates for changes in a system reference potential. In some instances, a system reference may be located a distance from the heart. The patch center subtraction algorithm computes a virtual reference coordinate from the non-driven patches and subtracts this coordinate from impedance coordinates of electrode measurements after bio-impedance scaling. Generally, such patch center subtraction re-references impedance coordinates to a location closer to the center of the heart. Other processing of the raw signals are possible and considered within the scope of the present disclosure.

At box 304 the flow diagram includes computing one or more state constraints to limit or otherwise refine the state distributions and thereby improve the overall accuracy of the system. Once such constraints are computed, the previous state may be projected to the constraints in box 306. Of note, this may include expanding the covariance matrix for the previous state to account for additional uncertainty or noise in the system for the upcoming prediction. Once this additional process noise is included in the previous state, the previous state may no longer be located on the constraint manifold. Accordingly, the previous state may be moved to the constraint manifold as discussed above.

Once the previous state is projected to the constraint(s), the next state of the system is predicted at box 308 of the flow diagram. That is, a new distribution (e.g., mean and covariance) of the state is generated using the state transition matrix $F_t$ which applies the effect of each system state parameter at time $k$-1 to the system state at time $k$. That is, a current state is predicted. Once the new state distribution (e.g., mean and covariance) is generated one or more constraints may be computed for the current state at box 310. The current state may be projected to the constraints at box 312.

In an embodiment, unlikely states in the current state are penalized to reduce the distribution of the current state. In an embodiment, a negative log likelihood is computed at box 314 of the flow diagram. In an embodiment, a probability density function is generated. This function may be applied to the current state distribution. That is, the current state distribution may be regularized at box 316 of the flow diagram.

Predicted measurements may be generated at box 318 of the flow diagram. That is, the observational model may be utilized to predict measurements (e.g., electrode and sensor location measurements) given the current predicted state to produce a distribution of predicted measurements having a mean and covariance. Once the measurements are predicted, they may be compared with the actual measurements. A difference between the predicted measurements and actual measurements may be utilized to correct the current predicted state at box 320 of the flow diagram. Once the current state is corrected, outliers may, in an embodiment, be identified and removed from the current state at box 322 of the flow diagram. At this point a new state distribution is generated for the current update (e.g., time step). From the new or updated state distribution, electrode locations may be calculated at box 324 of the flow diagram. Further, all state variables of the various models may be calculated from the updated state distribution.

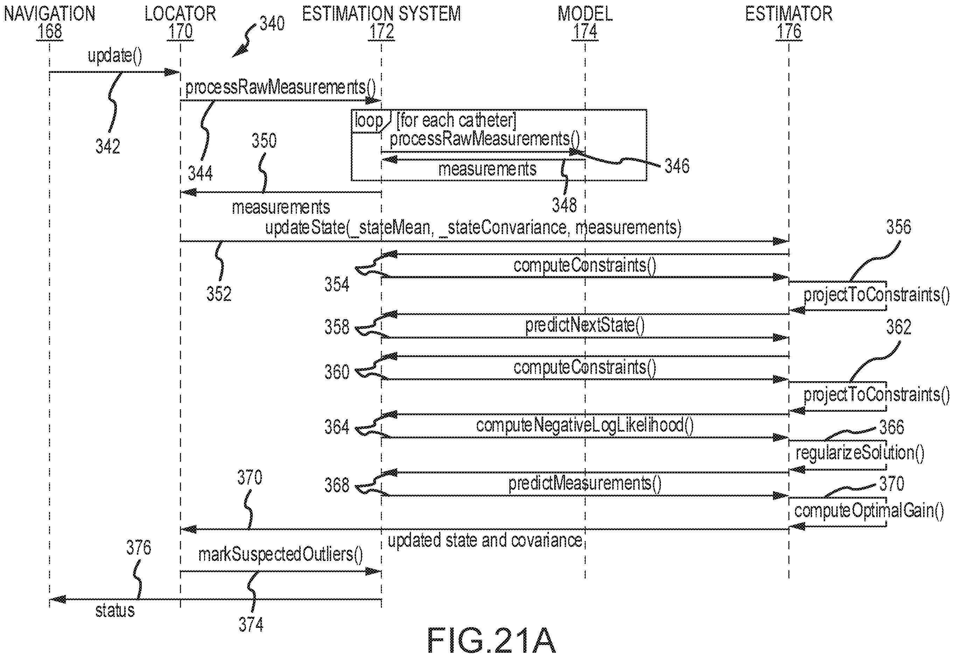

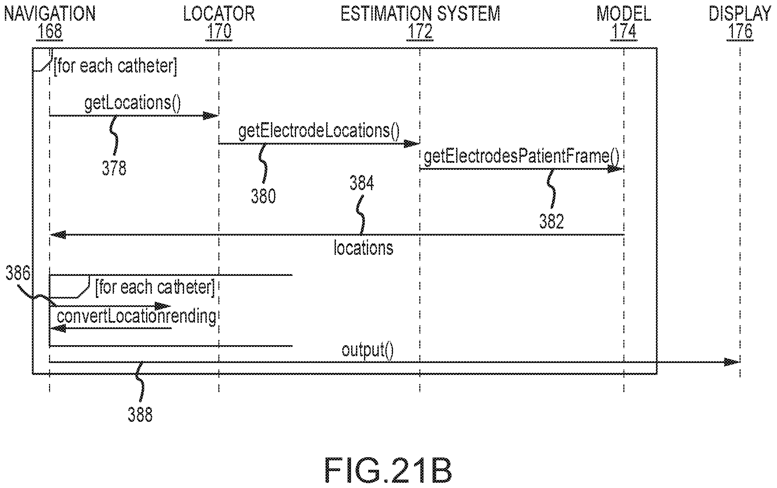

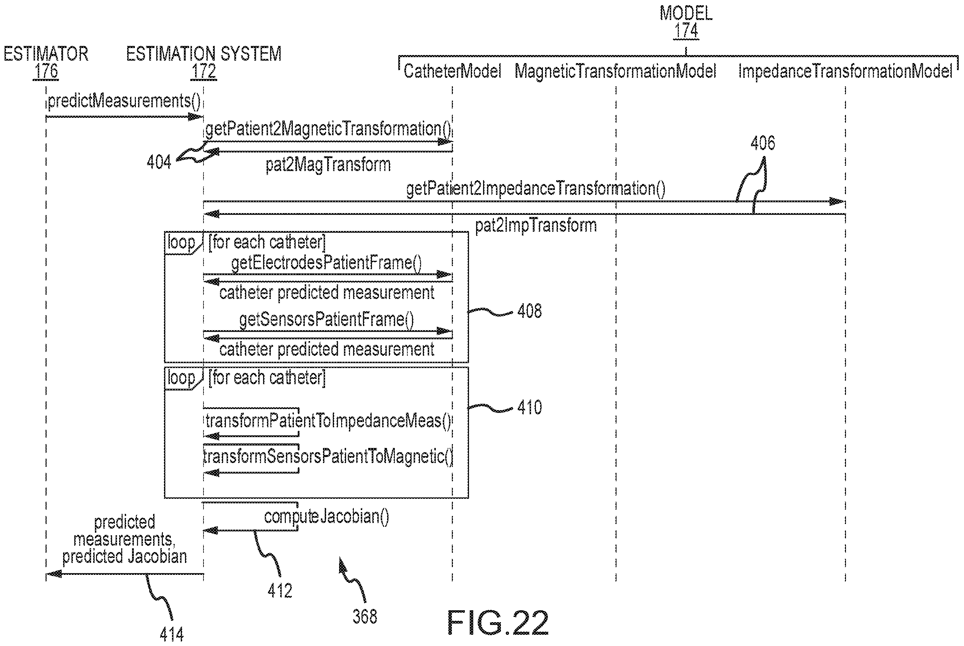

FIGS. 21A, 21B and 22 illustrate process call graphs that describe an embodiment of the interactions between the modules 168, 170, 172, 174, 176 described in FIG. 19. Referring to FIG. 21A, an update process call graph 340 is described. Initially, the navigation module 168 calls for an update 342 of the state from the locator module 170. The navigation module 168 provides new measurements (e.g., raw measurements) for the current time step (e.g., time $t$) to the locator module 170 in conjunction with the update request. Locator module 170 request 344 the estimation system module 172 process the raw measurements. The estimation system 172 communicates with a catheter model or multiple catheter models of the model module 174. More specifically, the estimation system requests 346 that the raw measurements be pre-processed in relation to the specific catheter model. Of note, in instances where multiple catheters are within a patient reference frame, this process may be performed for multiple catheters utilizing multiple catheter models. The model module 174 returns 342 preprocessed measurements to the estimation system module 172 which returns these measurements to the locator module 170. The preprocessed measurements are provided by the locator module 170 to the estimator module 176 with a request 352 to update the state distribution (e.g., state mean and state covariance) of the system for the previous time step ($k$-1). The request 352 also includes measurements for the current time step.

The estimator module 176 works with estimation system module 172, which describes the stochastic process of the system, to generate the updated state mean and state covariance for the previous time step. The estimator module 176 requests that the estimation system module 172 compute constraint(s) for the system (e.g., for the state vector) and the estimator system module 172 provides constraint(s) to the estimator module 176 in a request and a response loop 354. The estimator 176 the projects 356 the state (e.g., *k-1*) to the constraint(s). Once constrained, the estimator module 176 requests that the estimation system module 172 predict the next state of the system and the estimation system 172 returns a next state distribution estimate (e.g., mean and covariance) for the system to the estimator 176 in a request and response loop 358. The estimator module 176 and estimation system module 172 compute updated constraints for the next state distribution estimate (e.g., at time t) in a request and response loop 360. The estimator projects 362 the next state distribution estimate to the updated constraints. The estimator module 176 and estimation system module 172 compute a likelihood function for the state distribution estimate in a request and response loop 364. The estimator module 176 utilizes the likelihood function to produce 366 a regularized state distribution estimate.

The estimator module 176 and estimation system module 172 then predict measurements (e.g. electrode and sensor measurements) through application of the observation model, which maps the regularized state distribution estimate to the measurement space in a request and response loop 368. This loop 368 is further discussed in relation to FIG. 22 herein. Based on the predicted measurements the estimator module 176 determines the correspondence 370 of the predicted measurements with the actual measurements. In an embodiment where the estimator module 176 is an extended Kalman filter, this is a determination of an optimal Kalman gain. The correspondence between the predicted measurements and the actual measurements is used to correct the state distribution estimate to generate an updated state mean and covariance (e.g., updated state). That is, the regularized state distribution estimate is corrected to generate an updated state mean and covariance at time *k*, which is provided 372 to the locator module 170. In an embodiment, the locator module requests 374 the estimation system module to identify 374 suspected outliers in the updated state mean and state covariance. The locator module estimation system module 172 returns 376 the status to the navigation module 170.

If the status of the updated state mean and state covariance is acceptable, the call graph continues on FIG. 21B. In an embodiment, the navigation module requests for each catheter, locations of electrode and/or sensors within the patient frame based on the updated state mean and covariance. That is, the navigation module 168 requests 378 that the locator module 170 obtain locations (e.g., electrode locations) in the patient frame of reference. The locator module 170 requests 380 the electrode location from the estimation system based on the updated state. The estimation system 170 requests a transform from the model module 174 (e.g., patient to impedance transformation). Applying the transformation to the updated electrode locations predicts true electrode locations in the patient reference frame, which are provided to the navigation module 170. The navigation model 170 may then render or otherwise process 386 the electrode locations for an imaging system and output the electrode locations to a display 16.

As noted above, the estimator module 176 and estimation system module 172 predict measurements (e.g. electrode and sensor measurements) through application of the observation model, which maps the regularized state distribution estimate to the measurement space in a request and response loop 368 of FIG. 21A. FIG. 22 further illustrates this request and response loop. As shown, the estimator module 176 initially requests 402 the estimation system module 172 predict measurements. At this time, the estimation system module 172 interfaces with the model module 174. More specifically, the estimation system 172 provides a magnetic portion of the state distribution estimate to the magnetic transformation model 174C and provides an impedance portion of the state distribution estimate to the impedance transformation model 174C. For instance, in a request and response loop 404, the estimation system requests the patient frame of reference to magnetic frame of reference transformation from the magnetic transformation model 174B. In such a request, the estimation system module 172 may, for each state in the estimate, provide six variables (e.g., in the case of a 6 degree of freedom sensor) to the magnetic transformation model 174B, which provides a transformation matrix in response. In a request and response loop 406, the estimation system requests the patient frame of reference to impedance frame of impedance transformation from the impedance transformation model 174C. In such a request, the estimation system module 172 may, for each state, provide impedance parameters to the impedance transformation model 174C, which provides a transformation matrix in response. In a loop 408, the estimation system module 172 may obtain predicted coordinates (e.g., locations) of the sensors and electrodes in the patient frame of reference from the catheter model(s) 174A. In a second loop 410, the estimation system module 172 may apply the obtained transformations to each of the predicted locations and catheter model(s) 174A. In an embodiment, a Jacobian is calculated 412 for each state at the mean. The Jacobian determinant describes the local deltas for each state that result due to the transformation of the state space. The predicted measurements and the Jacobian are returned 414 to the estimator module 176, which compares the predicted measurements with actual measurements to compute a gain (e.g., correction) for the estimated state distribution and thereby generate the updated state and updated covariance for time *k*.

Confidence and Fault Detection

As noted in relation to FIG. 20, the sensor fusion process may include determining the reliability of raw measurements (e.g., box 302) as well as identifying outliers in the estimated state distributions (e.g., box 322). Along these lines, measured locations and/or estimated locations of catheter features such as electrodes, magnetic sensors, or the catheter itself (e.g., catheter spline) have an indication whether the location is trustworthy. In an embodiment, such an indication may be based on a statistical confidence interval computed from the estimated state distribution and on the convergence time. When the reliability of a location is poor, collection of historical data points is suppressed.

For each group of measurements (e.g. the 6 impedance interrogations (hereafter 'impedance mods') from an electrode, measurements from a sensor, each patch, all patch impedance data combined, all magnetic data combined, each single impedance mod, all impedance data combined, each single catheter) a reliability value indicates presence or absence of a plurality of measurement problems, such as disconnected or incorrectly connected electrodes, or measurements that cannot be explained by the state transition and observation models. When these measurement problems are detected, an indication of the fault may be displayed to the user and/or data collection from the corresponding medical device may be suppressed for some types of faults. Detection of some measurement problems excludes the corresponding measurements from the sensor fusion process. Generally, it is desirable to: 1) determine whether the corresponding estimated catheter locations are reliable; 2) detect measurement faults requiring user action to remedy and/or detect faulty measurements to be excluded from the sensor fusion algorithm; and/or 3) determine statistical outliers.

Reliability

In an embodiment, all location estimates may be marked as unreliable during a designated time interval, after the sensor fusion process is started. This unreliability period may be implement at the estimations of the magnetic transformation, impedance transformation, catheter shapes, and other state variables can take time to settle. Accordingly, all location estimates may be marked as unreliable during a designated time interval, such as 120 seconds, after the sensor fusion process is started. Location estimates on a catheter can also be marked as unreliable for a time after the catheter is introduced, while the catheter is inside a sheath, for a time after the catheter leaves a sheath, or when the catheter is disconnected.

In an embodiment, bounds may be put on various distance errors and/or mark the participating catheter feature locations as unreliable. In such an embodiment, the bounds may include determining: an error distance between the true and estimated location of a catheter feature; an error in distance between a catheter feature and an anatomic location, such as a nearby surface, nearby lesion, or nearby mapping point; and/or an error in distance between two separate catheter features, on the same or distinct catheters. For such distances, a tolerance is set. The tolerance may be set as a physical distance (e.g., 2mm) or as a percentage of an estimated value (e.g., 10%). In application, the estimator produces a probability distribution over the state variables of the system. For example, a one dimensional state distribution (e.g., bell curve). For each possible state, locations of all catheter features can be computed. The system computes the probability that each distance of a catheter feature relative to the mean for the state variable exceeds its tolerance. If the probability exceeds a threshold (e.g., two standard deviations in a one dimensional distribution), then catheter feature locations participating in that distance are marked as unreliable.

In an embodiment, a numerical method to compute the probability of a distance exceeding its tolerance includes Monte Carlo sampling. In such an embodiment, states are sampled at random from the probability distribution of states. Distances are computed for each sample. The percentage of distances that that exceed their tolerance is determined. If more than a threshold percentage of the distances exceed the tolerance then the distance is considered unreliable.

In an embodiment, a numerical method to compute the probability of a distance exceeding its tolerance includes an analytic heuristic. In this embodiment, for a Gaussian state distribution, local linearization of catheter feature offsets from the estimate are computed. Each offset will then have a Jacobian. If the state distribution has mean x and covariance S, the distribution of the offset is well approximated by:

$$N(0, JSJ^T)$$

If the error is close to isotropic, then the square root of $trace(JSJ^T)$ approximates the standard deviation of the squared distance error. When this value exceeds an upper bound then the distance is considered unreliable and therefore the participating location estimate( s) are considered unreliable.

Faulty measurements

Several heuristic fault detection algorithms may be implemented to detect problems with impedance data. The following circumstances detect electrodes considered disconnected or physically faulty. If these circumstances are detected, the measurements are excluded from the estimator. In an embodiment, electrodes whose impedance values are larger than a threshold are considered disconnected or faulty. In another embodiment, electrodes having an impedance value that differs from the average of all electrodes, which are not considered faulty.

The following embodiments detect electrodes considered disconnected, physically faulty, or with interchanged connections. These measurements are excluded from the estimator. In an embodiment, electrodes whose location estimates according to the sensor fusion process differ by more than a threshold from their estimates according to an impedance-primary algorithm, are excluded. In another embodiment, a polyline is fit the electrode locations. If the polyline through a series of adj acent electrodes has a sharp angle at one of the electrodes, that electrode is considered a candidate for misconnection detection. Alternatively if the average bend angle exceeds a threshold, all electrodes on the catheter spline are considered candidates for misconnection detection. Then misconnection candidates that are not actually misconnected are removed from consideration as follows: if there is a single electrode that is flagged as potentially misconnected and no other electrodes have bad measurement status or were marked as disconnected, then that single electrode is removed. In an embodiment, an electrode is removed if the sensor fusion process estimate of the electrode location is closer to the impedance location of that electrode than to the impedance location of any other misconnection candidate.

Statistical outliers.

The state estimator computes a probability distribution over all measurements. For each measurement group, a test statistic may be computed comparing the measurements to their expected distribution. In an embodiment having a generally Gaussian distribution computed by the estimator a Mahalanobis distance from the mean to the measurements may, in an embodiment, form the test statistic. The Mahalanobis distance is a measure of the distance between a point P and a mean of a distribution D containing the point. If the distance exceeds a predetermined threshold, then the measurement group is marked as a statistical outlier.

Embodiments are described herein of various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment(s) is included in at least one embodiment. Thus, appearances of the phrases "in various embodiments," "in some embodiments," "in one embodiment," or "in an embodiment," or the like, in places throughout the specification, are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the devices. Joinder references (e.g., affixed, attached, coupled, connected, and the like) are to be construed broadly and can include intermediate members between a connection of elements and relative movement between elements. As such, joinder references do not necessarily infer that two elements are directly connected and in fixed relationship to each other. It is intended that all matter contained in the above description or shown in the accompanying drawings shall be interpreted as illustrative only and not limiting.

## Claims

1.    A method for use in identifying and outputting locations of electrodes on a display, comprising:

defining relative positions of physical electrodes ($33_1$-$33_4$) in a catheter shape model (42), wherein the relative positions correspond to spacings of the physical electrodes (33) of a physical catheter (50);

predicting locations of model electrodes ($30_1$-$30_4$) in a patient reference frame (6) by utilizing the catheter shape model (42) including locations of model electrodes ($30_1$-$30_4$) and a model magnetic sensor ($28_1$) in a catheter reference frame (8), wherein the model electrodes ($30_1$-$30_4$) and model magnetic sensor ($28_1$) correspond to a set of the physical electrodes ($33_1$-$33_4$) and a magnetic sensor ($28_2$) of the physical catheter (50) disposed within the patient reference frame;

generating predicted impedance responses ($31_1$-$31_4$) for the predicted model electrode locations by applying an impedance model (48) of an applied electrical potential field to the model electrode locations, wherein the impedance model (48) transforms each model electrode location to a predicted impedance response;

measuring impedance responses ($35_1$-$35_4$) for the physical electrodes ($33_1$-$33_4$) of the physical catheter (50) in response to the applied electrical potential field; and

based at least on the predicted impedance responses ($31_1$-$31_4$) and the measured impedance responses ($35_1$-$35_4$), generating calculated locations ($37_1$-$37_4$) of the physical electrodes (33); and

outputting the calculated locations of the physical electrodes ($33_1$-$33_4$) to the display (16).

2. The method of Claim 1, further comprising:

predicting a location of the model magnetic sensor ($28_1$) in the patient reference frame (6) by utilizing the catheter model (42);

generating a predicted magnetic response for the model magnetic sensor location;

measuring a magnetic response in a magnetic reference frame (4) of the physical magnetic sensor ($28_2$) in response to an applied magnetic field;

wherein the calculated locations are further based on the predicted magnetic response and the measured magnetic response.

3. The method of Claim 1, further comprising:
applying a catheter transformation to the catheter shape model (42) to transform a position and orientation of the model electrodes ($30_1$-$30_4$) of the catheter shape model (42) from the catheter reference frame (8) to the patient reference frame (6).

4. The method of Claim 3, wherein applying the catheter transformation to the catheter shape model (42) comprises applying a rigid body six-degree-of-freedom transformation to the catheter shape model (42).

5. The method of Claim 3, wherein applying the catheter transformation to the catheter shape model (42) further comprises:
applying the catheter transformation to the model magnetic sensor ($28_1$) to transform a position and orientation of the model magnetic sensor ($28_1$) from the catheter reference frame (8) to the patient reference frame (6).

6. The method of Claim 1, wherein generating the predicted impedance responses further comprises:
updating the impedance model (48) based the predicted impedance responses and the impedance responses of the physical electrodes (33).

7. The method of Claim 1, f,r wherein the catheter shape model (42) and the impedance model (48) are state variables of a composite model (40) that models the physical catheter (50) in the patient reference frame.

8. The method of Claim 7, wherein an Extended Kalman Filter is used to infer the state variables.

9. The method of Claim 7, further comprising:
using the composite model (40) to generate an estimated state distribution of potential electrode locations, wherein the calculated locations are generated using the state distribution.

10. The method of Claim 9, further comprising:
applying at least a first constraint to the estimated state distribution, where the first constraint constrains at least one of the state variables, wherein the first constraint limits the estimated state distribution.

11. The method of Claim 9, further comprising:
applying a function to the estimated state distribution to remove unlikely states from the estimated state distribution.

**12.** The method of Claim 9, further comprising:

comparing the predicted impedance responses with the impedance responses; and
generating a correction based on the comparison.

**13.** The method of Claim 12, further comprising:

applying the correction to the estimated state distribution to generate an updated state distribution, wherein the calculated locations are generated using the updated state distribution, and
identifying outlying states in the updated state distribution, wherein outlying states are removed from the updated state distribution.

**14.** A non-transitory computer-readable medium storing instructions to identifying locations of electrodes, executable to perform the steps of the method according to any one of claims 1 to 13.

**15.** A system for identifying locations of electrodes, comprising:

a physical catheter (50) having physical electrodes (33) disposed in a three-dimensional space;
a medical positioning system to measure impedance responses of the physical electrodes in response to an applied electrical potential field;
a processor (160) and memory (166) for storing non-transitory computer-readable instructions, and
a display operatively connected to the processor and memory,
wherein the processor (160) is adapted to execute the instruction to perform the steps of the method according to any one of claims 1 to 13.

**Patentansprüche**

**1.** Ein Verfahren zur Verwendung bei der Identifikation und Ausgabe von Orten von Elektroden auf einer Anzeige, das Folgendes umfasst:

Definieren relativer Positionen physischer Elektroden ($33_1$-$33_4$) in einem Kathetergestaltmodell (42), wobei die relativen Positionen den Beabstandungen der physischen Elektroden (33) eines physischen Katheters (50) entsprechen;
Vorhersagen von Orten von Modellelektroden ($30_1$-$30_4$) in einem Patientenbezugsrahmen (6) durch Einsetzen des Kathetergestaltmodells (42), das Orte von Modellelektroden ($30_1$-$30_4$) einschließt, und eines Modellmagnetsensors ($28_1$) in einem Katheterbezugsrahmen (8), wobei die Modellelektroden ($30_1$-$30_4$) und der Modellmagnetsensor (281) einem Satz der physischen Elektroden ($33_1$-$33_4$) und einem Magnetsensor ($28_2$) des physischen Katheters (50) entsprechen, der innerhalb des Patientenbezugsrahmens angeordnet ist;
Erzeugen vorhergesagter Impedanzantworten ($31_1$-$31_4$) für die vorhergesagten Modellelektrodenorte durch Anwenden eines Impedanzmodells (48) eines angelegten elektrischen Potentialfelds auf die Modellelektrodenorte, wobei das Impedanzmodell (48) jeden Modellelektrodenort in eine vorhergesagte Impedanzantwort transformiert;
Messen von Impedanzantworten ($35_1$-$35_4$) für die physischen Elektroden ($33_1$-$33_4$) des physischen Katheters (50) als Antwort auf das angelegte elektrische Potentialfeld; und
basierend mindestens auf den vorhergesagten Impedanzantworten ($31_1$-$31_4$) und den gemessenen Impedanzantworten ($35_1$-$35_4$), Erzeugen berechneter Orte ($37_1$-$37_4$) der physischen Elektroden (33); und
Ausgeben der berechneten Orte der physischen Elektroden ($33_1$-$33_4$) an die Anzeige (16).

**2.** Verfahren nach Anspruch 1, das ferner Folgendes umfasst:

Vorhersagen eines Orts des Modellmagnetsensors ($28_1$) in dem Patientenbezugsrahmen (6) durch Einsetzen des Kathetermodells (42);
Erzeugen einer vorhergesagten Magnetantwort für den Modellmagnetsensorort;
Messen einer Magnetantwort in einem Magnetbezugsrahmen (4) des physischen Magnetsensors ($28_2$) als Antwort auf ein angelegtes Magnetfeld;
wobei die berechneten Orte ferner auf der vorhergesagten Magnetantwort und der gemessenen Magnetantwort basieren.

3. Verfahren nach Anspruch 1, das ferner Folgendes umfasst:
Anwenden einer Kathetertransformation auf das Kathetergestaltmodell (42), um eine Position und Orientierung der Modellelektroden ($30_1$-$30_4$) des Kathetergestaltmodells (42) von dem Katheterbezugsrahmen (8) in den Patientenbezugsrahmen (6) zu transformieren.

4. Verfahren nach Anspruch 3, wobei das Anwenden der Kathetertransformation auf das Kathetergestaltmodell (42) das Anwenden einer Starrkörper-Sechs-Freiheitsgrad-Transformation auf das Kathetergestaltmodell (42) umfasst.

5. Verfahren nach Anspruch 3, wobei das Anwenden der Kathetertransformation auf das Kathetergestaltmodell (42) ferner Folgendes umfasst:
Anwenden der Kathetertransformation auf den Modellmagnetsensor ($28_1$), um eine Position und Orientierung des Modellmagnetsensors ($28_1$) von dem Katheterbezugsrahmen (8) in den Patientenbezugsrahmen (6) zu transformieren.

6. Verfahren nach Anspruch 1, wobei das Erzeugen der vorhergesagten Impedanzantworten ferner Folgendes umfasst:
Aktualisieren des Impedanzmodells (48), basierend auf den vorhergesagten Impedanzantworten und den Impedanzantworten der physischen Elektroden (33).

7. Verfahren nach Anspruch 1, wobei das Kathetergestaltmodell (42) und das Impedanzmodell (48) Zustandsvariablen eines zusammengesetzten Modells (40) sind, das den physischen Katheter (50) in dem Patientenbezugsrahmen modelliert.

8. Verfahren nach Anspruch 7, wobei ein erweitertes Kalmanfilter zum Ableiten der Zustandsvariablen verwendet wird.

9. Verfahren nach Anspruch 7, das ferner Folgendes umfasst:
Verwenden des zusammengesetzten Modells (40), um eine geschätzte Zustandsverteilung potentieller Elektrodenorte zu erzeugen, wobei die berechneten Orte unter Verwendung der Zustandsverteilung erzeugt werden.

10. Verfahren nach Anspruch 9, das ferner Folgendes umfasst:
Anwenden mindestens einer ersten Einschränkung auf die geschätzte Zustandsverteilung, wobei die erste Einschränkung mindestens eine der Zustandsvariablen einschränkt, wobei die erste Einschränkung die geschätzte Zustandsverteilung begrenzt.

11. Verfahren nach Anspruch 9, das ferner Folgendes umfasst:
Anwenden einer Funktion auf die geschätzte Zustandsverteilung, um unwahrscheinliche Zustände aus der geschätzten Zustandsverteilung zu entfernen.

12. Verfahren nach Anspruch 9, das ferner Folgendes umfasst:

Vergleichen der vorhergesagten Impedanzantworten mit den Impedanzantworten; und
Erzeugen einer auf dem Vergleich basierenden Korrektur.

13. Verfahren nach Anspruch 12, das ferner Folgendes umfasst:

Anwenden der Korrektur auf die geschätzte Zustandsverteilung, um eine aktualisierte Zustandsverteilung zu erzeugen, wobei die berechneten Orte unter Verwendung der aktualisierten Zustandsverteilung erzeugt werden, und
Identifizieren von Ausreißerzuständen in der aktualisierten Zustandsverteilung, wobei die Ausreißerzustände aus der aktualisierten Zustandsverteilung entfernt werden.

14. Ein nichtflüchtiges computerlesbares Medium, das Anweisungen zur Identifizierung von Orten von Elektroden speichert, die ausgeführt werden können, um die Schritte des Verfahrens nach einem der Ansprüche 1 bis 13 durchzuführen.

15. Ein System zur Identifizierung von Orten von Elektroden, das Folgendes umfasst:

einen physischen Katheter (50), der physische Elektroden (33) aufweist, die in einem dreidimensionalen Raum angeordnet sind;

ein medizinisches Positionierungssystem zum Messen von Impedanzantworten der physischen Elektroden als Reaktion auf ein angelegtes elektrisches Potentialfeld;

einen Prozessor (160) und Speicher (166) zum Speichern nichtflüchtiger computerlesbarer Anweisungen und eine Anzeige, die betriebsfähig mit dem Prozessor und Speicher verbunden ist,

wobei der Prozessor (160) zum Ausführen der Anweisung eingerichtet ist, um die Schritte des Verfahrens nach einem der Ansprüche 1 bis 13 durchzuführen.

## Revendications

1.  Procédé destiné à une utilisation pour l'identification et la sortie d'emplacements d'électrodes sur un affichage, comprenant :

    la définition de positions relatives d'électrodes physiques ($33_1$-$33_4$) dans un modèle de forme de cathéter (42), les positions relatives correspondant à des espacements des électrodes physiques (33) d'un cathéter physique (50) ;
    la prédiction d'emplacements d'électrodes modèles ($30_1$-$30_4$) dans un cadre de référence de patient (6) en utilisant le modèle de forme de cathéter (42) comprenant des emplacements d'électrodes modèles ($30_1$-$30_4$) et un capteur magnétique modèle ($28_1$) dans un cadre de référence de cathéter (8), les électrodes modèles ($30_1$-$30_4$) et le capteur magnétique modèle ($28_1$) correspondant à un ensemble des électrodes physiques ($33_1$-$33_4$) et à un capteur magnétique ($28_2$) du cathéter physique (50) disposé dans le cadre de référence de patient ;
    la génération de réponses d'impédance prédites ($31_1$-$31_4$) pour les emplacements d'électrodes modèles prédits en appliquant un modèle d'impédance (48) d'un champ de potentiel électrique appliqué aux emplacements d'électrodes modèles, le modèle d'impédance (48) transformant chaque emplacement d'électrode modèle en une réponse d'impédance prédite ;
    la mesure de réponses d'impédance ($35_1$-$35_4$) pour les électrodes physiques ($33_1$-$33_4$) du cathéter physique (50) en réponse au champ de potentiel électrique appliqué ; et à partir au moins des réponses d'impédance prédites ($31_1$-$31_4$) et des réponses d'impédance mesurées $35_1$-$35_4$), la génération d'emplacements calculés ($37_1$-$37_4$) des électrodes physiques (33) ; et
    la sortie des emplacements calculés des électrodes physiques ($33_1$-$33_4$) sur l'affichage (16) .

2.  Procédé selon la revendication 1, comprenant en outre :

    la prédiction d'un emplacement du capteur magnétique modèle ($28_1$) dans le cadre de référence de patient (6) en utilisant le modèle de cathéter (42) ;
    la génération d'une réponse magnétique prédite pour l'emplacement du capteur magnétique modèle ;
    la mesure d'une réponse magnétique dans un cadre de référence magnétique (4) du capteur magnétique physique ($28_2$) en réponse à un champ magnétique appliqué ;
    dans lequel les emplacements calculés sont en outre basés sur la réponse magnétique prédite et la réponse magnétique mesurée.

3.  Procédé selon la revendication 1, comprenant en outre :
    l'application d'une transformation de cathéter au modèle de forme de cathéter (42) pour transformer une position et une orientation des électrodes modèles ($30_1$-$30_4$) du modèle de forme de cathéter (42) en les faisant passer du cadre de référence de cathéter (8) au cadre de référence de patient (6).

4.  Procédé selon la revendication 3, dans lequel l'application de la transformation de cathéter au modèle de forme de cathéter (42) comprend l'application d'une transformation à six degrés de liberté de type corps rigide au modèle de forme de cathéter (42).

5.  Procédé selon la revendication 3, dans lequel l'application de la transformation de cathéter au modèle de forme de cathéter (42) comprend en outre :
    une application de la transformation de cathéter au capteur magnétique modèle (281) pour transformer une position et une orientation du capteur magnétique modèle ($28_1$) en les faisant passer du cadre de référence de cathéter (8) au cadre de référence de patient (6).

6.  Procédé selon la revendication 1, dans lequel la génération des réponses d'impédance prédites comprend en outre :
    une mise à jour du modèle d'impédance (48) à partir des réponses d'impédance prédites et des réponses d'impédance des électrodes physiques (33).

7. Procédé selon la revendication 1,
dans lequel le modèle de forme de cathéter (42) et le modèle d'impédance (48) sont des variables d'état d'un modèle composite (40) qui modélise le cathéter physique (50) dans le cadre de référence de patient.

8. Procédé selon la revendication 7, dans lequel un filtre de Kalman étendu est utilisé pour déduire les variables d'état.

9. Procédé selon la revendication 7, comprenant en outre :
une utilisation du modèle composite (40) pour générer une distribution d'états estimée des emplacements d'électrodes potentiels, les emplacements calculés étant générés en utilisant la distribution d'états.

10. Procédé selon la revendication 9, comprenant en outre :
l'application d'au moins une première contrainte à la distribution d'états estimée, la première contrainte s'appliquant à au moins une des variables d'état, la première contrainte limitant la distribution d'états estimée.

11. Procédé selon la revendication 9, comprenant en outre :
l'application d'une fonction à la distribution d'états estimée pour supprimer des états improbables de la distribution d'états estimée.

12. Procédé selon la revendication 9, comprenant en outre :

une comparaison des réponses d'impédance prédites avec les réponses d'impédance ; et
la génération d'une correction à partir de la comparaison.

13. Procédé selon la revendication 12, comprenant en outre :

une application de la correction à la distribution d'états estimée pour générer une distribution d'états mise à jour, les emplacements calculés étant générés en utilisant la distribution d'états mise à jour,
et
l'identification d'états aberrants dans la distribution d'états mise à jour, les états aberrants étant supprimés de la distribution d'états mise à jour.

14. Support non transitoire lisible par ordinateur sur lequel sont stockées des instructions pour l'identification d'emplacements d'électrodes, exécutables pour réaliser les étapes du procédé selon l'une quelconque des revendications 1 à 13.

15. Système d'identification d'emplacements d'électrodes, comprenant :

un cathéter physique (50) comportant des électrodes physiques (33) disposées dans un espace tridimensionnel ;
un système de positionnement médical pour mesurer des réponses d'impédance des électrodes physiques en réponse à un champ de potentiel électrique appliqué ;
un processeur (160) et une mémoire (166) pour stocker des instructions non transitoires lisibles par ordinateur, et
un affichage fonctionnellement connecté au processeur et à la mémoire,
dans lequel le processeur (160) est conçu pour exécuter l'instruction de réaliser les étapes du procédé selon l'une quelconque des revendications 1 à 13.

FIG.1

EP 3 852 623 B1

FIG.2

EP 3 852 623 B1

FIG.3A

FIG.3B

FIG.3C

FIG.3D

FIG.4

EP 3 852 623 B1

FIG.5A

FIG.5B

FIG.5C

EP 3 852 623 B1

FIG.6A

FIG.6B

FIG.7A

FIG.7B

FIG.7C

FIG.8B

FIG.8A

FIG.8C

FIG.8D

FIG.9

FIG.10

FIG. 11A

FIG.11B

FIG.11C

nomPatToMag

100

26

26A

100A

PatToNomPat

4

FIG.12A

EP 3 852 623 B1

FIG.12B

NECK
PATCH
64

Zn-b

BACK
PATCH
62

Zr-b

RIGHT
PATCH
58

30

24

Zb-l

Zc-r

LEFT
PATCH
56

Zl-c

CHEST
PATCH
60

Zleg-b

LEG
PATCH
66

FIG.13A

FIG.13B

FIG.14

FIG.15

FIG.16

FIG.17

EP 3 852 623 B1

FIG.18

12

162

PROCESSING
RESOURCE
160

MEMORY
RESOURCE
166

COMPUTER READABLE MEDIUM

NAVIGATION MODULE
168

LOCATOR MODULE
170

ESTIMATION SYSTEM MODULE
172

MODEL MODULE
174

ESTIMATOR MODULE
176

164

FIG.19

RAW PATCH IMPEDANCE MODS

RAW PATCH CONTINUITY DATA

RAW CATHETER IMPEDANCE MODS

RAW MAGNETIC DATA (CATHETER SENSORS + PATIENT REFERENCE SENSORS)

PROCESS RAW MEASUREMENTS 302

COMPUTE CONSTRAINTS 304

PROJECT PREVIOUS STATE TO CONSTRAINTS 306

PREDICT NEXT STATE 308

COMPUTE CONSTRAINTS 310

PROJECT CURRENT STATE TO CONSTRAINTS 312

COMPUTE NEGATIVE LOG LIKELIHOOD 314

REGULARIZE SOLUTIONS 316

PREDICT MEASUREMENTS 318

CORRECT CURRENT STATE 320

MARK SUSPECTED OUTLIERS 322

STATE DISTRIBUTION

300

COMPUTE LOCATIONS UPDATE STATE VARIABLES 324

# FIG.20

FIG.21A

FIG.21B

MODEL
174

ESTIMATOR     ESTIMATION SYSTEM
176                 172                          CatheterModel   MagneticTransformationModel   ImpedanceTransformationModel

predictMeasurements()

getPatient2MagneticTransformation()

404 — pat2MagTransform

getPatient2ImpedanceTransformation()                                      406

pat2ImpTransform

loop [for each catheter]
getElectrodesPatientFrame()
catheter predicted measurement
getSensorsPatientFrame()                                408
catheter predicted measurement

loop [for each catheter]                                  410

transformPatientToImpedanceMeas()

transformSensorsPatientToMagnetic()

predicted
measurements,        computeJacobian()
predicted Jacobian

412            368

414

FIG.22

**REFERENCES CITED IN THE DESCRIPTION**

**Patent documents cited in the description**

- US 62756941
- US 62756915
- US 62756926
- US 62756931
- US 62756936
- WO 2016205809 A1
- WO 2016205807 A1
- US 2012150022 A1
- US 650932
- US 7263397 B, Hauck

- US 20070060833 A1, Hauck
- US 980515
- US 7386339 B
- US 2013066193 A
- US 7197354 B
- US 6233476 B
- US 20130066193 A
- US 20160367168 A
- US 8744599 B